# EUROPEAN PATENT SPECIFICATION

(11) **EP 4 376 919 B1**
(45) Date of publication and mention of the grant of the patent: **18.11.2026**
(21) Application number: 22760879.1
(22) Date of filing: 28.07.2022
(51) Int. Cl.: A61M 5/142, A61M 5/162, A61M 5/158, A61M 5/172, A61M 39/08, A61M 39/10

(54) **APPARATUS FOR ADMINISTRATION OF MEDICINE INCLUDING MONITORING AND USER FEEDBACK**
VORRICHTUNG ZUR VERABREICHUNG VON MEDIKAMENTEN MIT ÜBERWACHUNG UND BENUTZERFEEDBACK
APPAREIL POUR ADMINISTRATION DE MÉDICAMENTS INCLUANT LA SURVEILLANCE ET LA RÉTROACTION DE L'UTILISATEUR

(30) Priority: 28.07.2021 US 202163226494 P; 28.07.2021 US 202163226498 P; 28.07.2021 US 202163226499 P; 27.07.2022 US 202263392539 P
(43) Date of publication of application: 05.06.2024
(73) Proprietor: SHL Medical AG, 6302 Zug (CH)
(72) Inventor: COYNE III, Martin Michael, Boonton, New Jersey 07005 (US); FRANZESE, Christopher James, Boonton, New Jersey 07005 (US); LARSON-WAKEMAN, Molly Christine, Boonton, New Jersey 07005 (US); MARTINEZ, Alejandra Linares, Boonton, New Jersey 07005 (US); D'AURIA, Matthew Conner, Boonton, New Jersey 07005 (US); BRIGHAM, Katherine Ford, Boonton, New Jersey 07005 (US); KARLSSON, Sebastian, 6302 Zug (CH); EGLOFF, Christoph, 6302 Zug (CH); ANDERSON, Madeleine Hayden, Boonton, New Jersey 07005 (US)
(86) International application number: PCT/EP2022/071260
(87) International publication number: WO 2023/006907

(56) References cited:
- US-A1- 2006 224 141
- US-A1- 2016 175 521
- US-A1- 2020 281 503
- US-A1- 2020 390 350

## Description

### RELATED APPLICATIONS

US provisional application 63/226,494, filed on 28 July 2021; US provisional application 63/226498, filed on 28 July 2021; and 63/226499, filed on 28 July 2021.

The disclosure of US provisional application 63/392,539, filed on 27 July 2022.

### TECHNICAL FIELD

Embodiments of the disclosure generally relate to apparatus, systems and methods for administration of therapeutic medicines. More particularly, embodiments of the disclosure relate to apparatus, systems and methods configured to power and/or monitor one or more sensors on a patient, the sensors configured to communicate with either a separate or integrated medication delivery apparatus and to communicate commands from a medication delivery apparatus to a needle assembly on a patient. In some embodiments of the disclosure, the apparatus may also be configured to provide a user of the system visual feedback on the device status.

Further embodiments of the disclosure generally relate to apparatus and methods for large volume infusion of therapeutic medicines. Specific embodiments of the disclosure pertain to apparatus and methods configured to deliver a one or more therapeutic medications to a patient at a known, preselected, and controlled flow rate.

### BACKGROUND

Infusion and injection are commonplace medical procedures used to administer a wide variety of therapeutic medicines of interest to treat a variety of diseases. As used herein, "infusion," "injection," and "administration" are used interchangeably, taking place by subcutaneous (SC), intramuscular (IM), intravenous (IV), or enteral routes, also terms used interchangeably. Liquid medication formulations are commonly infused and injected through a variety of needle assemblies, tubing sets, and fluid pumps through the intravenous, subcutaneous, and intramuscular routes.

Existing prefilled syringe (PFS) and autoinjector (Al) devices are suited for delivery of a small volume (≤ 2mL) of a single biologic medicine, which are common treatments for chronic cardiovascular, gastrointestinal, autoimmune, immunologic, hematologic, endocrinology, and respiratory diseases. These medications are taken on a daily, weekly, biweekly, monthly, or quarterly basis. In general, a single medication is given, the dose does not change at each dosing interval, and the medications rarely provoke serious reactions in patients, facilitating straightforward administration at home. Thus, PFS and AI devices are designed for intuitive and rapid (~10-15 sec) delivery of a single fixed-dose medicine by a user lacking clinical training, such as the patient themselves or a caregiver. While PFS and AI devices are well suited to simple low-volume monotherapies, novel delivery apparatus is needed to meet the needs of more complex clinical regimens.

Clinically, complex regimens are characterized by one or more aspects including, by way of example, large, delivered fluid volumes, numerous therapeutic medications, potentially variable dosing, medications given before and/or after a therapeutic medicine, and different sequences of medication administration. Administration may extend over a period of many hours and is currently performed in a hospital or clinic setting, overseen by a trained healthcare provider.

In a hospital or clinic setting, medication administration often includes supplemental monitoring of a patient's physiologic attributes using hospital-grade telemetry equipment that is separate from the drug delivery device and, like the delivery device, is durable equipment used for multiple patients. These physiologic parameters may include by way of example, one or more of heart rate, blood pressure, respiratory rate, blood oxygen saturation (SpO₂), and temperature. Healthcare providers monitor patient physiologic aspects before, during, and after medication administration to distinguish serious adverse events, such as a life-threatening systemic infusion reaction (SIR), from less side effects. Based on such data and clinical judgment, they must also make any appropriate changes to medication delivery. For example, if an SIR is detected by the clinician, they may stop medication delivery and administer one or more medications to treat the reaction. Thus, drug delivery devices for home administration of complex regimens should permit configuration to contingently administer emergency medications (emergency medicaments) at home based on physiologic data without a healthcare provider present, or with a clinically untrained user, such as a patient.

For home administration of complex medication regimens, it is obviously infeasible to routinely provide hospital-grade administration or patient monitoring equipment to every patient or place a clinician into each patient's home for many hours. Such equipment is bulky and expensive, requires clinical experience to safely operate, and relies upon clinical judgment for appropriate decisions related to safe medication administration. Untrained users, such as patients or caregivers at home, may be confused or overwhelmed by the multitude of data, settings, configurations, and connections with hospital grade administration or patient monitoring equipment; moreover, such confusion may lead to incorrect decisions or operation that may lead to situations that cause harm.

Thus, there is a need to provide improved drug delivery apparatus that allow safe home administration of more complex medication regimens, that allow intuitive use by those without clinical training, that provide patient monitoring capability to identify needed changes to medication administration without the presence of a healthcare provider, and that provide a user intuitive feedback about the status of administration.

For example, there is need to provide a drug delivery apparatus with medication reservoirs and a drive unit beside a patient or in a small bag, with only a small, lightweight needle assembly worn on the patient. The needle assembly may also include one or more sensors, or sensors may be separate from the needle assembly and attached to the patient. Such reservoirs and drive unit are fluidically connected to the needle assembly by a tubing set, the subject of the present disclosure.

Administration route is based on a specific medication's pharmacokinetic (PK) profile, formulation components, approved regulatory labeling, individual clinical judgment, or clinical necessity.

The SC or IM route is frequently used for administration of smaller volumes using prefilled syringes and autoinjectors. Biologic medicines are frequently administered via the SC route with these devices. However, medications with larger volumes are not suitable for these devices, and the IV route is typically chosen, generally in hospitals or outpatient clinics. Given the safety risks and patient burden of at-home IV administration, pharmaceutical companies and patients generally prefer at-home SC administration. SC administration is generally considered less invasive and more straightforward for patients. As physiologic uptake of medication is slower via the SC route, there is potential for improved tolerability compared to IV administration.

Given these significant advantages in safety, tolerability, and convenience, the pharmaceutical industry has invested heavily in transitioning formulations from IV to SC administration and medication administration from the clinic to the home setting. However, many large volume delivery devices such as syringe or volumetric pumps are intended for use only by trained healthcare professionals and are unsuitable for home use.

Ambulatory pumps for home use have been developed that provide an alternative to hospital-grade devices. However, they require configuration by a healthcare provider, aseptic assembly of components by patients, and may not work properly if specific components are unavailable or inadvertently substituted. These errors may lead to infection, medication errors, and serious adverse events. As a result, applicability of these devices is limited. To fully realize the benefits of large volume administration in the home setting, there is a need for simple, error-proof, safe, and intuitive delivery devices suitable for use by patients who are not trained healthcare providers.

While SC administration is highly preferred by pharmaceutical companies and patients, not all medicines are readily transitioned from IV-to-SC administration. Bioavailability is determined through in-human clinical trials, is molecule-specific, and generally lower for the SC route versus the IV route. For the same molecule, larger SC volumes are likely required to provide equivalent bioavailability compared to IV delivery. However, these volumes may exceed the capacity of current large-volume SC devices, such as on-body injectors (OBIs), which are supplied in fixed volume increments, such as 3mL, 5mL, 10mL, 25mL, and 50mL. Should volume requirements exceed available OBI devices, or require customization of an OBI, follow-on clinical trials or commercial launch of medications using the OBI may be delayed.

Individual medications are often part of a larger regimen of medicines, with standardized regimens corresponding to a specific disease state, treatment regimen, or medication. In a clinic setting, order sets contain all the information required to administer a standardized regimen. For example, an oncology regimen might include pre-medications, oncology treatments, and post-medications, all contained in an order set. Existing drug delivery devices are designed to administer a single medication and cannot support delivery of multi-medication regimens, limiting the ability to move therapy from the clinic to the home setting. There are no delivery devices that can detect and respond to a suspected infusion reaction, making administration of certain medications currently infeasible in the home setting and confining these medications to in-clinic delivery.

Furthermore, medication order sets may direct clinical staff to perform specific patient monitoring and permit contingent administration of emergency medication. This is particularly important for medications that cause infusion-related reactions in certain patients. Infusion reactions are potentially fatal, systemic reactions related to mode of action of the medication. Systemic infusion reactions are clinically distinct from localized injection site reactions or erythema from administration of a single agent such as would occur with an autoinjector, prefilled syringe, or OBI device, which are uncomfortable but not life-threatening. They demand an immediate halt to medication administration and administration of one or more counteracting medications. However, prior art devices neither allow detection of systemic infusion reactions nor delivery of emergency medication and cannot be safely used to administer medications where systemic infusion reactions could occur. This is a particular concern for biologic therapies and is especially relevant to oncology treatments.

In the clinic setting, administration of a medication regimen, associated monitoring, and clinical decision-making are documented in the patient's record within an electronic health record (EHR) system. The purpose of the EHR is to provide a complete clinical record of care for a patient, and safely manage medication regimens without relying on human memory or introducing human error. Healthcare providers update and review the EHR system in real-time for a given patient. Current drug delivery devices for home use do not have EHR interfaces, preventing their use with multi-medication regimens, contingent medication administration, or specific patient monitoring requirements. Moreover, administration of medication via other drug delivery devices, such as OBIs, may not be reflected in an EHR system.

In the clinic setting, EHR systems also provide vital patient safety functions. EHR systems ensure patients may safely receive certain medications based on physical vital signs, laboratory testing values, or administration of prior medications as scheduled. However, prior art delivery devices used in the home setting are focused on a single medication, lack integration into EHR systems, and thus cannot provide safety interlocks that are present in the clinic. As a result, present devices cannot prevent administration of medications in unsafe conditions.

Accordingly, there is a need for allowing administration of other medications before, during, and after the therapeutic medication, even if outside the clinic setting. There is also a need for drug delivery systems which do not impose arbitrary volume restrictions or "breakpoints" upon the drug development process and decouple formulation development and clinical trials from delivery device, apparatus and system design. Furthermore, there is a need for drug delivery devices, apparatus and systems that are configured for detecting system infusion reactions through specific sensors, arresting delivery of a medicine, and administering one or more emergency counteracting medications. There is also a need for apparatus, systems and methods that provide EHR integration, advance the art of drug delivery devices, apparatus and systems by allowing home delivery of complex regimens as ordered, updating administration in a patient's record, and allowing healthcare providers to review a complete regimen history for a patient without extra effort. There is also a need to provide apparatus, systems and methods that allow integration with an EHR system and only allowing administration of medications under safe conditions, replicating the safety measures at home that are currently present in clinic settings.

US 2006/224141 A1 discloses a medication infusion device intended for the treatment of diabetes which administers a single type of fluid (insulin) and adjusts the speed of administration based on measured analyte (glucose) level.

### SUMMARY

Embodiments of the disclosure provide apparatus, systems and methods for administering large volumes of parenteral or enteral medicines to a patient via a tubing set. The apparatus or system enables different configurations of tubing sets to effectuate medication administration to a patient.

In one or more embodiments, the tubing sets described herein are used with a needle assembly configured with one or more sensors placed on a patient's skin, and a large volume medication delivery system that is located off the patient's body, containing one or more medications, a fluid pump, and a controller. Medication is delivered through one or more lumens provided in the tubing set, which fluidically connects the drug delivery device and patient needle assembly. Before, during, and after medication administration, the sensors allow the drug delivery system to monitor the physiologic status of a patient receiving the medication. In one or more embodiments, the tubing sets are also provided with a series of internally or externally situated conductors that enable electrical or optical communication between the needle assembly and a medication delivery device. In one or more embodiments, the conductors may comprise an electrical or optical conductor. In one or more embodiments, the externally situated conductors may comprise an electrically conductive printed ink.

One or more embodiments of the disclosure are directed to providing apparatus, systems and methods comprising communication systems and methods for drug delivery systems used in the home setting that are configured to communicate sensor data from the patient to the controller of a medication delivery system. One or more embodiments of the disclosure are directed to providing a more reliable, direct connection between a medication delivery system and a sensors within a needle assembly. In one or more embodiments, communication between the drug delivery system controller and needle assembly is used by the controller to measure a patient's physiologic status as reported by the sensor.

Additional embodiments of the disclosure are directed to providing apparatus, systems, and methods comprising powering systems and methods for drug delivery systems used in the home setting that are configured to power sensors located on a patient to allow continuous monitoring of physiologic parameters before, during, and after administration of one or more therapeutic medications. One or more embodiments of the disclosure are directed to providing drug delivery system configured with a controller with a more reliable, direct powering of sensors within a needle assembly. In one or more embodiments, communication between the drug delivery system controller and needle assembly is used to power the sensor in the needle assembly.

Further embodiments of the disclosure are directed to providing a medication delivery apparatus used for administration of complex medication regimens with a single feedback state as a consolidation of the system state, enabling simple, easily interpreted feedback to a user of the apparatus. In one or more embodiments, a drug delivery system controller is provided with an illuminated indicator in optical communication with a tubing set and is configured to provide a variety of visual feedback states to a user. An apparatus including a medication delivery device connected to a tubing set described herein may be provided with an illumination source proximal to the tubing set. When desired, the illumination source is piped through the tubing set or one or more optical conductors therein to use the tubing set itself as a feedback mechanism to the user. In some embodiments, use of single colors, combinations of colors, or flashing patterns may communicate one or more states to a user.

One or more embodiments of the disclosure are directed to an apparatus configured to deliver one or more therapeutic medications to a patient, the apparatus comprising a one or more reservoirs containing therapeutic medications; a patient interface configured to deliver contents of the reservoir into the body of the patient; a flexible tubing set in fluid communication with the reservoirs at the proximal end, and the patient interface at the distal end; and a fluid pump configured to expel the therapeutic medication from the reservoirs through the flexible tubing set and into the patient interface, wherein the flexible tubing set comprises a predetermined length and one or more internal medication lumens comprising a consistent internal diameter, the flexible tubing set configured to establish a specific, calibrated flow rate based on specific characteristics of the therapeutic medications passing through the internal lumen, the specific characteristics selected from the group consisting of viscosity, shear thinning behaviors, shear thickening behaviors, desired delivery time to the patient, and combinations thereof. In some embodiments the apparatus is modular. In some embodiments, the apparatus is configured to deliver the therapeutic medication to the patient at a known, preselected, and controlled flow rate. In some embodiments, the apparatus is configured to deliver the therapeutic medication to the patient at a known, preselected maximum flow rate. In some embodiments, the apparatus is configured to deliver a first medication at a known, preselected, and controlled first flow rate through a first lumen, and to deliver a second medication at a known, preselected, and controlled second flow rate through a second lumen, wherein the first flow rate is faster than the second flow rate.

Additional embodiments of the disclosure are directed to an apparatus configured to deliver a therapeutic medication to a patient, the comprising one or more reservoirs, each of the one or more reservoirs containing a therapeutic medication; one or more reservoirs containing a pre-medication to be administered before or a post-medication to be administered after the one or more therapeutic medications; a patient interface configured to expel contents of the reservoirs into the body of the patient; a flexible tubing set in fluid communication with the reservoirs at a proximal end of the flexible tubing set, and a patient interface at a distal end of the flexible tubing set; and a fluid pump to expel the therapeutic medication from each of the one or more reservoirs through the flexible tubing set and into the patient interface, wherein the flexible tubing set is provided with predetermined length and internal lumen of consistent internal diameter to provide a specific, calibrated flow rate based on characteristics of the therapeutic medications passing therethrough, the characteristics selected from the group consisting of viscosity, shear thinning behaviors, shear thickening behaviors, desired delivery time to the patient, and combinations thereof.

Further embodiments are directed to an apparatus configured to deliver one or more therapeutic medications to a patient, the apparatus comprising one or more reservoirs containing one or more therapeutic medications; an emergency reservoir containing an emergency medication; a patient interface configured to expel contents of the one or more reservoirs and the emergency reservoir into the body of the patient; and a flexible tubing set in fluid communication with the one or more reservoirs at a proximal end of the flexible tubing set, and the patient interface at a distal end of the flexible tubing set, wherein the flexible tubing set is provided with predetermined length and internal lumen of consistent internal diameter configured to provide a specific, calibrated flow rate based on characteristics of the therapeutic medications passing therethrough, the characteristics selected from the group consisting of viscosity, shear thinning behaviors, shear thickening behaviors, desired delivery time to the patient and combinations thereof.

Further embodiments are directed to an apparatus configured to deliver one or more investigational medicines during a clinical trial at one or more controlled flow rates, the apparatus comprising one or more reservoirs, each of the one or more reservoirs containing an investigational therapeutic medication; a patient interface configured to deliver contents of the reservoirs into the body of the patient; a flexible tubing set in fluid communication with the one or more reservoirs at a proximal end of the flexible tubing set, and the patient interface at a distal end of the flexible tubing set; and a fluid pump configured expel the investigational therapeutic medication from the reservoir through the flexible tubing set and into the patient interface, wherein each of several the flexible tubing sets is provided with a predetermined length and an internal lumen of a consistent internal diameter to provide a specific, calibrated flow rate based on characteristics of the investigational therapeutic medications passing therethrough, the characteristics selected from the group consisting of dose, concentration, viscosity, shear thinning behaviors, shear thickening behaviors, desired delivery time to the patient and combinations thereof, the characteristics corresponding to one or more clinical trial study conditions.

Another aspect of the disclosure is directed to a method for delivering an investigational therapeutic medication to a patient at one or more controlled flow rates during a clinical trial of an investigational medicine, the method comprising providing a clinical trial kit comprising an investigational therapeutic medication, a reservoir, a fluid pump, and one or more flexible tubing sets, each of the one or more flexible tubing set corresponding to a specific controlled flow rate for a specific investigational therapeutic medication and associated with one or more clinical trial conditions; selecting a selected flexible tubing set from the one or more flexible tubing sets corresponding to an individual patient's clinical trial condition, as specified in a clinical trial protocol or randomization schedule; attaching a proximal end of the flexible tubing set to the fluid pump to establish fluid communication with the fluid pump; attaching a distal end of the flexible tubing set to a patient interface; and administering an investigational therapeutic medication to the patient.

In another embodiment of a method, a method of providing an optimized tubing set for delivery to a patient a therapeutic medication exhibiting substantially non-Newtonian characteristics delivered by a single pump unit at one or more known, preselected, and controlled flow rates is provided. The method comprises identifying one or more desired flow rates of the therapeutic medication for administration to a patient based on desired pharmacokinetics of the therapeutic medication; identifying one or more ambient temperatures at which delivery of the therapeutic medication will occur; conducting testing to identify a relationship between temperature, viscosity, and concentration of the therapeutic medication in a pharmaceutical formulation for delivery to the patient; specifying values of an internal diameter, a length, and interior surface roughness of an experimental tubing set associated with one or more of the desired flow rates, based on one or more of theoretical calculations and computational fluid dynamic analysis; characterizing a force required to propel the therapeutic medication exhibiting non-Newtonian characteristics through the experimental tubing set; experimentally determining a required fluid pump power to dispense the therapeutic medication within the experimental tubing set at a plurality of temperatures and flow rates; adjusting the values of the experimental tubing set to accommodate an observed flow rate versus a desired flow rate and selecting the optimized tubing set; and confirming the desired flow rate through the optimized tubing set.

Further aspects concern tubing sets and apparatus comprising these tubing sets, as set out in more detail in the description below.

### BRIEF DESCRIPTION OF THE DRAWINGS

Figure 1A shows a simplified partial cutaway front view diagram showing anatomic location of patient interface components to effectuate intravenous medication delivery using four common vascular access devices (VADs) featuring terminating luer taper connections in accordance with one or more embodiments;
Figure 1B shows a simplified partial cutaway front view diagram showing anatomic location of patient interface components to effectuate intravenous medication delivery using an implanted vascular access device (VADs) or "port" and Huber needles in accordance with one or more embodiments;
Figure 1C shows a simplified partial cutaway front view diagram showing anatomic location of patient interface components to effectuate subcutaneous and intramuscular administration using a variety of straight in and angled needle placements in accordance with one or more embodiments;
Figure 1D shows a simplified partial cutaway front view diagram showing anatomic location of patient interface components to effectuate placement of a soft, flexible administration cannula and provide subcutaneous and intramuscular administration in accordance with one or more embodiments;
Figure 2A shows a block diagram of selected functional components implemented in the drug delivery apparatus to deliver three medications in accordance with one or more embodiments;
Figures 2B-1 through 2B-5 show block diagrams of selected functional components implemented in the drug delivery apparatus to deliver combination therapy of several medications, illustrating exemplary administration sequences and time-delays based on regimen requirements, in accordance with one or more embodiments;

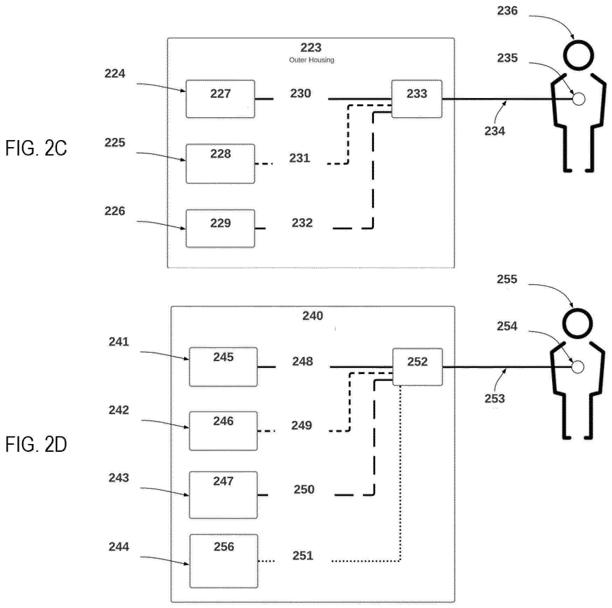

Figure 2C shows a block diagram of selected functional components implemented in the drug delivery apparatus to deliver a therapeutic medication preceded and/or succeeded by certain other medications as part of a full medication regimen in accordance with one or more embodiments;
Figure 2D shows a block diagram of selected functional components implemented in the drug delivery apparatus to deliver a medication of interest as well as various flushing solutions in accordance with one or more embodiments;

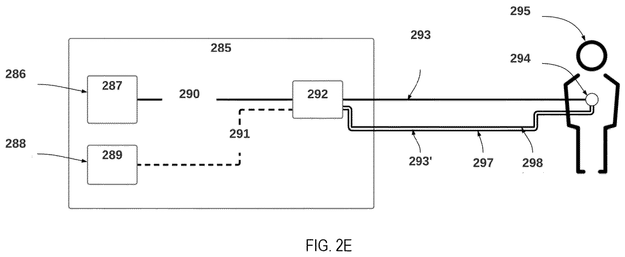

Figure 2E shows a block diagram of selected functional components implemented in the drug delivery apparatus to deliver a medication of interest and contingently administer an emergency medication to counteract a systemic infusion reaction in accordance with one or more embodiments.

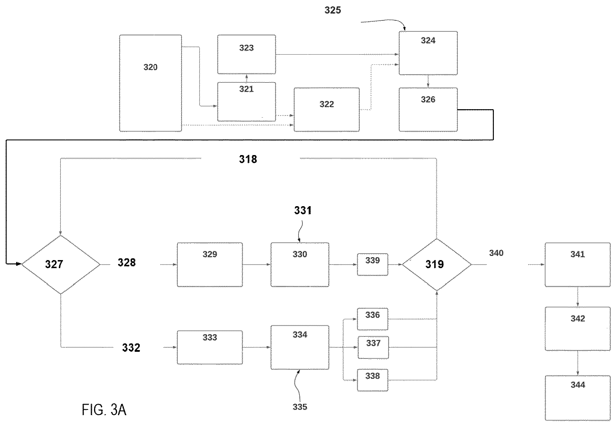

Figure 3A shows a flow diagram of a clinical trial study process, illustrating how the present disclosure is integrated therein in accordance with one or more embodiments.

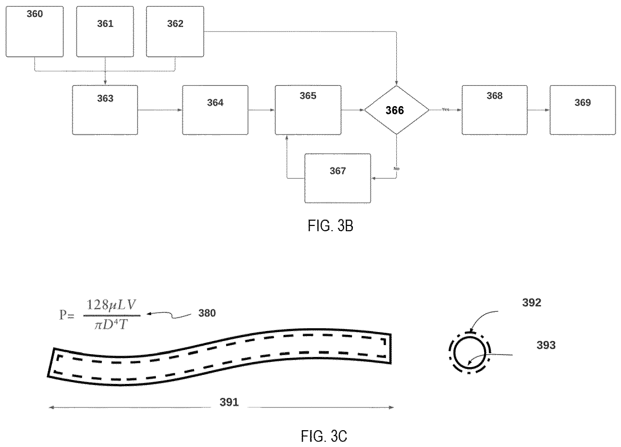

Figure 3B shows a flow diagram of a process within one embodiment to design and refine a tubing set to deliver a non-Newtonian therapeutic medication at one or more rates based on formulation characteristics, expected pharmacotherapeutic effect, and expected dosing regimens studied as part of a human clinical trial.
Figure 3C shows a schematic diagram of the governing parameters to design and refine a tubing set to deliver a substantially non-Newtonian therapeutic medication given formulation characteristics in accordance with one or more embodiments.

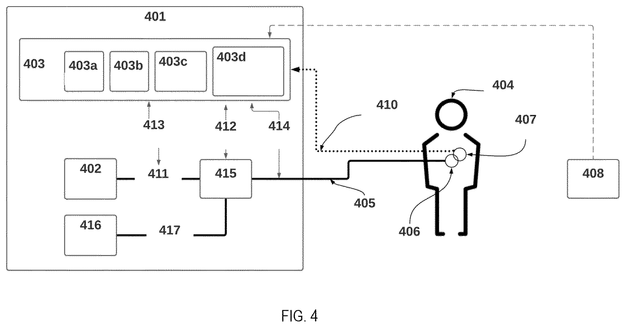

Figure 4 shows a block diagram of selected functional components implemented in the drug delivery apparatus to provide closed-loop monitoring of patient status in order to detect a systemic infusion reaction, and allow one or more appropriate clinical responses to the systemic infusion reaction in accordance with one or more embodiments.

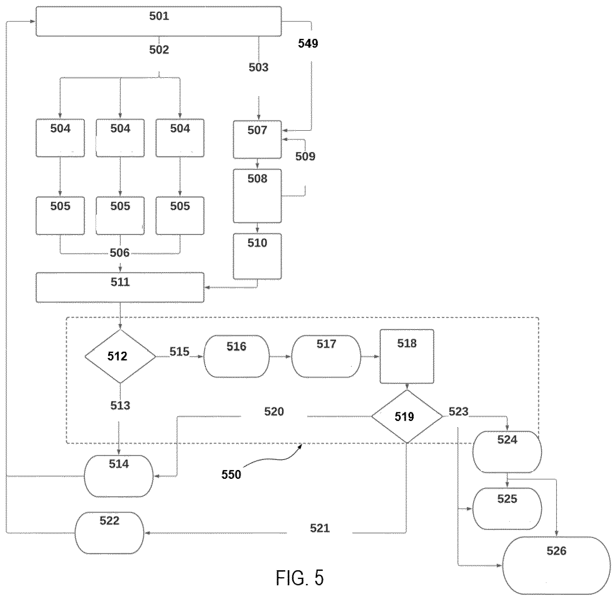

Figure 5 shows a flow diagram of a process of one embodiment for detecting and responding to a patient infusion reaction during or after administration of one or more therapeutic medications.

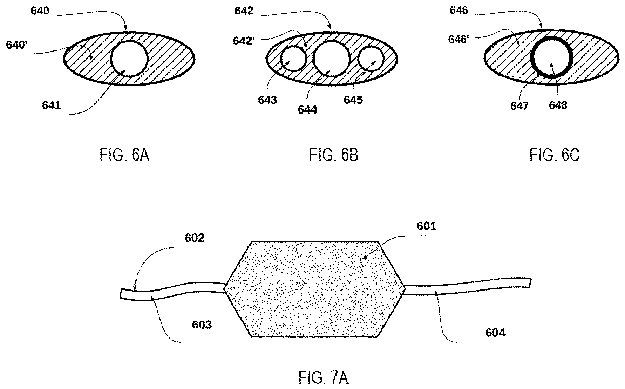

Figures 6A-C show a cross-sectional diagram of tubing sets and medication lumens in accordance with one or more embodiments.

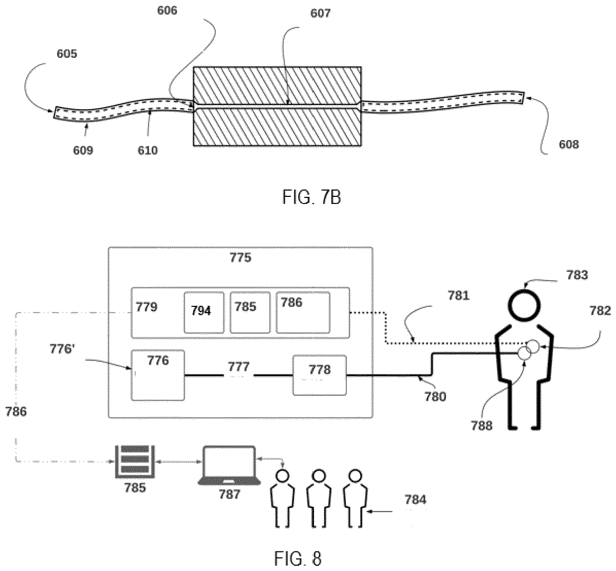

Figures 7A-B illustrate a portion of a tubing set containing an inline filter and flow restrictor in accordance with one or more embodiments.
Figure 8 shows a block diagram of selected functional components implemented in the drug delivery apparatus to provide clinical study data integrity for an investigational therapeutic medication in accordance with one or more embodiments.

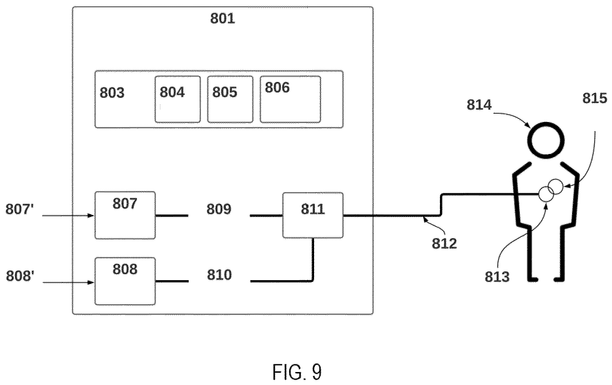

Figure 9 shows a block diagram of selected functional components to deliver one or more therapeutic medications to a patient.

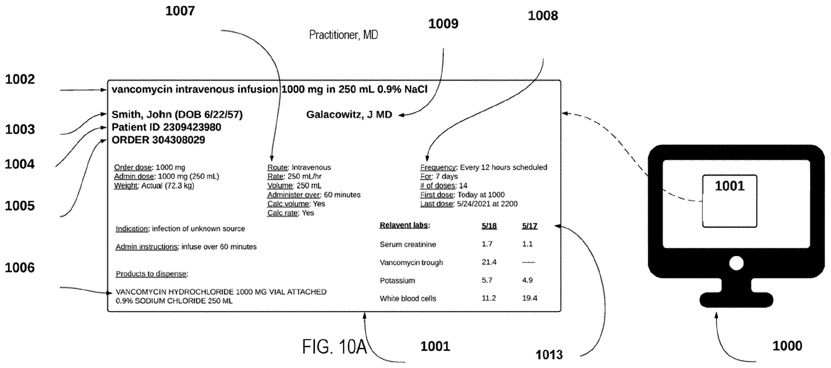

Figure 10A is a representative example of information in a medication order for a single medication contained within an electronic health record system.

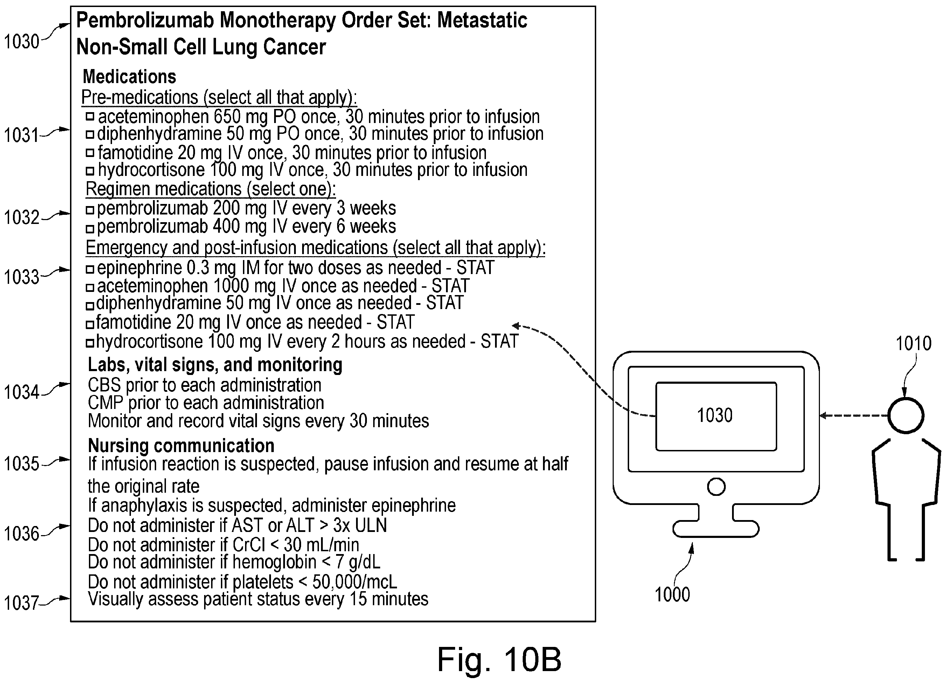

Figure 10B is a representative example of information in a medication order set contained within an electronic health record system for administration of a medication regimen, including a variety of medication administration and other care instructions for a patient.

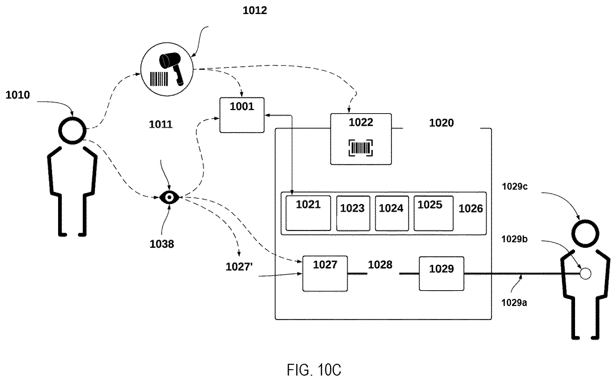

Figure 10C shows a block diagram of selected functional components implemented in the drug delivery apparatus to provide association and verification of a drug delivery apparatus against a medication order in accordance with one or more embodiments.

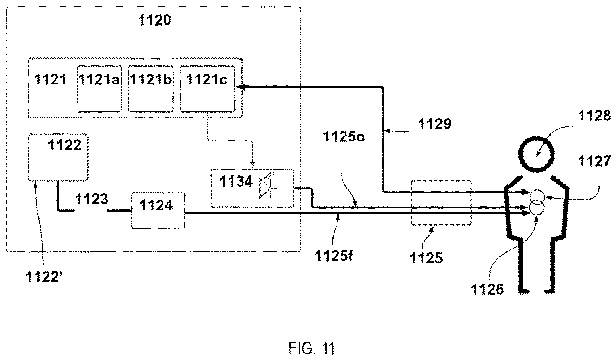

Figure 11 shows a block diagram of selected functional components of a medication delivery apparatus or system incorporating the tubing set described herein in one or more embodiments.

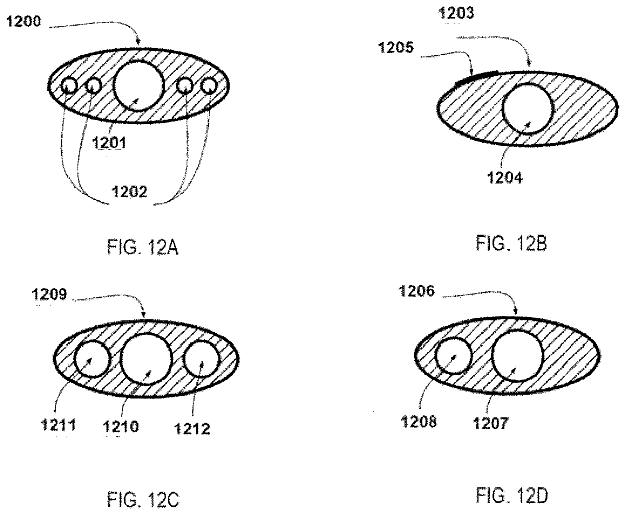

Figures 12A-I show cross-sectional views of selected tubing sets and conductors in accordance with one or more embodiments.

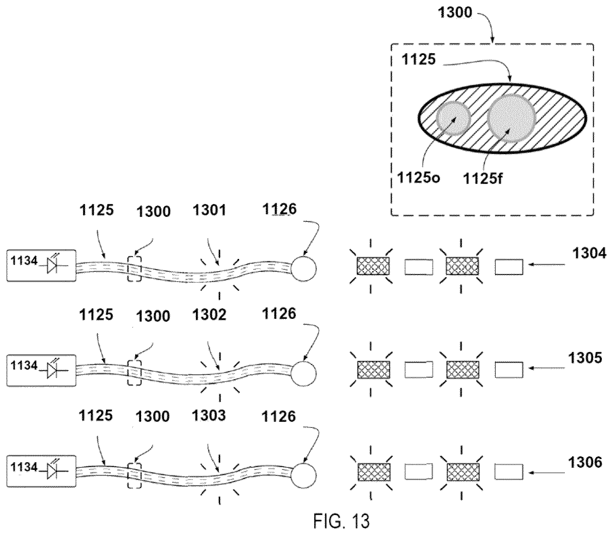

Figure 13 shows a schematic view of tubing sets providing visual feedback in accordance with one or more embodiments.

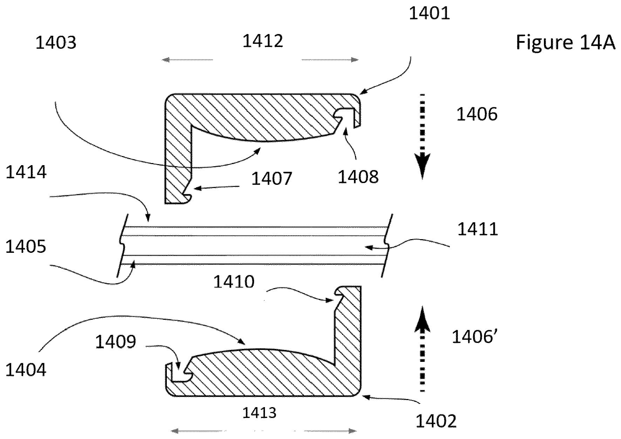

Figure 14A shows a partial cutaway side view of a constraint being oriented to a tubing set in accordance with an embodiment of the disclosure.

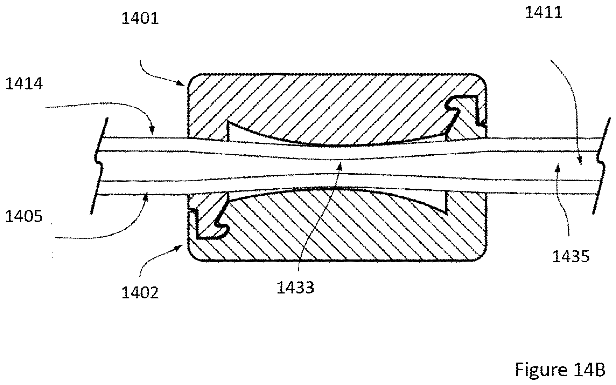

Figure 14B shows a partial cutaway side view of a constraint placed on a tubing set in accordance with an embodiment of the disclosure.

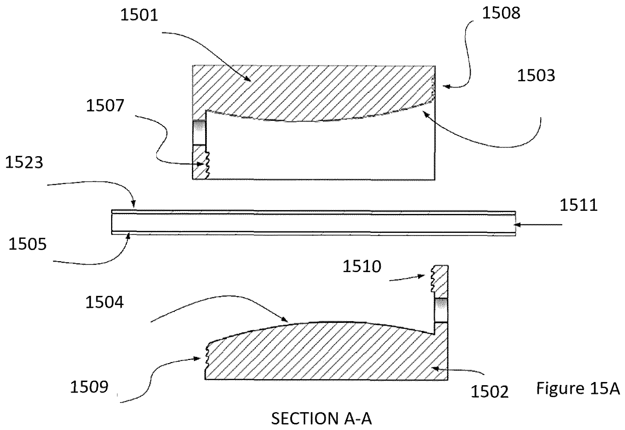

Figure 15A shows a cross-sectional view of an adjustable constraint apparatus, configured to provide multiple discrete degrees of constraint to a tubing set in accordance with an embodiment of the disclosure.

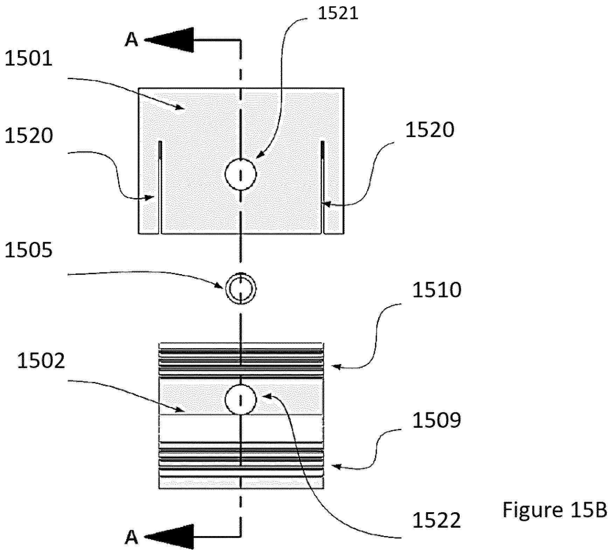

Figure 15B shows an end view of an adjustable constraint apparatus, configured to provide multiple discrete degrees of constraint to a tubing set in accordance with an embodiment of the disclosure.

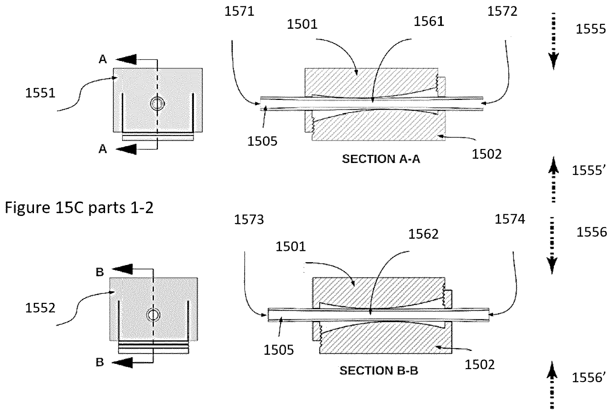

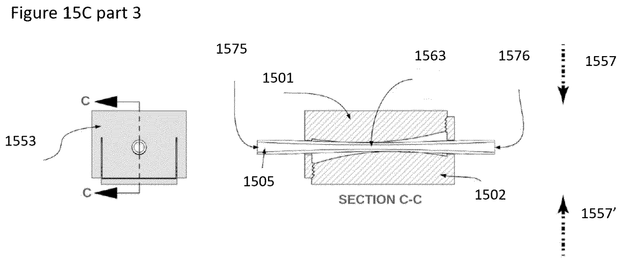

Figure 15C shows an end and cross-sectional view of an adjustable constraint apparatus, illustrating progressive assembly of the device through three levels of successively larger constraint to a tubing set in accordance with an embodiment of the disclosure.

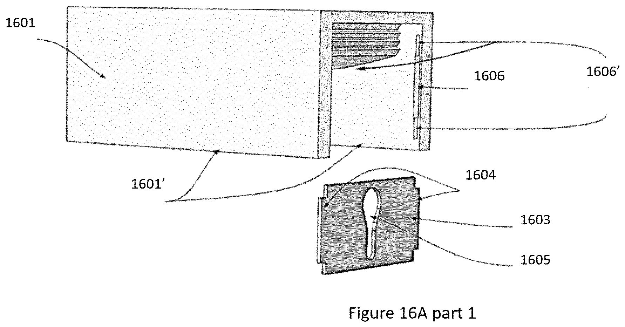

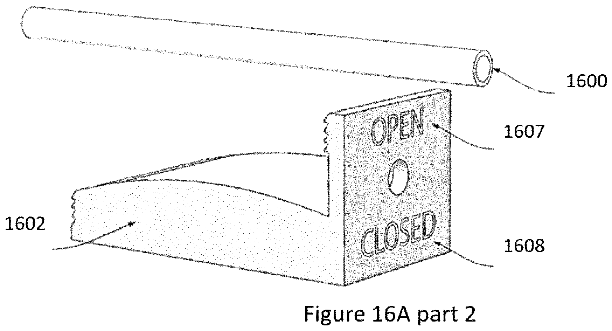

Figure 16A shows a perspective exploded view of an adjustable constraint apparatus provided with an emergency tubing set shutoff clamp and configured to provide multiple discrete degrees of constraint to a tubing set in accordance with an embodiment of the disclosure.

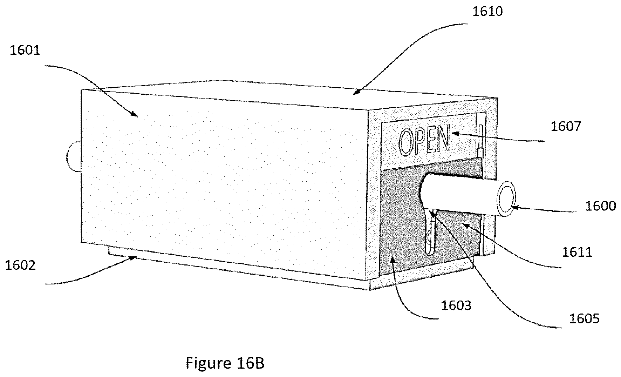

Figure 16B shows a perspective view of the assembled components of Figure 16A and the shutoff clamp in a first of two discrete positions.

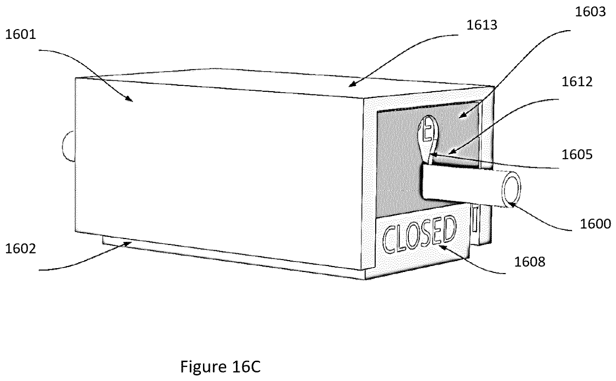

Figure 16C shows a perspective view of the assembled components of Figure 16A and the shutoff clamp in a second of two discrete positions.

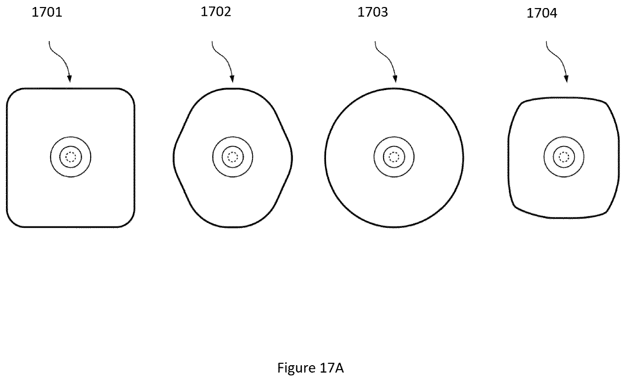

Figure 17A shows an end view of several constraints assembled onto tubing sets, illustrating a variety of shapes in accordance with one or more embodiments of the disclosure.

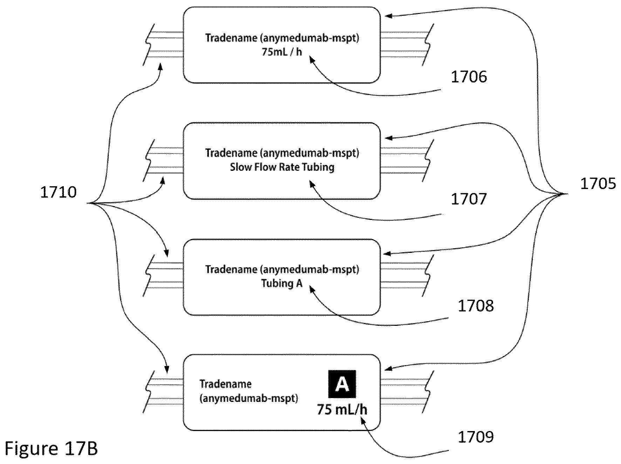

Figure 17B shows a top view of several constraints assembled onto tubing sets and visible indicia after assembly, in accordance with one or more embodiments of the disclosure.

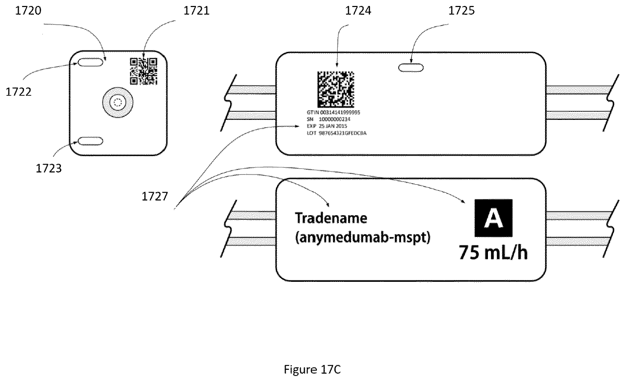

Figure 17C shows a side view, top view, and bottom view of a constraint assembled onto a tubing set and visible and machine readable indicia after assembly, in accordance with one or more embodiments of the disclosure.

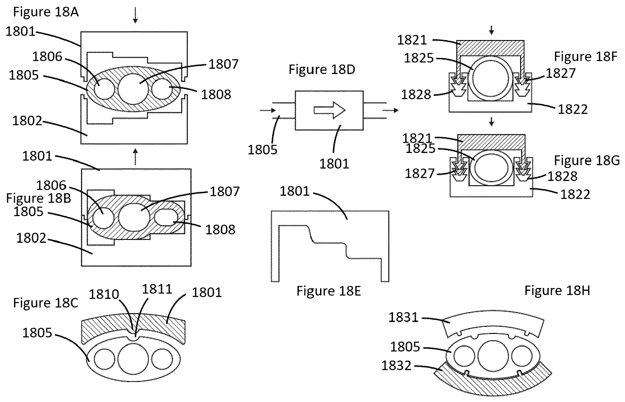

Figures 18A, 18B, 18C, 18D, 18E, 18F, 18G, 18H, 19A, 19B, 19C, 20A and 20B show various solutions for compression of a tube.

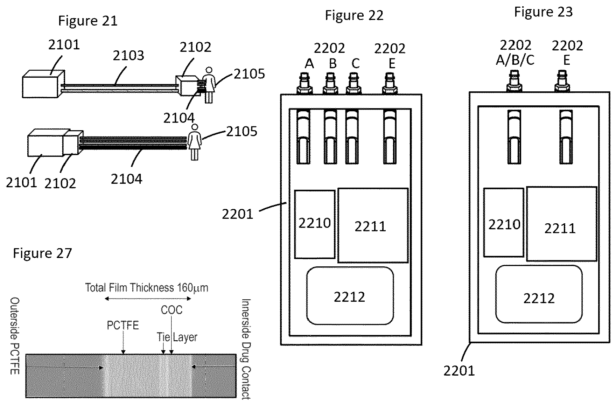

Figure 21 shows two configurations of a medicament delivery device.
Figures 22 and 23 shows example configurations of a powerpack.

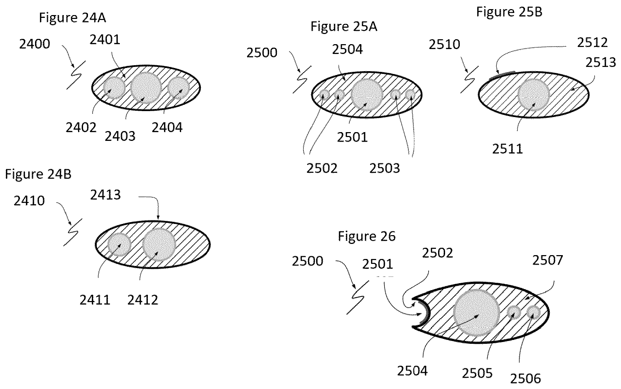

Figures 24A, 24B, 25A, 25B and 26 show example tubing set configurations.
Figure 27 shows an example tubing structure.

### DETAILED DESCRIPTION

Before describing several exemplary embodiments of the disclosure, it is to be understood that the disclosure is not limited to the details of construction or process steps set forth in the following description. The disclosure is capable of other embodiments and of being practiced or being carried out in various ways.

The design and use of large volume medication delivery apparatus and systems raise new challenges. The characteristics of medications are often different than those administered with existing devices. Some medications administered in larger volumes may trigger systemic infusion reactions during or after administration. These infusion reactions are potentially fatal, systemic reactions related to mode of action of the medication and are distinct from localized injection site reactions or erythema from administration of a single agent with a prefilled syringe, autoinjector, or on-body injector, which are uncomfortable but not life-threatening. Such apparatus and systems may require an immediate halt to medication administration and administration of one or more counteracting emergency medications, for example, epinephrine.

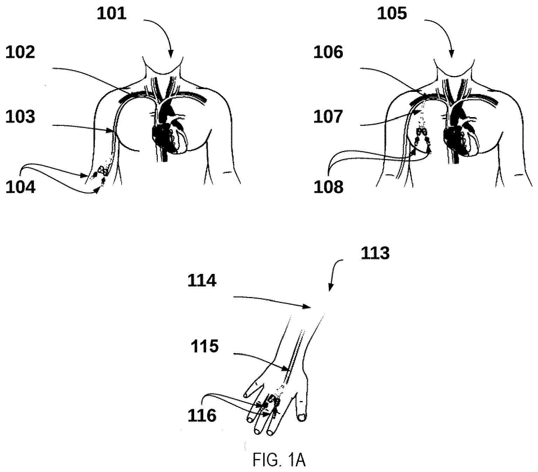

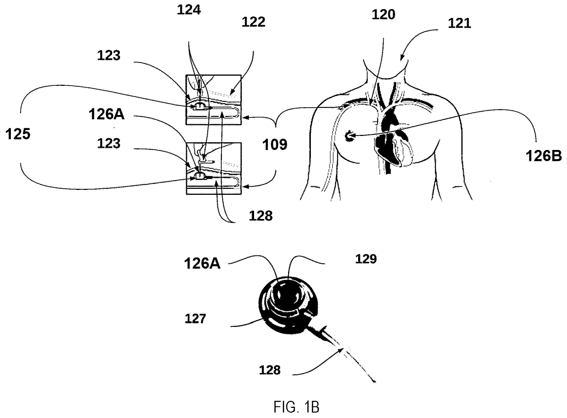

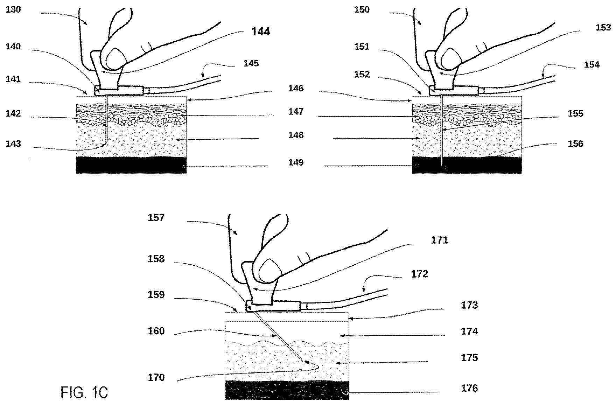

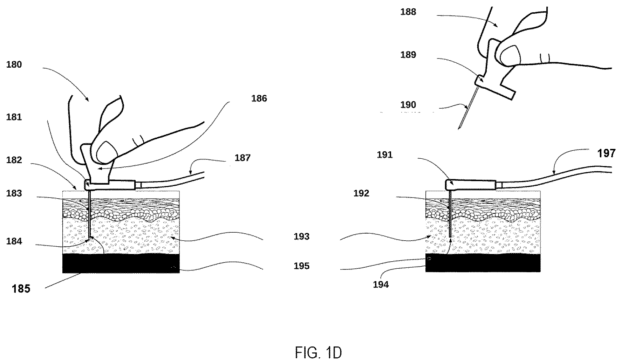

Each of the embodiments described with respect to Figures 11-13 may be combined with each of the embodiments described in Figures 1-10C and the corresponding claims.

Figure 11 illustrates an exemplary large volume medication delivery system comprising an outer housing 1120, and one or more reservoirs 1122' for one or more therapeutic medication(s) 1122 fluidically connected 1123 to a fluid pump 1124, by which the reservoirs 1122' may be emptied by the fluid pump 1124 to administer the medication 1122 to the patient 1128 by way of the tubing set 1125 disclosed herein and patient interface 1126.

The medication delivery system is also provided with a controller 1121 that communicates with components of the apparatus via either a wired or wireless connection. In one or more embodiments, the controller according to one or more embodiments comprises a processor 1121a, a memory 1121b coupled to the processor 1121a, input/output devices 1121c coupled to the processor 1121a, and support circuits to provide communication between the different components of the system, namely the components of the system described herein. In one or more embodiments, processes to operate the system are stored in the memory 1121b as a software routine that, when executed by the processor, causes the system to perform methods described in the present disclosure. In one or more embodiments, the processes to operate the system are performed in hardware. In one or more embodiments, the software routine to operate the system may also be stored and/or executed by a second processor that is remotely located from the hardware being controlled by the processor.

In one or more embodiments, the tubing set 1125 is provided with one or more medication lumens 1125f by which medication 1122 is instilled by fluid pump 1124 into the patient 1128 through the patient interface 1126. Said patient interface 1126 can comprise a luer-taper or Luer-Lok^{®} fitting if a luer-activated IV connector is used, or a variety of needle assemblies corresponding to an intravenous, subcutaneous, or intramuscular route. Sensors 1127 are located on a patient, either separately or integral to the patient interface 1126. In one embodiment, patient interface 1126 may comprise a subcutaneous, intramuscular, or intravenous needle set with integral sensors 1127.

In one or more embodiments, tubing set 1125 is also provided with one more conductors 1129 by which the sensors 1127 are coupled to the controller 1121. In one or more embodiments, the tubing set 1125 is also provided with an optical conductor 1125o which may be used to provide one or more visual feedback indicators to a patient 1128 or another user of the device.

Figures 12A-I illustrate exemplary embodiments of medication lumens and conductors according to one or more embodiments. Although certain arrangements of medication lumens and conductors are depicted in Figures 12A-I, it will be apparent to those skilled in the art that many arrangements of medication lumens and conductors are within the scope of the present disclosure, and that the illustrative figures provided are for purposes of illustration and shall not limit the arrangements of conductors and lumens.

As seen in Figures 12A-I, in one or more alternative embodiments, a tubing set 1200 is provided with one or more medication lumens 1201 and one or more internal conductors 1202. In one or more alternative embodiments, a tubing set 1203 is provided with one or more internal medication administration lumens 1204 and one or more external conductors 1205, wherein the one or more conductors 1205 are substantially situated on the exterior of the tubing 1203. Said conductors 1202, 1205 enable coupling and communication between a controller and one or more sensor(s), while said lumens 1201, 1204 enable medication administration. Conductors 1202, 1205 are situated in a parallel manner to medication lumens 1201, 1204 over the length of the tubing set, which may be varied based on the delivery devices and patient interfaces used.

In one or more alternative embodiments, a tubing set 1206 is provided with one or more internal medication lumens 1207 and one or more internal optical conductors 1208. Optical conductors 1208 may be used as described herein to provide visual feedback to one or more users of a medication delivery device incorporating tubing set 1206.

In one or more embodiments, a tubing set 1209 is provided with one or more internal medication lumens 1210, one or more internal optical conductors 1211, and one or more conductors 1212. Optical conductors 1211 may be used to provide visual feedback to one or more users of a medication delivery device, while conductors 1212 may be used to communicate sensor data from a patient interface to a medication delivery system controller, both as described herein. incorporating tubing set 1206. In an alternative embodiment, conductors 1212 are used to power sensors within the patient interface.

In an alternative embodiment, a tubing set 1220 with one or more external conductors 1222 is provided with an interposing barrier coating 1223 to isolate medication within one or more fluid lumens 1221 from potential contaminant leachable or extractable compounds from the conductors 1222 or the conductor application process.

In an alternative embodiment, a tubing set 1225 with one or more external conductors 1226 and one or more medication lumens 1227 is provided with an interposing barrier coating 1228 situated between the medication lumen 1227 and tubing set 1225 material, to isolate medication within one or more fluid lumens 1221 from potential contaminant leachable or extractable compounds from the external conductors 1226 or the conductor application process. In one embodiment, the barrier coating 1223, 1228 comprises a PTFE or other fluoropolymer material. In another embodiment, the barrier coating 1223, 1228 is co-extruded as the tubing set is manufactured.

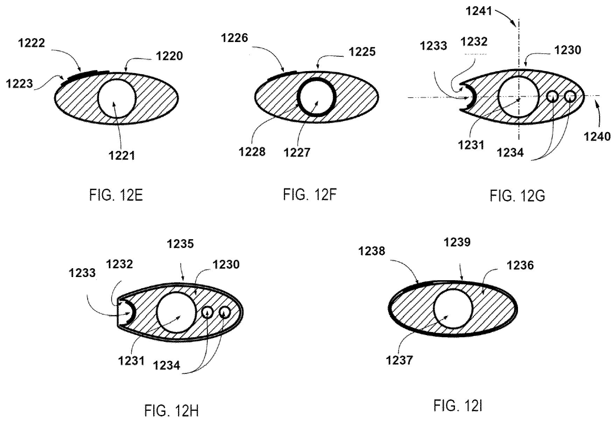

Referring to Figures 12G and 12H, in one or more alternative embodiments, a tubing set 1230 is provided with one or more internal medication lumens 1231, an undercut 1232, and one or more external conductors 1233 situated in said undercut 1232, to protect said external conductors 1233 from damage, for example from chafing or friction. In some embodiments, one or more undercuts 1232 may be provided in the tubing set 1230 to accommodate and protect additional external conductors 1233. In some embodiments, the tubing set 1230 may have a combination of one or more external 1233 and internal conductors 1234. The undercut is a recess (for example a groove).

In some embodiments, the tubing set 1230 comprises an asymmetric cross-section with differing bending stiffness about a first axis 1240 and a second axis 1241 orthogonal to the first axis 1240. In some embodiments, one or more undercut features is 1232 situated on an exterior contour of the tubing set 1230 and oriented on a cross-sectional axis with a higher degree of bending stiffness to protect said external conductors 1233 from damage, for example from bending, fatigue, or stress cracking.

In some embodiments, the tubing set 1230 may be provided with an outer protective sheath 1235 substantially enclosing the undercut 1232 and part or all of the outer surface of the tubing set 1230 to protect said conductors from damage, such as by friction or chafing. In some embodiments, the material of tubing set 1230 has a lower flexural stiffness compared to that of the protective sheath 1235 protecting the external 1233 and or internal conductors 1234 from abrasion and damage due to excessive bending. In some embodiments, the tubing set 1236 may be provided with one or more external conductors 1238 and an outer protective sheath 1239 covering part or all of the outer surface of the tubing set 1236 to protect said external conductors 1238 from damage, such as by friction or chafing. In one embodiment, protective sheaths 1235, 1239 are applied after manufacturing of the tubing set and application of one or more external conductors 1233, 1238. In one embodiment, protective sheaths 1235, 1239 comprise a textile material to provide a patient with additional privacy or discretion during medication administration.

The tubing set and barrier coating(s), if present, may be fashioned from one or more of silicone, PVC, PVC without DEHP, EVA, HDPE, LDPE, TPU, PTFE, fluoropolymer, or other suitable flexible material. In some embodiments, the tubing set is manufactured from a flexible polymer that is an electrical insulator. Tubing sets of some embodiments are extruded but may be formed by other means that provide sufficient dimensional and tolerance control on the inner medication lumens as described herein. In one or more embodiments, the tubing material is chosen to be a material selected for low leachable and extractable compounds that may contaminate a medication, and that exhibits high biocompatibility with biologic medications.

The tubing set and barrier coating(s), if present, may be fashioned from one or more of silicone, COC, COP, PVC, PVC without DEHP, EVA, HDPE, LDPE, TPU, PTFE, PCTFE, fluoropolymer, or other suitable flexible material. In some embodiments, one or more tie layers may be provided to allow bonding of two or more materials comprising the tubing set, such as a drug contacting material and a barrier coating. In some embodiments, the tubing set is manufactured from a flexible polymer that is an electrical insulator. Tubing sets of some embodiments are extruded but may be formed by other means that provide sufficient dimensional and tolerance control on the inner medication lumens as described herein. In one or more embodiments, the tubing material is chosen to be a material selected for low leachable and extractable compounds that may contaminate a medication, and that exhibits high biocompatibility with biologic medications. In one or more embodiments, the tubing set material comprises a COC drug contacting layer on the innerside and PCTFE film (for example from Aclar) on the outerside, bonded through an intermediary tie layer, as shown in Figure 27. The 160-micron thickness as shown in Figure 27 is exemplary, and could be varied.

In some embodiments, one or more conductors are manufactured from an electrical conductor, such as carbon, copper, nickel, or silver. In some embodiments, the conductors comprise a conductive ink. In one or more embodiments, the external conductors comprise a conductive ink. The material comprising the conductive ink may include a solution of metal nanoparticles. The conductive ink may be applied to tubing set during manufacture of the tubing or as a separate secondary operation. The conductive ink may be applied by flexographic, screen, inkjet, or stencil printing. In one or more alternative embodiments, the externally situated conductors comprise a conductive polymer. In one or more alternative embodiments, the conductive polymer is selected from one or more of polyacetylene, polythiophene, poly[3,4-(ethylenedioxy)thiophene], polypyrrole, polyaniline, or polyphenylene. In one or more alternative embodiments, the externally situated conductors 654 comprise an electrodeposited film. In one or more embodiments, the electrodeposited film is a polypyrrole-polyaniline composite conductive film.

In some embodiments, one or more conductors are optical conductors, comprising an optical fiber, silica fiber bundle, polymer, or flexible polymer tube and liquid core combination. In some embodiments, one or more conductors are optical conductors that can transmit light wavelengths outside the human visible spectrum. In some embodiments, one or more conductors are optical conductors that can transmit light wavelengths within the human visible spectrum.

In some embodiments, the tubing sets are sterilized using a low-energy method, such as ethylene oxide gas or vaporized hydrogen peroxide, to preserve electrical or optical conductor continuity. In some embodiments, the tubing sets are sterilized using a high-energy method, such as gamma irradiation or electron beam irradiation, configured in a manner to preserve electrical or optical conductor continuity.

In one or more embodiments of the disclosure herein, tubing sets may also be enabled to provide feedback to a user on the status of the medication delivery system. Feedback to the user may include, for example, confirmation of proper setup, readiness to administer medications, progress of one or more medication administrations, an error in configuration prior to administration, an error during administration of one or more medications, or completion of medication administration. Those skilled in the art will appreciate the wide variety of user feedback that may be provided to one or more users of a medication delivery system based on the user population, medication regimen, and clinical characteristics.

In one or more embodiments of the disclosure herein, tubing sets may also be enabled to provide feedback to a user on the status of the medication delivery system. Feedback to the user may include, for example, confirmation of proper setup, readiness to administer medications, progress of one or more medication administrations, an error in configuration prior to administration, an error during administration of one or more medications, or completion of medication administration. Feedback to a user may include start of dose, end of dose, or in-process feedback, as is customary with autoinjector devices, or may be specific to the nature or configuration of the device. For instance, in a device with multiple needle sets or tubing lumens, feedback related to confirmation of proper setup or readiness to administer medications may comprise an indicator of which needle set should be inserted and in which order, helping a user identify the appropriate needle set among many. Continuing this example, feedback related to completion of medication administration may comprise feedback indicating which needle is ready to be removed amongst a plurality of needles. Alternatively, in a device with multiple medication reservoirs, feedback related to confirmation of proper setup or readiness to administer medications may comprise an indicator of proper bag installation (i.e., fluidic and/or sensor communication) within the device. Also in the alternative, in a device with multiple needles or tubing set lumens, feedback related to confirmation of proper setup or readiness to administer medications may comprise an indicator of proper needle insertion. Feedback related to confirmation of proper setup may include, by way of example, an indication that multiple segments of an infusion set are properly connected (i.e., fluidically, electrically, optically, pneumatically), or that a needle set is properly connected to a tubing set and/or tubing set lumen. Feedback related to an error during administration of one or more medications may include, by way of example, an occlusion in the tubing set or needle, excessive (i.e., unintended, unprescribed, or unsafe) flow rate, or removal of a needle from the skin during injection. Those skilled in the art will appreciate the wide variety of user feedback that may be provided to one or more users of a medication delivery system based on the user population, medication regimen, drug delivery device configuration, and clinical characteristics.

In an alternative embodiment, referring to Figure 13, in one or more alternative embodiments of the medication delivery system, a tubing set 1125 is provided with a cross-sectional design 1300 featuring an optical conductor 1125o and a medication lumen 1125f. The tubing set 1125 is positioned so as to make optical contact with a selectively illuminated indicator 1134, housed in the medication delivery system as described elsewhere herein. When indicator 1134 is illuminated, as by a controller, light is conducted through the optical conductor 1125o in tubing 1125, enabling visual feedback 1301 over the length of the tubing set 1125 or a portion thereof.

In some embodiments, said indicator 1134 is an addressable LED which can display a plurality of different colors, enabling a variety of visual feedback states to be communicated to an end user. In some embodiments, the visual feedback comprises a visible indicator of differently colored lights 1301, 1302, 1303. In some embodiments, the visual feedback comprises a visible indicator of green, amber, and red lights 1301, 1302, 1303. In some embodiments, the visual feedback comprises a pulsed visual indicator of differently colored lights 1304, 1305, 1306. In another alternative embodiment, the visual feedback comprises a pulsed visual indicator of green 1304, amber 1305, or red 1306.

In some embodiments, intermittent light signals of color and/or flashing patterns (red flashing, red-yellow pulsing, or fast or slow flashing) may be used in place of different colors 1301, 1302, 1303. In another alternative embodiment, said indicator 1134 is pulsed in one or more feedback patterns 1304, 1305, and 1306, wherein the feedback patterns are independent of the indicator 1134 color.

In another alternative embodiment, the tubing 1125 is selectively masked in an opaque color, hiding and/or revealing one or more portions of light conducted through the optical conductor 1125o, and enabling further permutations of visual feedback to be presented to the end user. In another alternative embodiment, the tubing 1125 is selectively covered in an opaque or translucent textile material to protect a patient's privacy or provide additional discretion during medication administration.

In one or more embodiments, the tubing sets described immediately above with respect to Figures 11-13 are configured to be used or combined with the apparatus, systems and methods described with respect to Figures 1-10 and the embodiments described with respect to Figures 1-10 described below (apparatus and method for large volume medication administration). Thus, in some embodiments, the apparatus, systems and methods described with respect to Figures 1-10 further comprise the apparatus, tubing sets, methods and kits described immediately above with respect to Figures 11-13 in combination or addition to the various embodiments described below, including but not limited to the first 126 numbered embodiments.

As used herein, "infusion," "injection," and "administration" are used interchangeably, taking place by subcutaneous (SC), intramuscular (IM), intravenous (IV), or enteral routes, also terms used interchangeably. Administration route is based on a specific medication's pharmacokinetic (PK) profile, formulation components, approved regulatory labeling, individual clinical judgment, or clinical necessity.

Embodiments of the disclosure provide apparatus, system and methods for medication administration wherein the number of medications, administration order, volume, delivery time, and route of administration are independently selected. Embodiments of the apparatus, systems and methods provide a single architecture usable from initial human clinical trials in a research facility through commercial launch in a home setting after drug approval. One or more embodiments provide for use in the home setting, where the apparatus, systems and methods are intrinsically safe and intuitive for use by a patient or lay caregiver without healthcare training.

Accordingly, embodiments of the disclosure provide drug delivery apparatus, systems and methods allowing delivery of many different medications, including those historically limited to in-clinic settings, in the home in a variety of sequences, rates, and settings. As will be appreciated by one skilled in the art, there are numerous ways of carrying out the examples, improvements and arrangements of devices, apparatus and/or systems disclosed herein. Although reference will be made to the exemplary embodiments depicted in the drawings and the following descriptions, the embodiments disclosed herein are not meant to be exhaustive of the various alternative designs and embodiments that are encompassed by the present disclosure.

Embodiments of the present disclosure advance the art of drug delivery devices, apparatus or systems by allowing administration of other medications before, during, and after the therapeutic medication, even if outside the clinic setting. One or more embodiments of the disclosure do not impose arbitrary volume restrictions or "breakpoints" upon the drug development process and decouple formulation development and clinical trials from delivery device, apparatus or system design. In addition, embodiments advance drug delivery devices, apparatus or systems by allowing detection of system infusion reactions through specific sensors, arresting delivery of a medicine, and administering one or more emergency counteracting medications. One or more embodiments of the disclosure further provide apparatus, systems and methods that provide EHR integration, advance the art of drug delivery devices, apparatus or systems by allowing home delivery of complex regimens as ordered, updating administration in a patient's record, and allowing healthcare providers to review a complete regimen history for a patient without extra effort. One or more embodiments provide apparatus, systems and methods that allow integration with an EHR system and only allowing administration of medications under safe conditions, replicating the safety measures at home that are currently present in clinic settings.

Various embodiments of the disclosure are directed to improved systems or apparatus and methods configured for large volume infusion of therapeutic medicines. More particularly, embodiments provide systems, apparatus and methods comprising components configured to be combined to deliver one or more therapeutic medicines via one or more physiologic routes of administration in sufficiently large and varying volumes to achieve a desired therapeutic effect. In one or more embodiments, therapeutic medicines may also optionally include pre-, post- and emergency medication administration to effectuate a complete therapeutic regimen as ordered by a healthcare professional. In some embodiments, the components utilized are part of a kit, and may be referred to as a kit of components. The systems, apparatus and methods of one or more embodiments are used to determine the pharmacologic and physiologic effects of one or more therapeutic medicines when the characteristics are unknown, and may be then used to deliver the therapeutic medicine(s) at the desired parameters to achieve the therapeutic effect when administered in a variety of settings, such as in-clinic or at-home. In addition, the system, apparatus and methods of one or more embodiments improve usability, safety, and convenience based on the administration setting and end user of the drug delivery device, apparatus or system.

One of more embodiments of the disclosure provides new and/or improved apparatus, systems, and methods for administering large volumes of parenteral or enteral medicines to a patient. Intravenous, subcutaneous, intramuscular, and enteral administration of large volumes are provided by the disclosure herein. More specifically, One of more embodiments of the disclosure allows medications currently limited to the clinic setting to be administered at home by patients or lay caregivers, without the need for highly trained healthcare professionals or clinic visits. As a result, One of more embodiments of the disclosure is ideally suited for home administration of large volume biologics, such as monoclonal antibodies.

Embodiments described herein provide drug delivery apparatus, system or methods with a configurable plurality of medication reservoirs to administer a variety of medication regimens, including multi-drug regimens, as are common in oncology. Regimens may be administered over time in a sequential, parallel, time-delayed, or contingent manner. In one or more embodiments, the drug delivery system is provisioned with an interface to an electronic health record system and one or more medication orders or order sets, allowing administration of a multi-drug regimens and contingent medication administration based on laboratory values or physiologic monitoring. One of more embodiments of the disclosure provides home administration of more complex medication regimens that exceed the capability of existing prior art devices.

In one or more embodiments, tubing sets are provided with restricted flow rates corresponding to one or more clinical trial conditions or dosing regimens for an approved medication. One of more embodiments of the disclosure also provides both pre-approval clinical trials and commercialized medicines to be administered with the same device, apparatus or system, greatly reducing cost, time to market, and device, apparatus or system complexity.

In one or more embodiments, reservoirs may be individually designed for short- or long-term drug stability, based on the medication regimen being administered with the device, apparatus or system. Reservoirs may be filled at point of use in the home by a patient or caregiver, by a dispensing pharmacy, or by a pharmaceutical manufacturer. Optionally, the drug delivery system may be configured with intravenous flush solutions before and after administration in some embodiments.

In one or more embodiments, the drug delivery system is provisioned with a controller, algorithm, and sensors coupled to the controller to detect a patient's potentially life-threatening systemic infusion reaction. Further, embodiments of the drug delivery apparatus, system and method can administer a countervailing emergency medication in response to a systemic infusion reaction autonomously or at the direction of a remote clinician monitor, permitting home administration of medications that would otherwise be confined to in-clinic administration due to monitoring requirements and safety considerations. Moreover, in one or more embodiments, the drug delivery system is configured to deliver prophylactic medications before and after a medication with propensity for causing infusion reactions.

In one or more embodiments, the drug delivery system is provided with an input/output interface to a clinical trial data management system. In some embodiments, the data management system contains permanent storage for data collected during the clinical trial from one or more drug delivery systems herein. In some embodiments, data within the permanent data storage is used to support a regulatory submission for drug approval. In some embodiments, one or more drug delivery apparatus or systems is associated with one or more investigational therapeutic medications and/or clinical trial administration conditions for a specific patient.

### Patient Interface

Selection of the physiologic administration route dictates the patient interface used to deliver medication to the patient. While the most common physiologic routes are shown in Figures 1A through 1D, many other configurations of a patient interface will be apparent those skilled in the art, and descriptions herein are for illustrative purposes only, and shall not be construed as limiting the present disclosure.

Referring to Figures 1A and 1B, for patients receiving medication via a peripheral intravenous catheter (PIV) 115 or central venous access device (CVAD) 107 and 103, the patient interface 104 is provided by means of a Luer-Lok^{®} or luer taper connection familiar to those skilled in the art. For patients receiving medication via an implanted venous port 127 and catheter 128, the patient interface 125 is provided by means of percutaneous access to the needle entry septum 129 with a specialized steel needle, such as a Huber needle 124.

Referring to Figure 1C, for patients receiving medication via the subcutaneous route the patient interface comprises a subcutaneous (SC) needle assembly 140 and 158 placing needles at 90° 142 or 45° 160 to the injection site, thereby accessing to the SC tissue 148 and 175, through hollow-bore needle points 143 and 170. For intramuscular (IM) administration with embodiments of the apparatus or system, the patient interface comprises an IM needle assembly 151, wherein a hollow-bore needle 155 is placed into the patient's muscle tissue 149 through open needle point 156. The material of needles 142, 155, and 160 are siliconized rigid medical grade stainless steel common in the art. Medications are delivered to the patient via integral tubing sets 145, 154, and 172.

Referring to Figure 1D, for patients receiving medication via the SC or IM routes the patient interface may alternately comprise a flexible soft cannula placed by a removable, rigid inserter needle. A needle assembly 181 is inserted against the patient skin 182 by a patient or caregiver 180, optionally using one or more insertion affordances 186. Upon placement against the patient skin 182, a first portion of needle assembly 181 is removed by the user 188, retaining a portion 191 in the skin comprising the soft, flexible cannula 192 with open tip 194. The first removed portion of the needle assembly 189 comprises the steel inserter cannula 190 and the insertion and removal affordances 189. The retained portion of the needle assembly 191 includes a tubing set 195 for medication administration to the patient's SC tissue 193. IM administration is also provided simply by increasing the length of the flexible cannula 183 and inserter needle 184 to place the open end of the flexible cannula 194 into the patient's muscular tissue 195. The material of the inserter needle 190 is rigid siliconized medical grade stainless steel, and the material of the flexible administration cannula 184 may be any biocompatible polymer, such as PFTE.

### Drug Delivery System Components

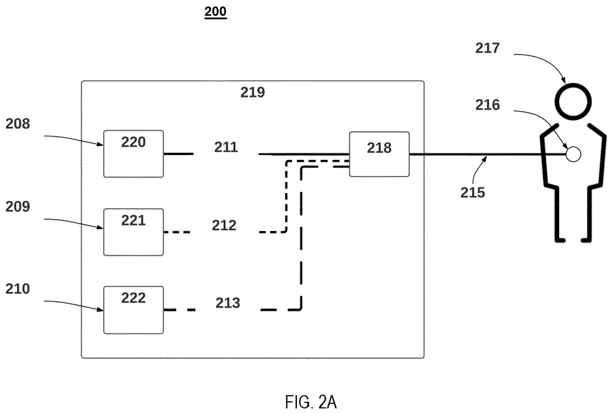

Figure 2A illustrates variations of an exemplary drug delivery apparatus or system comprising an outer housing 219, a plurality of reservoirs 208, 209, and 210 for one or more therapeutic medication(s) 220, 221, and 222, fluidically connected 211, 212, and 213 to a fluid pump 218, by which the reservoirs may be emptied by the fluid pump 218 to administer the medication to the patient 217 by way of a tubing set 215 and patient interface 216. Although three reservoirs 208, 209, and 210 are described herein, many configurations of reservoirs are apparent based on the desired medication regimen, and are presented for illustrative purposes only, without limiting the present disclosure. As the exemplary embodiments make clear, any number of medications can be administered by the present system as desired.

In some embodiments, the outer housing 219 substantially encloses one or more reservoirs 208, 209, 210 and fluidic communication 211, 212, 213 between the reservoirs and fluid pump 218. In some embodiments, the outer housing 219 substantially encloses the fluid pump 218 and fluidic communication 211, 212, 213 between the reservoirs and fluid pump 218, and partially encloses one or more reservoirs 208, 209, 210.

In some embodiments, the outer housing is a rigid enclosure. In some embodiments, the outer housing is substantially flexible to conform to a patient's body or pocket. In some embodiments, the outer housing is configured with a single contoured side oriented towards and situated to conform to the patient's body. In some embodiments, the rigid plastic material, such as polypropylene, polycarbonate, acrylonitrile butadiene styrene, polyamide, or polystyrene. In some embodiments, the outer housing is over-molded on the side closest to the patient's body with a soft, compliant material, such as thermoplastic elastomer or thermoplastic polyurethane. In some embodiments, the outer housing is provided with a soft, compliant gel material on the side closest to the patient's body. In some embodiments, the outer housing is configured with a clip to allow attachment to a patient's clothing, pocket, or belt.

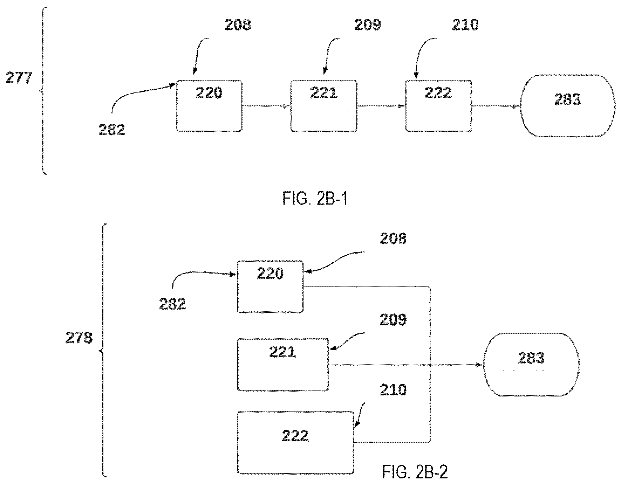

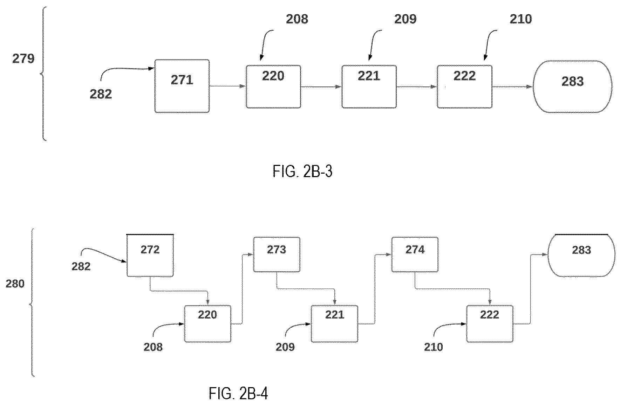

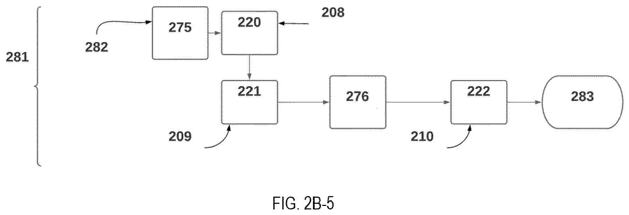

Referring to Figure 2B, embodiments of the present drug delivery apparatus or system provide sequential, concurrent, time-delayed, and contingent administration of a variety of medications in a time sequence with a beginning 282 and end 283. During the time sequence, a plurality of medications 220, 221, 222 may be delivered in a prescribed sequential order 277 (as shown in Figure 2B-1), in a concurrent manner 278 (as shown in Figure 2B-2), in a prescribed sequential order 279 (as shown in Figure 2B-3) in beginning after a prescribed time-delay 271, or in a in a prescribed sequence 280 (as shown in Figure 2B-4) separated by one or more equally or unequally spaced time-delays 272, 273, and 274. Alternatively, during the time sequence, a plurality of medications 220, 221, 222 may be delivered in a prescribed sequence 281 (as shown in Figure 2B-5), wherein certain medications are administered concurrently 220 and 221 after an optional time delay 275, after which other medications 222 are administered after a prescribed time-delay 276. The foregoing examples are for illustrative purposes and shall not be construed as limiting the number of medications or configurations that will be apparent to those skilled in the art.

Figure 9 illustrates variations of an exemplary drug delivery apparatus or system comprising an outer housing 801, a plurality of reservoirs 807', 808' for one or more therapeutic medication(s) 807, 808 fluidically connected 809, 810 to a fluid pump 811, by which the reservoirs 807', 808' may be emptied by the fluid pump 811 to administer the medication 807, 808 to the patient 814 by way of a tubing set 812 and patient interface 813. Although the plurality of reservoirs 807', 808' shows only two reservoirs, this is for illustration only, and the apparatus and systems described herein are not limited to a particular number of reservoirs. In one or more embodiments there can be any suitable number of reservoirs. The drug delivery system is also provided with a controller 803 that communicates with components of the apparatus via either a wired or wireless connection. In one or more embodiments, the controller according to one or more embodiments comprises a processor 804, a memory coupled to the processor 805, input/output devices 806 coupled to the processor 805 and support circuits to provide communication between the different components of the system, namely the components of the system described herein. In one or more embodiments, processes to operate the system are stored in the memory 805 as a software routine that, when executed by the processor, causes the system to perform methods described in the present disclosure. In one or more embodiments, the processes to operate the system are performed in hardware. In one or more embodiments, the software routine to operate the system may also be stored and/or executed by a second processor that is remotely located from the hardware being controlled by the processor. In some embodiments, the second processor comprises a cloud computing service or server. In some embodiments, the second processor comprises a remote patient monitoring system used by a healthcare provider. In some embodiments, the second processor comprises an electronic health record (EHR) system interface. In some embodiments, the second processor comprises a clinical trial data management system interface. In some embodiments, the second processor comprises a smartphone, smart tablet, smart television set, or voice activated assistant.

In one or more embodiments, one or more input/output devices 806 comprises a light source that may be illuminated upon receiving instructions or a signal from the controller 803. In one or more embodiments, the light source is coupled to an optical conductor in the tubing set 812. In one or more embodiments, one or more input/output devices 806 comprises a power source electrically coupled to a conductor within the tubing set 812.

In one or more embodiments, controller 803 may be also coupled to the fluid pump 811 to sense and/or control fluid flow therein. In one or more embodiments, controller 803 may be also coupled to one or more fluidic connections 809, 810 sense and/or control fluid flow therein. In one or more embodiments, controller 803 may be also coupled to one or more sensors and reservoirs 807', 808' containing medication 807, 808. In one or more embodiments, the outer housing 801, reservoirs 807', 808', and/or tubing set 812 may be configured with sensors also coupled to the controller 803. In one or more embodiments, controller 803 may be also coupled to one or more sensors 815 on the patient 814.

### Therapeutic & Other Medications

Various medications may be delivered by the present disclosure, including therapeutic medications, prophylactic pre-medications, prophylactic post-medications, emergency medications, and flushing solutions. Thus, "therapeutic medication" is used as a term of convenience herein to distinguish medications used to treat a disease (e.g., an oncology agent) from other ancillary medications delivered by the system while administering a therapeutic medication (e.g., a premedication or saline flush).

In some embodiments, a therapeutic medication is for treating one or more diseases selected from the group of cardiovascular, gastrointestinal, autoimmune, immunologic, hematologic, oncology, endocrinology, and respiratory disease. In some embodiments, a therapeutic medication is a coformulation of one or more medications for treating one or more of the aforementioned diseases. In some embodiments, multiple therapeutic medications are provided as part of a combination therapy.

In some embodiments, one or more therapeutic medications is a small molecule drug, therapeutic protein, cytokine, hormone, blood product, biologic, monoclonal antibody, antibody-drug conjugate, bispecific antibody, fusion protein, chimeric antigen receptor T cell therapy, cell or gene therapy, oncolytic virus, or immunotherapy.

In some embodiments, one or more therapeutic medications is an immuno-oncology or bio-oncology medication. In some embodiments, one or more therapeutic medications is selected from the group of several proposed targets, such as immune checkpoints, cytokines, chemokines, clusters of differentiation, interleukins, integrins, growth factors, enzymes, signaling proteins, pro-apoptotic proteins, anti-apoptotic proteins, T-cell receptors, B-cell receptors, or costimulatory proteins.

In some embodiments, one or more therapeutic medications is selected from the group of proposed mechanisms of action, such as HER-2 receptor modulators, interleukin modulators, interferon modulators, CD38 modulators, CD22 modulators, CCR4 modulators, VEGF modulators, EGFR modulators, CD79b modulators, Trop-2 modulators, CD52 modulators, BCMA modulators, PDGFRA modulators, SLAMF7 modulators, PD-1/PD-L1 inhibitors/modulators, B-lymphocyte antigen CD19 inhibitors, B-lymphocyte antigen CD20 modulators, CD3 modulators, CTLA-4 inhibitors, TIM-3 modulators, VISTA modulators, INDO inhibitors, LAG3 (CD223) antagonists, CD276 antigen modulators, CD47 antagonists, CD30 modulators, CD73 modulators, CD66 modulators, CDw137 agonists, CD158 modulators, CD27 modulators, CD58 modulators, CD80 modulators, CD33 modulators, APRIL receptor modulators, HLA antigen modulators, EGFR modulators, B-lymphocyte cell adhesion molecule modulators, CDw123 modulators, Erbb2 tyrosine kinase receptor modulators, mesothelin modulators, HAVCR2 antagonists, NY-ESO-1 OX40 receptor agonist modulators, adenosine A2 receptors, ICOS modulators, CD40 modulators, TIL therapies, or TCR therapies.

In some embodiments, one or more therapeutic medications is selected from one of ipilimumab, nivolumab, pembrolizumab, atezolizumab, durvalumab, avelumab, cemiplimab, rituximab, trastuzumab, ado-trastuzumab emtansine, fam-trastuzumab deruxtecan-nxki, pertuzumab, transtuzumab-pertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab.

In some embodiments, one or more therapeutic medications is a part of a multi-medication treatment regimen. In some embodiments, one or more therapeutic medications is a part of a multi-medication treatment regimen selected from the group of AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, RCHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VelP, OFF, GTX, CAV, AD, MAID, AIM, VAC-IE, ADOC, or PE.

In some embodiments, one or more therapeutic medications is used for adjuvant chemotherapy. In some embodiments, the chemotherapeutic compound is used for neoadjuvant chemotherapy. In some embodiments, the chemotherapeutic compound is an alkylating agent, plant alkaloid, antitumor antibiotic, antimetabolite, or topoisomerase inhibitor, enzyme, retinoid, or corticosteroid. In some embodiments, the chemotherapeutic compound is selected from the group of 5-fluorouracil, cisplatin, carboplatin, oxaliplatin, doxorubicin, daunorubicin, idarubicin, epirubicin, paclitaxel, docetaxel, cyclophosphamide, ifosfamide, azacitidine, decitabine, bendamustine, bleomycin, bortezomib, busulfan, cabazitaxel, carmustine, cladribine, cytarabine, dacarbazine, etoposide, fludarabine, gemcitabine, irinotecan, leucovorin, melphalan, methotrexate, pemetrexed, mitomycin, mitoxantrone, temsirolimus, topotecan, valrubicin, vincristine, vinblastine, or vinorelbine.

In some embodiments, one or more therapeutic medications is classified as a hazardous medication according to the Centers for Disease Control's "NIOSH List of Hazardous Drugs In Healthcare Settings" or as defined by US Pharmacopeia General Chapter <800> "Hazardous Drugs - Handling in Healthcare Settings."

When administering certain therapeutic medications, prophylactic medicines may be administered to a patient before (pre-medication) or after a therapeutic medication (post-medication) to avoid systemic infusion reactions or ease discomfort from a therapeutic medication's side effects. The pre-medication and post-medications may also comprise part of a medication regimen or medication order set, described elsewhere herein.

Figure 2C illustrates an exemplary drug delivery apparatus or system configured to administer certain prophylactic medicines in addition to one or more therapeutic medications also contained within the system. In one or more embodiments, the drug delivery apparatus or system 223 contains a plurality of reservoirs for medication 224, 225, and 226. In some embodiments, reservoir 224 contains one or more prophylactic pre-medications 227 administered before the therapeutic medication(s) 228. In some embodiments, administration of the therapeutic medication 228 can take place only after complete administration of required pre-medication 224. In some embodiments, reservoir 226 contains one or more prophylactic post-medications 227 administered after the therapeutic medication(s) 228.

In one or more embodiments, one or more reservoirs 224 or 226 contains one or more medications selected from the group of 0.9% normal saline, 0.45% normal saline, 5% dextrose in water, 5% dextrose in 0.45% normal saline, Lactated Ringer's solution, albumin, and crystalloid fluids containing added electrolytes, such as potassium.

In one or more embodiments, one or more reservoirs 224 or 226 contains one or more medications selected from the group of analgesics, antipyretics, corticosteroids, antihistamines, antiemetics, antibiotics, anticoagulants, fibrinolytics, or antithrombolytics. In one or more embodiments, one or more reservoirs 224 or 226 contains one of diphenhydramine, acetaminophen, ondansetron, or famotidine.

In one or more embodiments, one or more reservoirs 224 or 226 are configured to reconstitute a lyophilized pre-medication or post-medication contained in a dual-chamber syringe featuring a bypass chamber. In one or more embodiments, one or more reservoirs 224 or 226 are configured to reconstitute a lyophilized pre-medication or post-medication in an anticipatory fashion to allow more timely administration.

When administering medications intravenously, it is necessary to flush the IV catheter system before and after medication administration. Flushing refers to the process of instilling a fluid volume after therapeutic medication delivery through the entire IV system to ensure all medication within the IV system is fully administered to the patient and to prevent clotting of the catheter system. In one or more embodiments, the drug delivery apparatus or system may also be configured to deliver of a therapeutic medication in conjunction with catheter flushing protocols.

Referring to Figure 2D, in one or more embodiments, the drug delivery apparatus or system is provided with flush reservoirs 241, 243, 244 and a reservoir for therapeutic medication 242. The delivery apparatus or system is configured to deliver one or more catheter flushing solutions before 245 and/or after 247, 256 administration of one or more therapeutic medications 246. In one or more embodiments, the delivery apparatus or system administers an 0.9% Normal Saline from a pre-administration flush reservoir 241 followed by one or more therapeutic medications 246 in a reservoir 242, followed by an 0.9% Normal Saline Flush in a first post-administration flush reservoir 243, followed by Heparin Lock Flush solution in a second post-administration flush reservoir 244. Flushing need not be limited to the beginning and end of an administration process; when multiple medications are administered, flush reservoirs may be interposed between therapeutic medication administrations if desired.

In some embodiments, one flushing solution is 0.9% Normal Saline. In some embodiments, one flushing solution is recombinant tissue plasminogen activator (r-TPA). In some embodiments, one flushing solution is one or more medications selected from the group of 0.9% Normal Saline, Heparin Lock Flush solution, 100 U/mL Heparin Lock Flush Solution, and 5000 U/mL Heparin Lock Flush Solution. In some embodiments, one flushing solution is an antimicrobial. In some embodiments, one flushing solution is an antimicrobial combined with an anticoagulant.

### Tubing Set

Figures 6A-C illustrate variations of an exemplary tubing sets for use with the present disclosure. In one embodiment, a tubing set 640 is provided with cross-sectional tubing profile 640' and at least one inner medication lumen 641. During use of the drug delivery system, inner medication lumen 641 is in fluidic communication with a fluid pump and a patient interface described elsewhere herein to deliver medications within the system to the patient.

It may be desirable to isolate one or more inner medication lumens 648 from potential contaminant leachable or extractable compounds from the tubing set material, thereby improving compatibility with the medication delivered therein. Accordingly, in some embodiments, barrier coating 647 may be interposed between an inner medication lumen 648 and tubing set material 646'. In one embodiment, the barrier coating comprises a PTFE fluoropolymer material. In another embodiment, the barrier coating is co-extruded as the tubing set is manufactured. In another embodiment, the interior medication-contacting surface of one or medication lumens are provided with a hydrophobic coating.

It may be desirable to offer multiple flowrates in the present drug delivery system without switching tubing sets. Accordingly, in one embodiment, a tubing set 642 is provided with cross-sectional tubing profile 642' and two or more medication lumens 643, 644, 645. The medication lumens may have different or similar diameters, thereby allowing administration of medications at flow rates in a variety of configurations. By way of example, the same medication administered through a first lumen 644 would flow more quickly than if administered through a second lumen 643 in the tubing set design exemplified in Figure 6C. In an alternative embodiment, medication delivery may be accelerated by switching flow from a smaller to a larger lumen (e.g., from 643 to 645). In an alternative embodiment, medication delivery may be decelerated by switching flow from a larger to a smaller lumen (e.g., from 645 to 643). In an alternative embodiment, one or more medication lumens may be engaged in parallel fashion (e.g., using 643 and 645, or 644 and 643) to provide faster administration of a single medication. In an alternative embodiment, one or more medication lumens may each deliver a different medication concurrently. In an alternative embodiment, one or more medication lumens remains unused by the system until desired, as in the case of emergency medication administration as described herein.

Elements of the tubing sets described herein may take various shapes and forms. In one or more embodiments, cross-sectional tubing profiles may take a substantially circular, elliptical, rectangular, or polygonal shape. The flexible portion of the tubing set may be fashioned from one or more of silicone, PVC, PVC without DEHP, EVA, HDPE, LDPE, TPU, PTFE, a fluoropolymer, or other suitable flexible material. In one or more embodiments, tubing sets are extruded but may be formed by other means that provide sufficient dimensional and tolerance control on the inner medication lumens as described herein. In one or more an embodiments, the tubing material is chosen to be a material selected for low leachable and extractable compounds that may contaminate a medication, and that exhibits high biocompatibility with biologic medications.

Optionally, the flexible portion of the tubing set may comprise segments of one or more flexible materials, providing different degrees of flexibility at different sections along the length. For instance, a more rigid material may be provided near the connections to the fluid pump for strain relief and anti-kinking, while a more flexible material may be selected near the patient interface for comfort against a patient's skin. The exterior of the tubing set may be provided with a PFTE fluoropolymer or other permanently lubricious coating to prevent dragging or snagging of the tubing set on a patient's skin or clothing.

In one or more embodiments, and referring to Figures 7A-B, one or more tubing sets is provided with an inline filter 601 to remove undesirable or immunogenic particulate matter 602 prior from the inflow medication 603 prior to patient administration at the outflow side of the filter 604. The inline filter material is ideally selected to be low-sorbing, low protein binding, and compatible with the medication(s) therein. Optionally, the inline filter may comprise a multi-layer filter membrane, with each membrane layer featuring a different filter pore size.

In one or more embodiments, one or more tubing sets are provided with an engineered flow restriction 607 to provide an inflow medication 605 at a first rate, and outflow medication 608 at a second rate substantially less than the first rate. When used with biologic or shear-sensitive medications, the smoothed inlet 606 and engineered flow restriction 607 is in one or more embodiments designed to prevent protein damage or shearing.

### Fluid Pump

A variety of fluid pumps may be used in the disclosure herein, based on the configuration of reservoirs, viscosity of medications, and number of medications. In some embodiments, a single fluid pump is provided. In some embodiments, multiple fluid pumps are provided. In some embodiments, the fluid pump is configured to start, pause, or stop on demand. In some embodiments, the fluidic pump is configured with a transmission mechanism to provide selective engagement and disengagement of selected medication reservoirs. In some embodiments, the mechanical drive is coupled to a gear mechanism to reduce the form factor of the apparatus or system. In some embodiments, the gear mechanism comprises mating bevel gears. In some embodiments, the fluidic pump is prevented from operation if one or more medications is insufficiently viscous. In some embodiments, the fluidic pump is provided with a sensor to determine the temperature of a fluid at the fluid pump inlet.

Fluid pumps may be powered by, for example, a flat coil spring, wound helical spring, strip spring, pressurized gas, or an electrical motor. In some embodiments, a rotary power source may be coupled to one or more reservoirs through a worm screw and worm gear. In some embodiments, the worm screw and worm gear is used to hold a reservoir in a given position while other reservoirs are driven by the system. In another alternative embodiment, the fluid pump be driven by a power unit with rate control assembly, such as disclosed in US Patent 10,252,005. In another alternative, the fluid pump may be driven by a chemical engine, such as disclosed in US Patent 9,795,740. In another embodiment, the fluid pump may be drive by a power unit with progressive engagement mechanism, such as disclosed in US Patent 10,357,612. In another embodiment, the fluid pump may be driven by a rotary drive, such as disclosed in US Patent Nos. 8,617,109, 8,876,766, 9,022,982, 9,095,657, 9,132, 236, 9,446,201, 9,468,722, 9,737,668, 10,255,827, 10,307,543, 10,456,521, 10,507,289, 10,525,213, 10,632,248, 10,874,804, 10,881,811 and 11,065,387.

In an alternative embodiment, the fluid pump is a one-time use disposable design. In an alternative embodiment, the fluid pump is a reusable design for multiple medication administrations. In an alternative embodiment, the fluid pump is a reusable design designed to administer a single cycle of a medication regimen.

In one or more embodiments, one or more fluidic connections are designed to minimize internal volume that is not administered to the patient, thereby reducing medication waste and the need for medication overfill. Accordingly, in one or more embodiments, fluidic connections between one or more reservoirs and the fluid pump may comprise a manifold. In an alternative embodiment, each fluidic connection between one or more reservoirs and the fluid pump may have proportionally different relative to each other, permitting independent flow rate control of one or more medications beyond that provided by one or more tubing sets provided with the drug delivery system.

### Fluid Pump + Tubing Set Integration

In an embodiment, the fluid pump is sufficiently well-powered to deliver a full range of volumes, viscosities, and rates independently of the inner diameters of a tubing set, thereby allowing the same fluid pump design to be used for a variety of medications. This has the advantage of mass-producing fluid pumps and gaining efficiencies of scale. This approach allows design of a drug delivery apparatus or system without knowing medication formulation characteristics *a priori.* This is particularly important in clinical trials, where medication formulation characteristics are still in development, and dosing regimens are not yet finalized.

It is apparent that tubing sets in the present disclosure are used to control administration parameters for a therapeutic medication and accommodate flow characteristics of specific drug formulations without the need for complex or precise mechanical or electromechanical pumps. This is particularly important for biologic drug products or extended-release formulations displaying non-Newtonian shear-thinning and shear-thickening behaviors where modeling techniques are of limited usefulness.

Figure 3B depicts an embodiment of a process to design tubing sets for use in a clinical trial in accordance with the disclosure herein. Formulation characteristics 360, pharmacokinetic modeling parameters 361, and desired clinical trial conditions 362 are inputs to initial numeric modeling 363 using either Hagen-Pouiselle's equation 380 (FIG. 3C) or other modeling methods, such as computational fluid dynamics. Modeling 363 provides initial design and component selection 364, comprising minimally first estimated nominal tubing lengths 391, tubing nominal internal diameters 392, and corresponding tolerances 393 on the nominal internal diameters 392 (FIG. 3C).

Tubing may be manufactured based on initial design and component selection 364. However, for non-Newtonian fluids, initial numeric modeling 363 may be substantially different than predicted, and adjustments to tubing internal diameters 392, and corresponding tolerances 393 on the internal diameters 392 may be required. The adjustments may require time-consuming or costly changes to extrusion dies or other equipment, and multiple testing and adjustment cycles may be required.

Regardless, the flow rate provided by the initially selected components 364 are physically tested 365 with the drug formulation of interest and compared to the desired clinical trial conditions 324, 330, 334, and 342. Physical testing 365 may optionally include characterization of any damage to the drug product caused by the tubing set or flow rates, including protein damage or shearing effects which may render protein-based medications inactive or harmfully immunogenic to humans. Physical testing 365 may optionally be conducted at temperatures representative of the administration setting for the final medication in clinical practice, which is especially relevant for medications that exhibit a nonlinear viscosity-temperature-concentration relationship, such as biologics.

As many medications display non-Newtonian shear-thinning and shear-thickening behaviors, empirical results may also differ from theoretical calculations, in which case components are iteratively redesigned 367. Individual tubing sets corresponding to a specific flow rate for a specific medication are individually analyzed, refining either tubing lengths 391 or tubing diameters 392, or specifying precision tolerances 393 on the diameters 392. Once precisely designed, a plurality of tubing sets is manufactured 368 for use with the overall drug delivery system to execute a given clinical study design 369 as previously specified.

### Medication Reservoirs

Referring to Figure 2A in one or more embodiments, medication reservoirs 208, 209, and 210 are designed for short-term duration contact with therapeutic medications 220, 221, and 222, minimizing the technical burden and risk associated with long-term stability or container closure testing. In an alternative embodiment, the reservoirs 208, 209, and 210 are each selectively designed for short- or long-term drug contact based on the nature of the medicine 220, 221, and 222 therein.

Referring to Figure 2C, in one or more embodiments, medication reservoirs 224 and 226 are long-term stability primary containers that are prefilled with medications 227 and 228, and reservoir 225 with a therapeutic medication 228 is designed for short-term stability and filled just prior to administration.

In one or more embodiments, one or more reservoirs is a glass or plastic syringe or cartridge prefilled by the manufacturer. In one or more embodiments, the interior surface of one or more reservoirs contains controlled levels of a silicone lubricant. Optionally, the silicone lubricant may be crosslinked, as through radiation. In one or more embodiments, one or more reservoirs is a single syringe with a plurality of reservoirs, chambers, or compartments.

In an embodiment of the of the present disclosure, one or more reservoirs is a flexible nonelastic container. In one or more embodiments, the flexible nonelastic container is fully emptied through application of a compressive force. Optionally, the flexible nonelastic container may be contained in a rigid protective shell. In one or more embodiments, one or more reservoirs is a flexible elastomeric container. In one or more embodiments, one or more reservoirs is a flexible container with one or more segments, each containing a single medication.

In some embodiments, one or more reservoirs are manufactured from one or more materials selected from the group of borosilicate glass, cyclic olefin polymer, cyclic olefin copolymer, PVC, EVA, fluorinated ethylene propylene (FEP) resins or films, PTFE, a fluoropolymer, or other suitable material. In other embodiments, one or more reservoirs are manufactured from a low-sorbing material. In some embodiments, one or more interior reservoir surfaces in contact with medication has a hydrophilic coating or has been passivated to reduce protein sorbing or formation of protein aggregates.

In some embodiments, the reservoirs are filled by pharmacy before dispensing to a patient. In some embodiments, the reservoirs are filled by a patient or caregiver at home. In some embodiments, the reservoirs are prefilled and assembled into the drug delivery system prior to use by a patient. In one or more embodiments, one or more reservoirs is filled while contained in the drug delivery apparatus or system. In one or more embodiments, one or more reservoirs is filled outside the drug delivery apparatus or system, then installed into the drug delivery system as a secondary operation. In one or more embodiments, one or more reservoirs is filled by the patient, lay caregiver, or healthcare provider. In an alternative embodiment, one or more medication vials are provided with a vial transfer apparatus or system for filling a reservoir. In an alternative embodiment, the reservoir is pre-attached to a transfer apparatus or system to effectuate filling with a minimum of use steps and corresponding risk of aseptic breach. In an alternative embodiment, the reservoir is filled from a vial using pressure applied by a compressed gas. In an alternative embodiment, the reservoir is filled from a vial using pressure applied by an electromechanical pump assembly.

In one or more embodiments, the drug delivery system is equipped with one or more features to prevent unauthorized access to, or diversion of, one or more reservoirs containing a controlled substance after filling. The features may include a tamper-evident seal on the exterior of the drug delivery apparatus or system or internal sensors to detect unauthorized access to the drug delivery system and components within it, including medication reservoirs.

In some embodiments, one or more reservoirs is provided with a sensor to determine the temperature of a fluid therein. In some embodiments, the sensor is located on the exterior of the reservoir. In some embodiments, the sensor is a temperature probe making direct contact with the medication through the reservoir wall.

### Infusion Reaction Detection

Used herein as a term of convenience, infusion reactions include standard infusion reactions (SIRs), cytokine-release reactions, or IgE-mediated allergic reactions. As new categories of biologics with novel modes of action are developed and commercialized, additional types of patient infusion reactions may also become apparent beyond those listed herein. Thus, the foregoing infusion reactions cited herein are provided by way of example and shall not be construed as limiting the scope of disclosure of the disclosure herein.

Certain medications are associated with overall higher incidence of infusion reactions. For these medications, specific pre- and post-medications are administered to reduce incidence of infusion reactions or negative patient impacts should they occur. Administration of pre- and post- medications is provided by the present disclosure as illustrated in Figure 2C and described elsewhere herein.

However, even when prophylaxis is administered, infusion reactions can occur. Infusion reactions are clinically distinct from injection site reactions, which cause localized discomfort and are neither emergent nor life threatening to the patient. Onset of infusion reactions is sudden, systemic, and life-threatening; treatment requires unexpected and immediate administration of counteracting emergency medications. Due to rapid onset, healthcare providers monitor patients routinely in the clinic setting and intervene immediately.

Due to the serious nature of infusion reactions, it is highly desirable to anticipate potential infusion reactions at onset, especially in settings outside the clinic, which is also provided by alternative embodiments of the drug delivery apparatus or system herein. Figure 4 illustrates an exemplary drug delivery apparatus or system configured to include sensors to detect potential infusion reactions, a controller and algorithms, features to interrupt medication flow, and optional features for delivery of emergency medications in response to an infusion reaction.

Referring to Figure 8, in one alternative embodiment, data from coupled sensors 815 is processed by an algorithm within the controller 803 configured to detect suspected infusion reaction and deliver appropriate therapeutic treatment automatically or through intervention by a healthcare provider. In some embodiments, the algorithm utilizes historical data from a single patient to determine whether an infusion reaction is occurring. In some embodiments, the algorithm utilizes historical data from one or more users of the drug delivery system to determine whether an infusion reaction is occurring. In some embodiments, the algorithm utilizes historical data from one or more prior clinical trials with the therapeutic medication being administered to determine whether an infusion reaction is occurring. In some embodiments, the aggregated historical data is analyzed by a machine learning program to improve accuracy or timeliness of infusion reaction identification. In some embodiments, the algorithm uses historical data aggregated from many patients in conjunction with machine learning to compute a probabilistic estimate of whether an infusion reaction is occurring in a present instance.

In one or more embodiments, the drug delivery apparatus or system is configured to halt administration of one or more therapeutic medications immediately if an infusion reaction is detected. In a first alternative embodiment, drug delivery may be halted by the controller 803 interrupting fluidic connection with the tubing set 812. In a second alternative embodiment, drug delivery system may be halted by the controller 803 stopping the fluid pump 811. However, both preceding alternative embodiments are disadvantageous, as no further medications may be administered, including a counteracting emergency medication. In a third alternative and an embodiment, administration of a therapeutic medication may be halted by the controller interrupting fluidic connection between the reservoir 807 and the fluid pump 811, while leaving the fluid pump 811 and tubing set 812 operable to provide administration of a counteracting emergency medication 808 contained in reservoir 808'.

Referring to Figure 4, in an alternative embodiment, a drug delivery apparatus or system is provided with a reservoir 402 for containing a therapeutic medication, a reservoir 416 containing an emergency medication, and fluidic connections 411, 417 between the reservoirs and a fluid pump 415, a tubing set 405 fluidically connected between the fluid pump 415 and patient interface 406, one or more sensors 407, and one or more sources of patient data 408. Sensor data 410 is communicated to the controller 403 from the sensors 407. In one alternative embodiment, data from sensors 407 is processed by an algorithm within the controller 403 configured to detect suspected infusion reaction and deliver appropriate therapeutic treatment automatically or through intervention by a healthcare provider as described herein.

In one or more embodiments, the controller 403 according to one or more embodiments comprises a processor 403a, a memory coupled to the processor 403b, input/output devices 403c coupled to the processor 403a, and support circuits to provide communication between the different components of the system, namely the components of the system described herein. In one or more embodiments, processes to operate the system are stored in the memory 403b as a software routine that, when executed by the processor, causes the system to perform methods described in the present disclosure. In one or more embodiments, process to operate the system comprise an infusion reaction detection algorithm 403d based on one or more sensor data 410 from one or more patient sensors 407 or patient data 408. In one or more embodiments, patient data 408 comprises a self-report of symptoms by the patient 407. In one or more embodiments, patient data 408 is derived from a healthcare provider interaction with a patient 407. In one or more embodiments, the infusion reaction detection algorithm also is configured to respond to a detected infusion reaction in conjunction with the controller 403, whereby one or more emergency medications 416 may be administered, or whereby medication delivery may be halted to a patient 407 as described herein. In one or more embodiments, the processes to operate the system are performed in hardware. In one or more embodiments, the software routine to operate the system may also be stored and/or executed by a second processor that is remotely located from the hardware being controlled by the processor.

In one or more embodiments of the present disclosure, the drug delivery device is configured to halt administration of one or more therapeutic medications of interest immediately if an infusion reaction is detected. In a first alternative embodiment, the drug delivery system 401 may be provided with a fluid flow control 414 configured to interrupt fluidic communication between the fluid pump 415 and the tubing set 405. In a second alternative embodiment, the drug delivery system 401 may be provided with a fluid flow control 412 configured to interrupt the fluid pump 415 and cease all medication delivery to the patient 404.

However, both preceding alternative embodiments have the disadvantage that no further medications may be administered, including a counteracting emergency medication. Thus, in a third alternative and preferred embodiment, the drug delivery system 401 may be provided with a fluid flow control 413 configured to interrupt fluidic communication between the fluid pump 415 and a therapeutic medication reservoir 402, thereby preventing flow of a therapeutic medication 402 causing an infusion reaction, while leaving the fluid pump 415 and tubing set 405 configured to administer a counteracting emergency medication 416 to a patient 404.

Figure 5 provides a schematic of an embodiment of the decision-making algorithm within the controller 403 and sensor(s) 407 referenced in Figure 4, wherein diagnosis and treatment for infusion reactions are supported by the algorithm 403 as a form of decision support for a healthcare provider. The embodiment provides that during medication administration 501, the drug delivery system is configured to detect potential infusion reactions based on one or more of physiologic sensor data 502, in-person or remote observation of the patient's condition 503 by a healthcare provider, and patient self-report 527.

Physiologic data 502 for potential infusion reactions, may include by way of example but not limitation, heart rate, blood pressure, respiratory rate, blood oxygen saturation (SpO2), and temperature, which are collected by way of sensor(s) 407. The plurality of sensors sample the data 504, data is pre-processed 505 using the system's controller and algorithm 403 and the output is aggregated and consolidated 506, also by the controller and algorithm 403.

Sensor data may be supplemented with objective and subjective observation 507 of patients' conditions 503 from physical examination such as flushing, skin reactions, rigors, swelling, urticaria, angioedema, wheezing, stridor, cough, change in voice quality, or loss of consciousness. Sensor data may further be supplemented with data collected from patient interview or self-report 527, including by way of example, headache, shortness of breath, throat closing, diaphoresis, nausea, abdominal or back pain, itching, general anxiety, or self-reported sense of "impending doom."

Observations of the patient 507 prompt in-person or remote patient interactions and/or patient interviews 508, which are aggregated and evaluated by the healthcare provider in a feedback loop 509 until the patient evaluation is satisfactorily completed, whereupon the healthcare provider uses their clinical judgement and heuristics to arrive at an overall patient assessment 510. Quantitative sensor data 506 and qualitative patient assessment 510 is thus consolidated 511 into an overall patient assessment, which is used to assess whether the patient is experiencing an ongoing infusion reaction 512 and determine the need for emergent treatment.

If an infusion reaction is not suspected 513, administration 501 may be continued at the ongoing administration rate 514. If an infusion reaction is suspected 515, the medication infusion is automatically paused or stopped 516, the patient's situation is immediately escalated, and relevant clinical staff are provided with the appropriate data 517. Upon evaluating the totality of data 517 and the patient 518, the healthcare provider determines whether it is safe to restart the infusion 519. If the healthcare provider determines that the patient is not having an infusion reaction (i.e. "a false alarm") and it is safe to restart 520, the infusion may be continued at the same administration rate as previously tolerated 514.

If the healthcare provider determines that the patient is having a mild infusion reaction that can be remedied by slowing the infusion rate 521, the infusion may be continued at a reduced rate 522 pre-determined by the healthcare provider by administering medication using the smaller lumen of a multiple-lumen tubing as described elsewhere herein.

If the healthcare provider confirms the patient is experiencing an infusion reaction and determines it is unsafe to restart the infusion 523, they can opt to trigger an optionally provided feature within the drug delivery system to administer one or more emergency medications 524 and optionally call emergency medical services 525. In an alternative embodiment, the emergency medical services 525 are configured to provide a timelier response by virtue of geolocation data 526 provided by the drug delivery apparatus or system.

The treatment algorithm comprising 512, 513, 515, 516, 517, 518, 519, 523, and 524 is provided by way of example and not limitation. More generally, the present disclosure provides one of many alternative evaluation and treatment flows 550, which may be tailored based on the specific therapeutic medication, expected type and severity of infusion reaction, specifics in a prescribed medication order or order set, required counteracting medications, and other clinical considerations.

### Infusion Reaction Response

Contingent administration of emergency medications is particularly provided by the present disclosure, allowing safe administration of medications with propensity to cause side effects or reactions.

Figure 2E illustrates an exemplary drug delivery apparatus or system configured to contingently administer certain emergency medicines to counteract symptoms and/or to treat systemic infusion reaction caused by administration of one or more therapeutic medications also contained within the system.

In a first alternative embodiment, the drug delivery system is configured to administer one or more emergency medications using the same tubing set lumen used to administer one or more therapeutic medications. A drug delivery apparatus or system 285 is provided with a reservoir 286 for a therapeutic medication 287, a reservoir 288 containing an emergency medication 289, and fluidic connections 290, 291 between the reservoirs and a fluid pump 292, and a single lumen tubing set 293 fluidically connected between the fluid pump 292 and patient interface 295. Medication 287 is administered to the patient. In accordance with the disclosure herein, in the case of a suspected or actual infusion reaction, the emergency medication 221 is administered to the patient 294 through the patient interface 295.

In a second alternative embodiment, the drug delivery system is configured to administer one or more emergency medications in a pre-emptive manner using an alternative lumen than that used to administer one or more therapeutic medications. A drug delivery apparatus or system 285 is provided with a reservoir 286 for a therapeutic medication 287, a reservoir 288 containing an emergency medication 289, and fluidic connections 290, 291 between the reservoirs and a fluid pump 292, and a double lumen tubing set 293' fluidically connected between the fluid pump 292 and patient interface 295. Medication 287 is administered to the patient using a first medication lumen 297 within the double lumen tubing 293'. In accordance with the disclosure herein, in the case of a suspected or actual infusion reaction, flow of the therapeutic medication 287 is halted within the first medication lumen 297 and the emergency medication 221 is administered through a second medication lumen 298 within double lumen tubing 293' and into the patient 294 through the patient interface 295.

In some embodiments, the emergency medication is administered in response to a suspected systemic infusion reaction triggered by administration of one or more therapeutic medications. In some embodiments, the emergency medication is administered in response to a patient experiencing an adverse event. In some embodiments, the emergency medication is a reversal agent for one or more therapeutic medications.

In some embodiments, the emergency medication is epinephrine. In some embodiments, the emergency medication is naloxone. In some embodiments, the emergency medication is a corticosteroid. In some embodiments, the emergency medication includes one or more medications selected from the group of hydrocortisone, dexamethasone, or methylprednisolone. In some embodiments, the delivery apparatus or system is configured to reconstitute a lyophilized emergency medication prior to administration. In situations where time may be of the essence, the delivery apparatus or system may be configured to reconstitute a lyophilized emergency medication in an anticipatory fashion, such as when a potential infusion reaction is first detected by a sensor, but before administration has been ordered by a healthcare provider.

In some embodiments, the drug delivery apparatus or system is configured to administer an emergency medication automatically based on predetermined physiologic or clinical criteria. In some embodiments, the drug delivery apparatus or system is configured to administer an emergency medication based on instructions from a remote healthcare provider. In some embodiments, the drug delivery apparatus or system is configured to administer an emergency medication based on instructions from a user proximal to the apparatus or system.

### Clinical Trial Configuration

One primary benefit of embodiments of the drug delivery apparatus or systems disclosed herein is to allow commercial presentations of an approved medication to use the same delivery apparatus or system used in earlier clinical studies, without the need to design, validate, or test a second apparatus or system for commercial presentation. The present disclosure increases flexibility to accommodate a wide variety of pharmacokinetic profiles, even if the behaviors are not known in advance.

Pharmacokinetic (PK) profiles as used herein is a term of convenience, but components of PK profiles are well-understood by those skilled in the art and may include, by way of example but not limitation, bioavailability, Tₘₐₓ, Cₘₐₓ, Area Under Curve (AUC), C_{trough}, absorption rate constant, elimination rate constant, half-life, volume of distribution, clearance, and/or steady state concentrations. As used herein, Cₘₐₓ and C_{trough} are the maximum and minimum concentrations a drug reaches in the systemic circulation after administration of a given dose, respectively. Tₘₐₓ is the time required to reach Cₘₐₓ after administration of a given dose.

Figure 3A depicts a schematic of the preclinical and clinical development processes for dose determination of a typical parenteral drug *with the present disclosure incorporated.* In this process, the appropriate tubing set or sets 322 to employ in Phase 1 trials is determined in parallel with and influenced by formulation development 320 and pharmacokinetic modeling 321. Notably, the appropriate tubing set or sets, which govern flow desired *rate* in the clinical study 324, are decoupled from the dose *range,* which may be varied *independently.*

Phase 1 clinical trials are then conducted to establish dose ranges in a manner familiar to those skilled in the art. Tubing set(s) 325 as determined in 322 are supplied to the clinical trial site and are used to conduct the initial Phase 1 trial 324 according to desired clinical trial conditions, including the hypothesized dose ranges 323. Analysis of Phase 1 trial 326 data leads to dosing regimen refinement 327 used to design follow-on clinical trials.

If regimen refinement yields only a single dosing regimen 328, a single appropriate tubing set 330 will be designed for use in Phase 2 studies 330, corresponding to the desired clinical trial condition 339 from pharmacokinetic data and dose evaluation 326. If regimen refinement yields multiple possible dosing regimens 332, one alternative embodiment of the current disclosure provides for an appropriate kit of one or more tubing sets 333 to be designed for use in Phase 2 studies 334, wherein the kit components each correspond to one or more clinical trial conditions 336, 337, or 338.

Once the desired efficacy signal 340 is achieved with one or more dosing regimens, the appropriate tubing set or sets are determined 341 for the Phase 3 clinical trial, and then used in the Phase 3 trial 342 based on prior clinical trial results and corresponding to the desired clinical trial condition. Finally, upon regulatory approval, the appropriate tubing set or sets are selected for the commercial product 344 based on pivotal clinical trial results and the desired commercial presentation.

In an embodiment, during one or more clinical trials, staff select one or more tubing sets from a subset in Phase 1 324 and Phase 2 330 and 334 studies, then select a smaller subset of tubing sets for Phase 3 342 studies. In some embodiments, a smaller subset of tubing sets than those used for Phase 3 342 studies are provided to patients in a commercial presentation of the approved medication. In some embodiments, the same tubing sets used for Phase 3 342 studies are provided in a commercial presentation of the approved medication.

One advantageous aspect of the present disclosure is flexibility to accommodate use in both clinical trials and commercially marketed medications. Special considerations apply to drug delivery apparatus or systems used in clinical trials. Clinical trial data should be accurate, traceable, and reproducible; thus, data integrity is a cornerstone of successful clinical research and is an ethical and regulatory requirement designed to allow confident decision-making regarding approval of medicines.

Clinical trials take place in many different settings, depending on the clinical study phase, specific medication, and patient population. For instance, referring again to Figure 3A, many Phase 1 studies 304 and Phase 2 studies 309 and 310 are completed at clinical trial sites or in clinic. Phase 3 studies 313 may be completed at clinical trial sites, in clinic, or in the home setting. For Phase 3 studies 313 completed at home, alternative embodiments of the present disclosure are especially advantageous when configured to improve clinical trial data integrity through the incorporation of sensors, controllers, and permanent data storage meeting GCP or other regulatory requirements in a variety of configurations.

Referring to Figure 8, in an alternative embodiment, the drug delivery system 775 includes one or more sensors 782 coupled to a controller 779 to measure patient 783 vital signs at one or more stages before, during, and after administration of one or more therapeutic medications studied within a clinical trial 776. As medication administration progresses, data from the physiologic sensors 783 are recorded into permanent data storage 785 for later retrieval and analysis 787 by a clinical trial team 784. This provides later analysis of data by the clinical trial team 784 to identify any potential propensity for infusion reactions or other adverse physiologic effect as a result of the medication studied within the clinical trial 776.

In an alternative embodiment, the drug delivery system 775 includes one or more sensors 782 to measure the status of medication administration at one or more stages before, during, and after administration of one or more therapeutic medications studied within a clinical trial 776. As medication administration progresses, sensor 382 data are communicated 781 to a controller 779 and transferred 786 to permanent data storage 785 for later retrieval and analysis 787 by a clinical trial team 784. This provides for later analysis of data by the clinical trial team 784 and verification that each patient received a full medication dose as expected. In an alternative embodiment, the sensors may also be provided on one or more medication reservoirs 776' containing an investigational therapeutic medication 776.

In an alternative embodiment, the drug delivery system 775 includes one or more sensors 782 to monitor the patient interface throughout administration of one or more investigational therapeutic medications studied within a clinical trial 776. The sensor 782 data are communicated 781 to a controller 779 and transferred 786 to permanent data storage 785 for later retrieval and analysis 787 by a clinical trial team 784. This provides for later analysis of data by the clinical trial team 784 and verification that the medication was, in fact, administered directly to the patient as intended. In some embodiments, the sensor 782 comprises a skin sensor. In some embodiments, the sensor 782 comprises a flow sensor.

In an alternative embodiment, the drug delivery system 775 includes a controller and algorithm 779 to monitor the state of drug delivery system 775 throughout administration of one or more investigational therapeutic medications studied within a clinical trial 776, further communicating 781 any such detected failures to permanent data storage 785 for later retrieval and analysis 787 by a clinical trial team 784. This provides for later analysis of data by the clinical trial team 784 and verification that the drug delivery system 775 operated as intended during administration of an investigational therapeutic medication 776.

### Electronic Health Record Integration

Clinical trials occur in highly controlled settings to minimize confounding variability that could affect data integrity and mask positive or negative pharmaceutical efficacy. Once an investigational therapeutic medication is approved, administration may take place at home, in clinic, or both. In day-to-day patient care, treatment of diseases may be complex, necessitating the coordination of multiple medications, lab tests, and physical visits with a healthcare provider. Health-related information is often stored in an electronic health record (EHR), wherein patient information is centrally stored and accessible to authorized users, such as the patient's doctors, nurses, and pharmacists. By including EHR integration as described herein, the present disclosure provides continuity of care between the clinic and home, which is crucial when medications are given in both settings, as is true in the case of a medication regimen, such as for oncology.

EHRs may also contain orders, which are instructions to care for, diagnose, and treat each patient. Referring to Figure 10, in one or more embodiments, the drug delivery system 1020 is provided with a controller 1026 that communicates with components of the apparatus via either a wired or wireless connection. In one or more embodiments, the controller according to one or more embodiments comprises a processor 1023, a memory coupled to the processor 1024, input/output devices 1025 coupled to the processor 1023, an EHR interface 1021 coupled to the processor, and support circuits to provide communication between the different components of the system, namely the components of the system described herein. In one or more embodiments, processes to operate the system are stored in the memory 1024 as a software routine that, when executed by the processor, causes the system to perform methods described in the present disclosure. In one or more embodiments, the processes to operate the system are performed in hardware. In one or more embodiments, the software routine to operate the system may also be stored and/or executed by a second processor that is remotely located from the hardware being controlled by the processor.

In some embodiments, the EHR interface 1021 is implemented with a Wi-Fi, wireless local area network (WLAN), Bluetooth, near field communication (NFC), cellular, or internet protocol (IP) connection. In some embodiments, redundant input/output interfaces are provided if one communication interface fails. In some embodiments, the EHR interface 1021 features end to end encryption. In some embodiments, the EHR interface 1021 interface is implemented with an application programming interface (API).

The drug delivery apparatus or system 1020 interfaces with an EHR system 1000 via EHR interface 1021 and is thereby associated with one or more specific medication orders 1001 related to a therapeutic medication 1027. In some embodiments, the association includes corresponding order parameters 1007 and administration time 1008 for a therapeutic medication 1002. In some embodiments, the drug delivery apparatus or system is associated with one or more specific medication orders 1001 contained within EHR system 1000 via EHR interface 1021 and corresponding order parameters contained within the EHR system, wherein the order parameters include an identifying number 1005, prescriber 1009, medication name 1002, and administration parameters 1007 and time 1008. In some embodiments, the drug delivery apparatus or system 1020 is associated with one or more specific medication orders 1030 (shown in Figure 10B) contained in one or more order sets 1030 contained within the EHR system 1000 via EHR interface 1021.

Order sets may also be provided in EHR systems, comprising aggregation of multiple orders related to a single condition, process, or clinical situation, such as administration of one or more therapies to treat a disease. In some embodiments, the drug delivery system interfaces with an EHR system 1000 via EHR interface 1021 and is thereby associated with one or more specific medication orders 1001 contained in one or more order sets 1030 within an EHR system 1000, wherein the order sets contain administration instructions for one or more therapeutic medications 1032, medications given prior to 1031 and after 1033 one or more therapeutic medications 1032, and/or standing orders related to emergency medication administration 1033. In some embodiments, the drug delivery apparatus or system 1020 is associated with one or more specific medication orders 1001 contained in one or more order sets 1030 within an EHR system 1000, wherein the order sets contain physiologic monitoring instructions 1037 for a given patient.

Prior to administration, orders and order sets are also used in clinical practice to dispense medications to specific patients, and to verify that the proper medicines are dispensed to each patient. In some embodiments, referring to Figure 10C, the drug delivery apparatus or system 1020 is associated with one or more specific medication orders 1001 within an EHR system 1000, and the drug delivery apparatus or system 1020 contains means by which the contents of reservoir 1027' holding the therapeutic medication 1027 may be verified at 1011 against the order 1001 by a healthcare provider 1010 prior to dispensing to the patient.

In some embodiments, the drug delivery apparatus or system 1020 is associated with one or more specific medication orders 1001 contained in one or more order sets 1030 within an EHR system 1000, wherein the medication orders are referenced on the drug delivery apparatus or system using a barcode or data matrix 1022 that can be scanned by equipment connected to the EHR system 1000.

In some embodiments, the order set 1030 comprises one or more instructions for administration of one or more therapeutic medications 1032, administration of one or more pre-medications 1031 or post- medications 1032 related, administration of one or more emergency medications 1033, required laboratory values or patient monitoring 1034, or other instructions to nursing 1035, 1036, 1037.

In certain cases, administration of a medication may be subject to specific laboratory values being within specified ranges set forth in one or more medication orders 1001 or order sets 1030. Review of laboratory values may be performed manually by a healthcare provider, or through automated decision support within the EHR system. In some embodiments, the drug delivery apparatus or system 1020 is associated with one or more specific medication orders 1001 contained in one or more order sets 1030 within an EHR system 1000, wherein the order sets permit administration of one or more therapeutic medication(s) 1030 pending review of one or more diagnostic or laboratory criteria 1035 contained elsewhere in the EHR system 1000, wherein the review is completed by a healthcare provider. In some embodiments, the drug delivery apparatus or system 1020 is associated with one or more specific medication orders 1001 contained in one or more order sets 1030 within an EHR system 1000, wherein the order sets permit administration of one or more therapeutic medication(s) 1030 pending review of one or more diagnostic or laboratory criteria 1035 contained elsewhere in the EHR system 1000, wherein the review is completed automatically by a decision support tool also contained within the EHR system 1000.

Medication orders and order sets provide administration instructions, including administration rates. So-called "hard" limits cannot be overridden by a healthcare provider, while so-called "soft" limits may be overridden by a healthcare provider based on professional judgment. Embodiments of the present disclosure allows both types of limits to be implemented. In some embodiments, the drug delivery apparatus or system 1020 is provided with an EHR interface 1021 and is associated with one or more specific medication orders 1001 within an EHR system 1000, wherein the medication orders and EHR interface prohibit administration of one or more therapeutic medication(s) at parameters that are unsafe or clinically inappropriate, and wherein the prohibition may not be overridden by one or more healthcare providers 1010 in the interest of patient safety. In some embodiments, the drug delivery apparatus or system 1020 is provided with an EHR interface 1021 and is associated with one or more specific medication orders 1001 within an EHR system 1000, wherein the medication orders and EHR interface prohibit administration of one or more therapeutic medication(s) 1027 at parameters 1007 that are unsafe or clinically inappropriate, and a means for one or more healthcare providers 1010 to override such prohibition based on clinical judgment.

In some embodiments, the drug delivery apparatus or system 1020 is provided with an EHR interface 1021 and is associated with one or more specific medication orders 1001 within an EHR system 1000, and wherein communication between the EHR interface 1021 and drug delivery apparatus or system 1020 is bi-directional, allowing clinician review 1038 of the order 1001's corresponding parameters and administration progress thereto within the health record system.

In another aspect, the drug delivery system controller herein is provided with an input/output interface to allow communications between the administration location and a remote monitoring service. In some embodiments, all sensor data collected by the drug delivery apparatus or system is communicated to the remote monitoring service by the controller. In some embodiments, a subset of sensor data collected by the drug delivery apparatus or system is communicated to the remote monitoring service by the controller. In some embodiments, the remote monitoring service is manned by a healthcare provider. In some embodiments, the remote monitoring service is a computing apparatus or system. In some embodiments, the remote monitoring service is a healthcare provider aided by a decision support tool implemented in software. In some embodiments, the decision support tool employs a predictive or machine learning algorithm. In some embodiments, the decision support tool is an electronic health record (EHR) system.

In some embodiments, the drug delivery system is programmed based on an order set to monitor specific vital signs contained in one or more orders contained in an order set. In some embodiments, the drug delivery system is programmed to deliver specific therapeutic medications based on an individual medication order or orders contained within an order set. In some embodiments, the drug delivery system is programmed to allow delivery pending availability of certain laboratory test results contained within the EHR. In some embodiments, the drug delivery system is programmed to allow delivery only upon confirmation from the EHR that certain laboratory values are within predefined ranges. In some embodiments, the drug delivery system is programmed to prohibit delivery if certain laboratory values contained within an EHR are unavailable or outside predefined ranges. In some embodiments, the drug delivery system is programmed to prohibit delivery if certain laboratory values contained within an EHR are unavailable or outside predefined ranges unless the prohibition is overridden by a healthcare provider. In some embodiments, the drug delivery system is programmed to prohibit delivery if certain laboratory values contained within an EHR are unavailable or outside predefined ranges unless the prohibition is removed automatically by a decision support tool contained within the EHR.

One or more embodiments of the disclosure utilize at least one controller which can be coupled to various components of the apparatus and systems as described herein. In some embodiments, there are more than one controller connected to the individual components a primary control processor is coupled to each of the separate processors to control the system or apparatus described herein. The controllers may be one of any form of general-purpose computer processor, microcontroller, microprocessor, etc., that can be used in an industrial setting for controlling various delivery and/or treatment regimens.

A controller can have a processor, a memory coupled to the processor, input/output devices coupled to the processor, and support circuits to provide communication between the different electronic components. The memory can include one or more of transitory memory (e.g., random access memory) and non-transitory memory (e.g., storage). The memory, or computer-readable medium, of the processor may be one or more of readily available memory such as random access memory (RAM), read-only memory (ROM), floppy disk, hard disk, or any other form of digital storage, local or remote. The memory can retain an instruction set that is operable by the processor or controller to control parameters and components of the apparatus and methods described herein. The support circuits are coupled to the processor for supporting the processor in a conventional manner. Circuits may include, for example, cache, power supplies, clock circuits, input/output circuitry, subsystems, and the like.

Processes and methods such as treatment regimens may generally be stored in the memory as a software routine that, when executed by the processor, causes the apparatus and systems described herein to perform methods described in the present disclosure. The software routine may also be stored and/or executed by a second processor (not shown) that is remotely located from the hardware being controlled by the processor. Some or all of the method of the present disclosure may also be performed in hardware. As such, the process may be implemented in software and executed using a computer system, in hardware as, e.g., an application specific integrated circuit or other type of hardware implementation, or as a combination of software and hardware. The software routine, when executed by the processor, transforms the general purpose computer into a specific purpose computer (controller) that controls the chamber operation such that the processes are performed.

In some embodiments, the controller has one or more configurations to execute individual processes or sub-processes to perform the methods described herein.

Reference throughout this specification to "one embodiment," "certain embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in one embodiment" or "in an embodiment" in various places throughout this specification are not necessarily referring to the same embodiment of the disclosure. Furthermore, the particular features, structures, materials, or characteristics may be combined in any suitable manner in one or more embodiments.
Although the disclosure herein has been described with reference to particular embodiments, those skilled in the art will understand that the embodiments described are merely illustrative of the principles and applications of the present disclosure. It will be apparent to those skilled in the art that various modifications and variations can be made to the method and apparatus of the present disclosure without departing from the scope of the disclosure Thus, the present disclosure can include modifications and variations that are within the scope of the appended embodiments.

in another aspect, embodiments of the disclosure generally relate to apparatus, systems and methods for administration of therapeutic medicines. More particularly, embodiments of the disclosure relate to apparatus, systems and methods configured to control fluid flow within a tubing set within a drug delivery system, especially for biologic medicines.

Liquid medication formulations are commonly administered through tubing sets, and the ability to control flow rates through a tubing set is highly desirable for medication delivery. Many configurations of tubing sets and flow controls are available on the market.

Certain existing devices control flow rate with a pre-defined inner diameter, each corresponding to a flow rate for a given medication. For instance, two tubing sets, each with different internal diameters, may be labeled for 150 mL/h or 300 mL/h when using a specific fluid, such as saline. One disadvantage of this approach is that if an intermediate flow rate is desired (e.g., 200mL/h), another dedicated tubing set must be manufactured, adding cost and inefficiency.

A kit of components with multiple tubing sets may also be fluidically combined in series or parallel, allowing "mix and match" construction of a set providing a desired flow rate. For example, connection of two 100mL/h tubing sets in parallel allows construction by a user of a 200mL/h set, allowing efficient production of a single 100mL/h set at scale. While efficient from a manufacturing perspective, significant burden is placed onto the user, who must understand the concepts of fluid mechanics sufficiently to select and assemble the correct components in the right combination. This may prove challenging or inconvenient for users without clinical training, as would be present in a home setting. Moreover, it may present medication safety risks that are not apparent to an untrained user. Incorrectly assembled or missing components in such a system will provide flow rates substantially different than intended, leading to over- or under-delivery of medication and potential adverse events. The same risk may also be present if one or more components of the kit is unavailable due to a backorder or product recall. The Institute for Safe Medication Practices error reporting program has documented cases of patient hospitalization and death due to incorrect selection of rate control tubing and corresponding overdose in the home setting.

Another alternative is to provide a variable rate controller on a tubing set, allowing a user to increase or decrease the flow rate during administration, for instance, with an adjustment knob. If a medication is provided with a prescribed administration rate of 50mL/h, such a device would be provided in the "off" (0 mL/h) state at dispensing, and the user would be required to set the flow rate to 50mL/h on the rate controller accurately during administration. Healthcare providers in clinical settings are familiar with such devices and can use them safely. In the home setting, this presents potential safety risks to users, who often lack clinical training. They may increase the flow rate beyond that prescribed to complete administration sooner without understanding the pharmacokinetic effects of such an increase. They may misunderstand the instructions on a prescribed medication or for use of the rate control and set the rate incorrectly. In some instances, rate control devices may be provided to simply stop or start flow, even when specific flow rate control is not needed; untrained users may not understand and inadvertently make unintended flowrate adjustments.

Notably, both of these existing tubing set assemblies designed for constant preset flow and a rate controller both provide flow rates that are calibrated on a specific fluid. Most commonly, this fluid is a Newtonian fluid, such as water or saline, in which the viscosity remains constant with shear strain, defined as a proportional constant (coefficient of viscosity).

When used with non-Newtonian medications, different tubing sets and rate controllers may deliver drastically different flow rates. Certain medicines, such as biologics or long-acting injectable (LAI) formulations may display non-Newtonian behaviors, wherein the relationship between viscosity and shear strain may not be defined by a single constant. Shear thickening fluids exhibit increasing viscosity with increasing shear rate, while shear thinning fluids exhibit decreasing viscosity with increasing shear rate.

Biologics may also exhibit strong temperature-viscosity-concentration relationships which are a significant design consideration for drug delivery devices, and fluidic components such as tubing sets specifically. To reduce dosing frequency for patients, pharmaceutical companies desire higher biologic concentrations within a formulation, leading to generally higher viscosity that becomes exponentially more viscous with decreasing temperature. Thus, medications may flow differently in tubing sets with different inner diameters (and thus different shear rates) or fluid restrictors, and warmer or colder biologic medicines may exacerbate these behaviors in a way that is not obvious to an untrained user. In addition, a tubing set labeled for a specific flow rate (e.g., 900mL/h) with saline, a Newtonian fluid, may deliver a much lower flowrate (e.g., 60mL/h) with a more viscous non-Newtonian fluid, requiring complex conversion tables that patients and dispensing pharmacists must interpret (and may interpret incorrectly). Moreover, these nuances may not be apparent to the patient or dispensing pharmacist, resulting in the incorrect tubing set being dispensed to a patient. These shortcomings may result in dangerous under- or over-doses, with particularly deleterious safety and efficacy implications for administration of potent biologic medicines. These limitations with existing devices result in patients using a product that delivers medication differently than labeled. There is a need to avoid this issue.
Moreover, fine-grained control over flowrate provided by existing devices is often unneeded. Many medications have fixed dose regimens, where all patients receive a specific dose at a specific flow rate. Other medications commonly have "dose bands," where patients receive one of several discrete fixed doses, often based on body weight. Both scenarios are common with biologic medicines, where doses and rates do not vary from administration to administration. In these administration scenarios, the complexity of prior art systems is not only unneeded but also encourages unwanted or unsafe adjustment, particularly by a user without clinical training or with low health literacy.

Embodiments provide apparatus, systems and methods for administering large volumes of parenteral or enteral medicines at one or more predetermined rates, particularly non-Newtonian medicines exhibiting a concentration-viscosity-temperature relationship, such as a biologic medicine.

Before describing several exemplary embodiments of the disclosure, it is to be understood that the disclosure is not limited to the details of construction or process steps set forth in the following description. The disclosure is capable of other embodiments and of being practiced or being carried out in various ways.

The required dosing and associated flow rate of therapeutic medicines are intended to induce a specific physiologic effect in the patient receiving the medication. However, especially in the case of biologic medicines, the concentration, dose volume, and dosing rate are finalized through human clinical trials. Thus, dosing parameters needed to design delivery components, including a tubing set, are likely not be finalized until late in the drug development process after human clinical trial data becomes available. Given the non-Newtonian behavior of biologic medicines, changes to these parameters may require substantial redesign of fluidic components, including tubing sets. Thus, improved apparatus, systems and methods are needed to provide flexibility in clinical trial design and execution, and to accommodate late-stage formulation or drug concentration changes without the time and cost associated with a full redesign of the administration components. The present disclosure fulfills one or more of these needs.

Existing devices are focused on tubing sets designed to be used with a variety of different medicines and provide solutions that are inefficient and impractical to implement in a tailored fashion for a specific medication. The onus is placed on the user of existing solutions to adapt to the apparatus available. Instead, ready-to-use administration supplies providing accurate, discrete flow rates for medications with fixed-dose or dose-banded administration are provided by embodiments of the present disclosure. At the same time, manufacturing processes according to one or more embodiments disclosed herein allow for efficient high-volume manufacture of tubing sets tailored to a specific medication and one or more administration conditions, without placing undue burden on an untrained user or compromising medication safety as required by prior art solutions.

Finally, in many cases, it is desirable to administer numerous medications, especially as part of a medication regimen. Individual medications are often part of a larger regimen of medicines, with standardized regimens corresponding to a specific disease state, treatment regimen, or medication. A regimen may also include one or more pre-medications, post-medications, or emergency medications. Each of the foregoing may be administered to a patient at a flow rate different than a therapeutic medication, or on a contingent basis, as in the case of an emergency medication administered contingently upon detection of a systemic infusion reaction. Many existing solutions are limited to apparatus or kits of components that adjust flow rate of a single medication, but do not allow independent adjustment of flow rates for multiple medications. Embodiments of the present disclosure meet one or more of these needs.

Embodiments of the disclosure provide apparatus, systems and methods for administering parenteral or enteral medicines to a patient via a tubing set at one or more predetermined rates. The apparatus or system allows for different configurations of tubing sets to effectuate medication administration to a patient. More specifically, one or more embodiments of the present disclosure provides a device and approach to make a single tubing set, capturing the manufacturing efficiencies at scale, and then constrain the tubing set during manufacture to one or more discrete, preset rates that the end user cannot modify. While the tubing set may be used for a variety of medicines, the constraints may be easily and precisely molded for a specific medication and flow rate, and when provided with indicia as disclosed herein, provide a true flow rate for the specific medication, avoiding mix-ups, confusion, and medication errors. Embodiments of the present disclosure is particularly applicable to biologic medicines that exhibit non-Newtonian behaviors, such as biologic medicines, that are administered by untrained users in a home setting to treat chronic diseases.

One or more embodiments of the disclosure is directed to providing apparatus, systems and methods to constrain a tubing set and one or more internal medication lumens situated therein/ thereby reducing the flow rate to a specific predetermined rate. One or more constraints as described in embodiments herein are applied to the exterior of a tubing set, reducing the flow rate in one or more lumens of the set, and establishing a maximum flow rate for a lumen so constrained. Geometry of the constraints is matched to a specific therapeutic medication and one or more desired flow rates of the medication. The constraining profile in contact with the tubing set is fashioned with a generally rounded section to provide a gradual reduction of flow from the unconstrained inner diameter to the constrained one, which is particularly advantageous to avoid damage to a therapeutic protein. The length of the constraint may also be varied in conjunction with the constraint profile to enable a gradual reduction in flow. The design of the constraint is such that manufacturing and assembly may be conducted in an automated, efficient manner.

Further embodiments of the disclosure are directed to providing apparatus, systems, and methods to constrain a tubing set and one or more internal medication lumens situated therein, thereby reducing the flow rate to one of several specific predetermined rates during assembly of the constraint apparatus. In one or more embodiments, an adjustable constraint apparatus is provided with one or more discrete adjustment levels corresponding to one or more levels of progressively compressive assembly, successively reducing the flow rate in or more lumens of the set, wherein each distinct compression step corresponds to one flow rate of a specific medication at a specific administration condition, such as room temperature. In one or more embodiments, the constraints are designed to be adjustable in a manufacturing facility by automated equipment while preventing inadvertent or intentional adjustment by a user afterwards.

Additional embodiments of the disclosure are directed to providing apparatus, systems, and methods to accommodate administration of a variety of non-Newtonian biologic medications at a variety of flow rates. In the case of a medication exhibiting a concentration-viscosity-temperature relationship, such as a biologic medication, the flow rates provided by the constraint may optionally be calibrated to provide an intended flow at a specific temperature corresponding to the anticipated medication administration conditions, such as room temperature. In one or more embodiments, the constraint apparatus described herein may be calibrated to one or more desired flow rates during manufacturing for a specific therapeutic medication of interest, such as a biologic medication.

In one or more embodiments, one or more inner medication lumens constrained as described herein inherently limit allowable flowrates through the system at or below the clinically safe or tolerable of one or more medications administered through one or more of the lumens in the tubing set. In some embodiments, the constraint establishes a maximum flow rate for a single internal tubing lumen. In some embodiments, the constraints are placed to establish different maximum flow rates for a two or more internal tubing lumens independently. In one or more embodiments, the constraint apparatus includes an emergency clamp to completely restrict flow or allow flow at the maximum rate provided by the constrained tubing set lumen.

As described herein, the constraints are preferentially injection molded, providing tight dimensional control over the constraint geometry and enabling low-cost manufacturing and efficient assembly. In one or more embodiments, the constraint is designed symmetrically, allowing a single part to be used in pairs during an assembly operation. In one or more embodiments, the constraints are provided with an assembly force intentionally exceeding the physical ability of a human, allowing adjustment by manufacturing and assembly equipment while preventing unintended or inadvertent adjustment by an end user.

To improve intuitiveness for untrained users, especially in the home setting, in one or more embodiments, the constraints may be provided with human readable indicia regarding the drug name, flow rate, and other relevant information to avoid medication errors and improve intuitiveness for untrained users. In one or more embodiments, the constraints may also be provided with machine-readable indicia, such as a data matrix or NFC chip, to enable automated inspection and ensure that a therapeutic medication is provided only with the correct corresponding tubing set once constrained as described herein. In one or more embodiments, the constraint members are provided with contours or indicia to allow inspection by automated equipment to verify proper component selection, assembly, constraint level, or flow rate at a given administration temperature.

As will be appreciated by one skilled in the art, there are numerous ways of carrying out the examples, improvements and arrangements of the apparatus, systems and methods disclosed herein. Although reference will be made to the exemplary embodiments depicted in the drawings and the following descriptions, the embodiments disclosed herein are not meant to be exhaustive of the various alternative designs and embodiments that are encompassed by the present disclosure.

Embodiments of the constraint apparatus systems and methods described herein are for use with a tubing set featuring one or more inner medication lumens for administration of medicines to a patient as part of a drug delivery system. During use of a drug delivery system, a first end of the tubing set constrained as described herein is in fluidic communication with a drug delivery system, and a second end of the tubing in fluidic communication with a patient interface that delivers medications contained within the drug delivery system to the patient. In some embodiments, the patient interface comprises a subcutaneous, intramuscular, or intravenous needle set. In some embodiments, the patient interface comprises a Huber needle for accessing an implanted intravenous port. In some embodiments, the patient interface comprises a luer taper, Luer-Lok^{®} connector, luer-activated valve, luer-activated septum, or other luer-activated access device. In some embodiments, the patient interface comprises a threaded or snap-fit non-luer connector.

Each of the embodiments described with respect to Figures 14-17C may be combined with the embodiments described in Figures 1-10C and the corresponding claims. Thus, the embodiments described with respect to Figures 14-17C may be incorporated or combined with the apparatus, systems and methods of numbered embodiments 154-179 below.

Figure 14A shows an alternative exemplary embodiment of an external constraint apparatus to constrain a tubing set for a drug delivery system to a single flow rate. In one or more embodiments, the fully assembled apparatus comprises an interlocking set comprising a first constraint member 1401 and second constraint member 1402. The first constraint member 1401 contains a first contoured constraint profile 1403 that compressively engages the exterior of a tubing set 1405 when assembled to the second constraint member 1402. Likewise, the second constraint member 1402 contains a second contoured constraint profile 1404 that compressively engages the exterior of a tubing set 1405 when assembled to the first constraint member 1401. In one embodiment, the locking mechanism comprises a set of mating locking fingers 1407, 1410 and apertures 1408, 1409, whereby locking finger 1410 is disposed to align with aperture 1408 and locking finger 1407 is disposed to align with aperture 1409, both during a compressive assembly operation 1406, 1406' with the tubing set 1405 interposed between the constraint members 1401, 1402. In one or more embodiments, the compressive assembly operation 1406, 1406' comprises two substantially equal opposing forces.

In one or more embodiments, the first and second constraint members 1401, 1402 may be identical, wherein the two constraint members may be assembled when oriented 180° to each other, improving molding, manufacturing, inspection, part handling, and assembly.

Figures 14A & 14B illustrate a constraint for a single lumen; however, this concept may be extended to tubing sets with multiple lumens, as illustrated in Figure 18. A pair of constraint members may be provided in accordance with the prior description with a plurality of contoured segments, each of the contoured segments corresponding to a desired degree of constraint (or no constraint) for a specific tubing lumen. One or more constraint members may be contoured so the constraint may be properly oriented before engaging the outer surface of the tubing set, and may optionally be provided with a keyed feature to prevent misorientation prior to assembly, akin to the cooperating apertures and locking fingers described in Figure 14A.

In more detail, Figures 18A and 18B show an example with variable compression of a multi-lumen tube. A first constraint member 1801 and a second constraint member 1802 are shown relative to a tubing set 1805 in Figure 18A; the tubing set is unconstrained. In this example, the tubing set 1805 comprises three lumens 1806, 1807, 1808, although two, four or more lumens could alternatively be provided. All three lumens could be medication lumens, though the function of the lumens could also be varied, as discussed elsewhere herein. In Figure 18B, tubing set 1805 of Figure 18A is shown compressed by the constraint members 1801, 1802. Due to the internal shape of the constraint members 1801, 1802, the lumen 1806 is uncompressed, the lumen 1807 is compressed, and the lumen 1808 is compressed, with the degree of compression of the lumen 1808 being greater than the degree of compression of the lumen 1807. By varying the internal shape of the constraint members, some or all of the lumens can be compressed, and the lumens can be compressed to different degrees. The shape of the compressed lumens can also be varied by varying the internal shape of the constraint members. An example of an alternative internal shape for the first constraint member 1801 is shown in Figure 18E; in this example, the internal corners are curved to help minimize or avoid damage to the tubing.

Figure 18C shows another possible internal shape from the first constraint member 1801. In Figure 18C, another optional feature of the first constraint member 1801 is shown, namely a protrusion 1810 that is configured to engage a corresponding recess 1811 in the tubing set 1805. The keying structure created by the combination of the protrusion 1810 and the recess 1811 can help ensure that the constraint members 1801, 1802 and the tubing set 1805 are correctly aligned. Only one keying structure is shown, but one or more keying structures could be provided. Various other keying structures could also be used, for example with the protrusion on the tubing set and the recess on a constraint member, or with a snap fit. An alternative keying structure is shown in Figure 18H, with a non-fitting constraint member 1831 shown on one side and a fitting constraint member 1832 shown on the other side. In the example in Figure 18H, differing positions and shapes of protrusion and recess are used for the keying structures - differing both the shape and position is optional, but can help ensure that assembly is carried out correctly.

Figure 18D shows a top view of the constraint members and the tubing set of Figure 18A, thereby giving an example of what the structure could look like from the outside. In this example, an optional arrow is provided on the outer surface of the first constraint member 1801 to indicate orientation of the constraint members, which can help with assembly. The smaller arrows in the Figure 18D show the fluid flow direction in the tubing set when in use.

Figures 18E and 18F show another example, with a first constraint member 1821, a second constraint member 1822 and a tubing set 1825, which in this case comprises a single lumen. In Figure 18E, the tubing set is fully open. In Figure 18F, the tubing set is compressed. The level of compression can be varied by moving the position of the constraint members relative to one another, but hooks 1827 on the first constraint member 1821 and corresponding recesses 1828 on the second constraint member engage so that once the constraint members have been moved towards one another, they cannot be pulled apart again. This can help ensure that a user cannot tamper with a constraint setting applied by a pharmacist, for example. This can be achieved in various ways, such as with a ratchet and/or snap-fit arrangement.

In one or more embodiments, the first and second constraint members 1401, 1402 comprise different geometries that may be oriented and assembled as described herein. In one or more embodiments, the first and second constraint members 1401, 1402 also comprise assembly features to improve orientation and feeding of the device during high-speed assembly, by way of example with a feeding rail or vibratory bowl/rail feeder system. In one or more embodiments, the assembly features comprise a cross sectional design providing a predictable center of gravity to improve feeding and orientation of the constraints during high-speed assembly. In one or more embodiments, the assembly features comprise one or more slots in one or more exterior surfaces of the constraint members. In one or more embodiments, the assembly features comprise one or more protrusions on one or more exterior surfaces of the constraint members.

After compressive assembly 1406, 1406', and now referring to Figure 14B, the first constraint member 1401 and second constraint member 1402 creates a constrained inner diameter 1433 within the interposed tubing set 1405, whereas the portion of the tubing set 1405 outside the constraint members 1401, 1402 remains unconstrained 1414. The degree of constraint provided by assembled constraint members 1401 and 1402 to the tubing set 1405 is dependent on one or more of the geometry of the constraint profiles 1403 and 1404, the orientation of the constraint profiles 1403 and 1404 relative to the locking fingers 1407 and 1410 and the apertures 1408 and 1409, the length of the first constraint member 1412, the length of the second constraint member 1413, the outer diameter of the tubing set 1405, the diameter of the medication lumen 1411, the three-dimensional contact area between the constraint profiles 1403 and 1404 and tubing set 1405, and the material of the tubing set 1405.

For certain medicines, it may be desirable to provide a long, smooth transition from unconstrained to constrained inner tubing lumen to avoid protein damage or aggregation due to shearing effects at the medication-tubing lumen interface. Further, the risk of protein damage or aggregation due to shearing effects may vary based on the degree of tubing constraint. In some embodiments, the length of the constraint members 1412, 1413 parallel to the tubing set lumen 1411 axis may be extended to provide a smooth, gradual decrease in flow from the unconstrained portion 1414 to the constrained portion 1433, thus avoiding damage to or aggregation of protein-based or biologic medicines. In some embodiments, the contour of the constraint members 1403, 1404 may vary independently for each flow rate permitted by the constrained section. In some embodiments, the contour of the constraint members 1403, 1404 is engineered to smooth flow reduction while avoiding damage to or shearing of a protein medication flowing through a tubing set lumen 1411.

In an alternative embodiment shown in Figure 14B, the first contoured constraint profile 1403 and second contoured constraint profile 1404 may be designed to provide a substantially asymmetric cross-sectional constraint when reflected across the long axis of the tubing set 1405 once assembled as described herein. In an alternative embodiment, the first contoured constraint profile 1403 and second contoured constraint profile 1404 may be designed to provide a substantially symmetric cross-sectional constraint when reflected across the long axis of the tubing set 1405 once assembled as described herein. Although Figure 14B and the accompanying specification shows an asymmetric constraint profile by way of example, it will be clear to those skilled in the art that any arrangement of constraint profiles may be accommodated in the unassembled constraint members and assembled constraint members, and the foregoing example shall not be construed as limiting the apparatus to an asymmetric constraint upon the tubing set once assembled.

Referring to Figures 14A and 14B, locking fingers 1407, 1410 may be alternatively secured into the apertures 1408, 1409 in any suitable manner, including press fit, ultrasonic welding, heat staking, adhesive cured by exposure to ultraviolet light after application, or other suitable adhesives. In another embodiment, a UV adhesive is applied to either or both of the apertures 1408, 1409 or locking fingers 1407, 1410 prior to assembly and cured after compressive assembly as described herein to prevent further movement or inadvertent adjustment of the constraint apparatus after assembly.

Although the tubing set 1405 is shown in Figures 14A and 14B as having a substantially circular cross section, the apparatus herein may be readily adapted to other tubing designs and configuration of tubing set cross-sections and variations of lumens and conductors situated therein. The present apparatus may, for example, accommodate elliptical designs with cross-sectional designs featuring one or more medication lumens or other non-fluidic conductors disposed therein. Thus, the illustration of tubing set 1405 and accompanying specification is provided by way of illustration and not limitation.

Figures 15A and 15B show an alternative exemplary embodiment of an external constraint apparatus to constrain a tubing set for a drug delivery system to one of several discrete degrees of constraint in an adjustable manner during manufacture and assembly while preventing further movement or inadvertent adjustment of the constraint apparatus after assembly.

Referring to Figure 15A, in one or more embodiments, the fully assembled apparatus comprises an interlocking set comprising a first constraint member 1501 and second constraint member 1502. The first constraint member 1501 contains a first contoured constraint profile 1503 that compressively engages the exterior of a tubing set 1505 with unconstrained diameter 1523 placed through the tubing set entrance 1521 and subsequently assembled to the second constraint member 1502. Likewise, the second constraint member 1502 contains a second contoured constraint profile 1504 that compressively engages the exterior of a tubing set 1505 placed through the tubing set entrance 1522 and subsequently assembled to the first constraint member 1501. In one embodiment, the locking mechanism comprises a set of mating locking fingers 1507, 1510 and apertures 1508, 1509, whereby locking finger 1510 is disposed to align with aperture 1508 and locking finger 1507 is disposed to align with aperture 1509. Once aligned, locking fingers 1507, 1510 are configured to progressively engage with mating apertures 1508, 1509 as the constraints are assembled around an interposed tubing set 1505 using one or more increments of applied compressive forces 1506, 1506'.

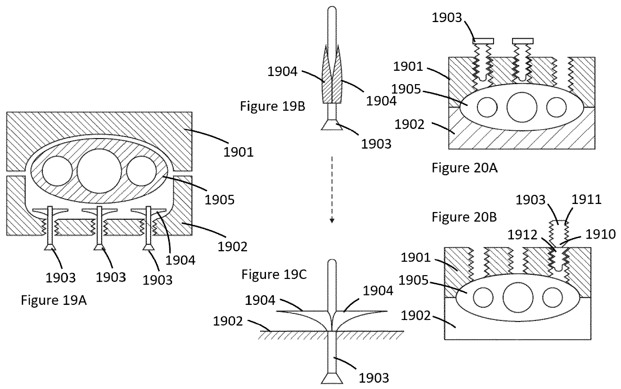

Referring to Figures 19 and 20, a ratcheting or threaded plunger may be situated in relationship to a desired tubing lumen to be constrained, wherein each plunger is independently adjustable. The plunger may also have a mechanism to prevent removal of a given constraint once adjusted. As previously mentioned, these examples show a tubing set with three lumens, but other numbers of lumens could also be used.

In more detail, Figure 19A shows a first constraint member 1901 and a second constraint member 1902 with a tubing set 1905, with the tubing set 1905 optionally having three lumens in this particular example as in the example in Figure 18A. In this example, three plungers 1903 can be seen; the plungers 1903 extend through the second constraint member 1902. In this way, the different lumens can independently be constrained. Optionally, the level of constraint on one or more of the lumens can be varied dynamically during use. Figure 19B shows a plunger 1903 with arms 1904 folded away. The arms can then be folded out, as shown in Figure 19C (and also in Figure 19A), to prevent removal of the plunger. Optionally, screwing or ratcheting the plunger in to the second constraint member 1902 results in the arms opening.

Figure 20A shows another option in which the plunger 1903 is simply a screw, again allowing individual manipulation of the compression level of each lumen. Figure 20B shows another option in which the plunger 1903 has a weak point 1910, so that the outer part 1911 of the plunger breaks off at a set torque, thereby leaving the inner part 1912 of the plunger in a fixed position. This approach can help avoid accidental manipulation of the plunger position after assembly. Breaking apart at a set torque like this can also allow the plunger to finish in a fixed position (at a point where a certain amount of resistance is provided by the squeezed tubing set), thereby potentially simplifying assembly.

Referring to Figure 12B, the constraint members may optionally be provided with a stress relief 1520 to allow for lower force assembly of the locking fingers and apertures, particularly if a rigid material is selected. The tubing set entrances 1521, 1522 need not fully enclose the tubing set 1505, and one or more entrances 1521, 1522 may be extended centrally towards the tubing set 1505, as in the form of one or more clearance slots, to facilitate more straightforward orientation around the tubing set and subsequent assembly.

The arrangement and spacing of apertures and locking fingers permits constraint of a tubing set to one or more discrete levels using a single set of constraint members, with the constraint level selected during the manufacturing operation. Figure 15C shows an exemplary illustration of assembly of the apparatus to provide three levels of successively greater constraint upon a tubing set during assembly. Applying a first compressive assembly force 1555, 1555' to the first and second constraint members 1501, 1502 advances the apertures and locking fingers to a first position 1551, providing a corresponding first degree of tubing constraint 1561 corresponding to a first fixed flow rate. Applying a second compressive assembly force 1556, 1556' to the first and second constraint members 1501, 1502 advances the apertures and locking fingers to a second position 1552, providing a correspondingly higher second degree of tubing constraint 1562 and correspondingly lower second fixed flow rate compared to the first fixed flow rate at the first degree of constraint 1561. Applying a first compressive assembly force 1557, 1557' to the first and second constraint members 1501, 1502 advances the apertures and locking fingers to a third (and in this example, final) position 1553, providing a correspondingly still higher third degree of tubing constraint 1563 and correspondingly still lower third fixed flow rate compared to both the first and second fixed flow rates corresponding to the first and second degrees of constraint 1561, 1562. Although Figure 15C and the accompanying specification shows three positions by way of example, it will be clear to those skilled in the art that any number of positions may be accommodated, and the foregoing example shall not be construed as limiting the apparatus to three degrees of constraint.

In one or more embodiments, the compressive forces 1555 and 1555', 1556 and 1556', and 1557 and 1557' are equal. In one or more embodiments, the compressive forces 1555 and 1555', 1556 and 1556', and 1557 and 1557' are unequal. In one or more embodiments, one or more of the compressive forces 1555 and 1555', 1556 and 1556', and 1557 and 1557' intentionally exceed the compressive force appliable by a human being without the use of mechanical assistance, such as with a tool or fixture. In some embodiments, the compressive force is predetermined, fixed by the design of the constraint members and is configured to prevent modification, tampering, or further adjustment of the compressive force by a user of the apparatus after manufacture and assembly to a tubing set. In one or more embodiments, the third compressive forces 1557 and 1557' exceed those of the second compressive forces 1556 and 1556' and the second compressive forces 1556 and 1556' also exceed those of the first compressive forces 1555 and 1555'.

In one or more embodiments, adjustable constraint members may be constrained adaptively during manufacturing based on the flow characteristics of a medication or medication placebo, which is particularly advantageous when designing fluidic systems for non-Newtonian fluids, such as biologics. Referring to Figure 15C, a first constraint member 1501 and second constraint member 1502 is applied to a tubing set 1505 through a first compressive assembly step 1555 and 1555', thus applying a first degree of tubing constraint 1551. During manufacturing, a medication formulation may be passed from the medication inlet 1571 to a medication outlet 1572, measuring the flow rate at both points, and comparing the difference in flow at the outlet 1572 to the desired flow rate. If the desired flow rate at outlet 1572 is within the tolerance of the expected flow rate, adjustment of the constraint members is completed. If the desired flow rate at outlet 1572 is lower than the expected flow rate, a second compressive assembly step 1556 and 1556' occurs during manufacturing, thus applying a second degree of tubing constraint 1552. A medication formulation is again passed from the medication inlet 1573 to a medication outlet 1574, measuring the flow rate at both points, and comparing the difference in flow at the outlet 1574 to the desired flow rate. If the desired flow rate at outlet 1574 is within the tolerance of the expected flow rate, adjustment of the constraint members is completed. If the desired flow rate at outlet 1574 is lower than the expected flow rate, a third compressive assembly step 1557 and 1557' occurs during manufacturing, thus applying a third degree of tubing constraint 1553. A medication formulation is again passed from the medication inlet 1575 to a medication outlet 1576, measuring the flow rate at both points, and comparing the difference in flow at the outlet 1576 to the desired flow rate. If the desired flow rate at outlet 1576 is within the tolerance of the expected flow rate, adjustment of the constraint members is completed. If the desired flow rate at outlet 1576 is lower than the expected flow rate, additional compressive assembly and flow testing cycles may be completed as described herein. Although Figure 15C and the accompanying specification shows adaptative adjustment during manufacturing for three positions by way of example, it will be clear to those skilled in the art that any number of positions may be accommodated, and the foregoing example shall not be construed as limiting the apparatus to three degrees of constraint during the adaptive manufacture described herein. In one or more embodiments, a first constraint member 1501 and second constraint member 1502 are provided with a plurality of apertures and locking fingers, the plurality comprising a number larger than the number of anticipated compressive cycles to achieve a desired flow rate as described herein.

The apparatus described herein provides a consistent flow rate based on one or more degrees of constraint placed upon a tubing set. It may also be advantageous in certain administration settings to provide the constraint with a slidable clamp to completely halt fluid flow, as in the case of an emergency or device malfunction. Figure 3 shows an alternative embodiment of an external constraint feature that is adjustable to one of several discrete degrees of constraint as described previously, further configured to allow emergency shutoff of a tubing set to prevent any and all flow through the lumens therein.

Referring to Figure 16A, a first constraint member 1601 and second constraint member 1602, each with internal constraint profile, locking fingers, and apertures are provided as described previously herein (refer to Figures 15A-15C), and serving to constrain a tubing set 1600 interposed and compressed between the two assembled constraint members 1601 and 1602, also as described previously. Also captured between the first constraint member 1601 and second constraint member 1602 is a clamp plate 1603 comprising one or more travel tabs 1604 and a clamp profile 1605. Clamp travel tabs 1604 are situated to slidably engage with a clamp travel slot 1606 provided in one or more open arms 1601' of the first constraint member 1601, with the tubing set passing through both constraint members 1601, 1602 and the clamp profile 1605 during assembly. Slidably engaging the clamp plate 1603 within the travel slot 1606 between the clamp travel stops 1606' allows flow in the tubing to be fully stopped or started depending on the position of the tubing set 1600 within the clamp profile 1605.

Figure 16B shows a constraint assembly 1610 comprising first constraint member 1601 and 1602 assembled to constrain a tubing set 1600, the constraint members also capturing a slidable clamp plate 1603 with a tubing set 1600 passing through clamp profile 1605. In one or more embodiments the clamp plate 1603 is slidably positioned into a first, open flow position 1611, permitting the tubing set 1600 to pass unrestricted through the clearance portion in the clamp profile 1605, and the flow within the tubing set being constrained only by the constraint profiles within the first and second constraint members 1601 and 1602. In one or more embodiments, when the position of the clamp plate 1603 is in the open flow position 1611, one or more clamp state indicators 1607 is provided to a user corresponding to the state of the clamp, such as the word "open," "ready," "go," "run," or another suitable term.

Figure 16C shows a constraint assembly 1613 comprising first constraint member 1601 and 1602 assembled to constrain a tubing set 1600, the constraint members also capturing a slidable clamp plate 1603 with a tubing set 1600 passing through clamp profile 1605. In one or more embodiments the clamp plate 1603 is slidably positioned into a second, closed flow position 1612, fully collapsing one or more medication lumens disposed within the tubing set 1600 within the narrow portion of the clamp profile 1605, thus preventing medication flow. In one or more embodiments when the position of the clamp plate 1603 is in the closed flow position 1612, one or more clamp state indicators 1608 is provided to a user corresponding to the state of the clamp, such as the word "closed," "stop," "pause," or another suitable term.

In one or more embodiments, the clamp profile 1605 is designed so that in the open position, the tubing set 1600 may freely pass through the clamp profile 1605 with no interference or further reduction in fluid flow, except as provided by the constraint profiles of the assembled constraint members 1601 and 1602. In one or more embodiments, the clamp profile 1605 comprises a rapidly narrowing design, whereby movement of the clamp profile 1605 from the open 1611 position to a second closed position 1612 quickly halts flow through the tubing set 1600. In one or more embodiments, the clamp profile 1605 is matched to the cross section of the unconstrained tubing set 1600. In one or more embodiments, the clamp is supplied to a user after manufacture in the open flow position 1611, allowing immediate medication administration at the constrained rate provided by the assembly 1610. In one or more embodiments, the clamp is supplied to a user after manufacture in the closed flow position 1612, preventing medication administration until the clamp plate 1603 is slidably moved to an open flow position 1611, thereafter allowing immediate medication administration at the constrained rate provided by the assembly 1610.

Many variations in construction, assembly, and design are possible for one or more embodiments of the apparatus described herein, depending on the clinical application, characteristics of the medication being delivered, tubing set design, desired flow rates of one or more medications, and pharmacokinetics of the medications once in the body.

The constraint members and clamp plates described herein can be made of, by way of example, a plastic material such as polyamide, polycarbonate, acrylonitrile butadiene styrene, polypropylene, high density polyethylene, polyester, polyoxymethylene, other polymers, combinations of polymers, or other materials having suitable rigidity and strength to withstand the assembly operation described herein. Alternatively, the constraint members described herein may be made of a composite material, such as a polymer reinforced with fiberglass, polymer reinforced with aramid fiber, polymer reinforced with carbon fiber, or another suitable composite of fiber- or metal- reinforced polymer. In one or more embodiments, the constraint members are molded from a single material. In one or more embodiments, the constraint members are molded from multiple materials, as with a two-shot or overmolding process. In one or more embodiments, the clamp plate and constraint members are the same materials. In one or more embodiments, the clamp plate and constraint members are different materials. In one or more embodiments, either or both of the clamp plate and constraint members contain a slip additive to reduce sliding friction.

In one or more embodiments, the material for one or more constraint members is chosen for compatibility with gamma, e-beam, or other radiation sterilization methods. In one or more embodiments, the design and material of one or more constraint members is chosen for compatibility with gamma, e-beam, or other radiation sterilization methods. In one or more embodiments, the design of one or more constraint members is chosen to ensure the assembled constraint members retain their strength against inadvertent disassembly or further adjustment after sterilization with gamma, e-beam, or other radiation sterilization methods.

In an embodiment, the design of the locking mechanism is such that the magnitude of the force used to assemble the constraint members into each other via the locking fingers and apertures intentionally exceeds the compressive force that may be manually applied by a human without use of the constraint apparatus, thereby requiring mechanical assembly equipment and preventing intentional or inadvertent post-manufacturing adjustment of the applied constraint apparatus by a user after manufacture. In an embodiment, the design of the locking mechanism is such that the magnitude of the force to separate the assembled constraint members intentionally exceeds the tensile force that may be manually applied by a human without use of the mechanical assistance, such as with a tool or fixture, thereby preventing intentional or inadvertent removal of the applied constraint apparatus by a user after manufacture. In one or more embodiments, the design of the constraint members is configured to prevent access to either or both of the locking fingers and apertures, discouraging tampering, removal, or further adjustment by a user of the apparatus after manufacture and assembly to a tubing set.

Other suitable locking mechanisms may be substituted to secure the constraint members including press fit, ultrasonic welding, heat staking, adhesive cured by exposure to ultraviolet light after application, or other suitable adhesives. In one or more embodiments, the assembly method is selected to reduce leachable or extractable components that may be introduced from the assembled constraint members through the wall of an interposed tubing set and one or more medications flowing within one or more tubing set medication lumen(s) situated therein. In another embodiment, a selectively curable adhesive is applied to either or both of the apertures and locking fingers on either or both constraint members prior to assembly, the members are assembled, and the adhesive is cured, thus preventing further movement or inadvertent adjustment of the constraint. In one or more embodiments, the selectively curable adhesive comprises an ultraviolet-cure adhesive.

In one or more embodiments, it may be desirable to isolate the material(s) comprising the constraint members or one or more adhesives used to assemble the constraints from the tubing set and one or more medications flowing therein. Accordingly, in one or more embodiments, some or all of the contoured constraint profiles may be provided with a barrier coating interposed between the constraint profile and tubing set surface. In one or more embodiments, the barrier coating comprises a PTFE or other fluoropolymer material.

In one or more embodiments, the constraint profiles have a substantially curvilinear cross-section. In one or more alternative embodiments, the constraint profiles have a substantially linear cross-section. In one or more alternative embodiments, the one or more contoured constraint profiles have a substantially constant cross-sectional design. In one or more alternative embodiments, the one or more contoured constraint profiles have a substantially variable cross-sectional design.

In one or more embodiments, the one or more contoured constraint profiles are configured to constrain one or more lumens situated within a tubing set equally. In one or more embodiments, the one or more contoured constraint profiles are configured to constrain one or more lumens situated within a tubing set unequally. In one or more embodiments, the one or more contoured constraint profiles are configured to constrain specific lumens situated within a tubing set, while also being configured to leave other specific lumens contained within a tubing set unconstrained. In one or more embodiments, the one or more contoured constraint profiles are configured to selectively engage with one or more medication lumens situated within a tubing set. In one or more embodiments, one or more constraint profiles are configured to selectively engage with one or more medication lumens situated within a tubing set. In one or more embodiments, one or more constraint profiles are configured to provide one or more different levels of constraint when disposed around a tubing set containing one or more medication lumens. In one or more embodiments, the one or more contoured constraint profiles are configured to constrain one or more fluid lumens in a desired manner, while also being configured to avoid avoiding constraint, crimping, or damage to an electrical or optical conductor disposed within the tubing set in a substantially parallel manner to the one or more medication lumens.

In one or more embodiments, the constraint is applied to the exterior of a tubing set that has already been sterilized. In one or more embodiments, the constraint is applied to a tubing set prior to sterilization of the assembled apparatus. In one or more embodiments, the material selection of constraint members and design of the locking fingers and apertures anticipates embrittlement or loss of strength after sterilization. In one or more embodiments, the material selection of constraint members and design of the locking fingers and apertures is designed to retain full design strength after radiation sterilization, for example by gamma or e-beam methods.

Elements of the tubing sets described herein may take various shapes and forms. In one or more embodiments, the outer surface of the tubing set may take a substantially circular or elliptical shape. The flexible portion of the tubing set may be fashioned from silicone, PVC, PVC without DEHP, EVA, HDPE, LDPE, TPU, PTFE, polyurethane, a fluoropolymer, or other suitable flexible material. In one or more embodiments, the flexible portion of the tubing set is manufactured from multiple connected segments of one or more flexible materials to provide a tubing set with different flexibility along the length. In one or more embodiments, the flexible portion of the tubing to which the constraint is applied may be manufactured from a more or less flexible material than the remainder of the tubing set.

Certain medications, such as biologic medications, displaying temperature-viscosity-concentration relationships and non-Newtonian behavior, such as shear thinning or shear thickening. In one or more embodiments, the constraint member profiles and length may be adjusted for each specific flow rate desired to lower the force needed for medication delivery, to provide sufficiently high flow rates, accommodate shear-thinning behavior, to accommodate shear-thickening behavior, to accommodate flow at a recommended administration temperature, to accommodate changes to concentration of a medication formulation at one or more administration doses, or to increase patient comfort.

In one or more embodiments, a kit of components is provided, comprising a plurality of tubing sets, each with assembled constraint apparatus applied as described herein, wherein the constraint member length and constraint profile of each applied constraint corresponds to one or more discrete desired flow rates for a specific medication at the anticipated administration temperature. In one or more embodiments, each constrained tubing set contained within the kit provides a different flow rate of a specific medication. In one or more embodiments, each constrained tubing set contained within the kit provides the same flow rate of a specific medication at one or more different administration temperatures. In one or more embodiments, each constrained tubing set contained within the kit provides the same flow rate of a specific medication at one or more different concentrations. In one or more embodiments, each constrained tubing set contained within the kit provides the same flow rate of a specific medication at one or more different concentrations at the same administration temperature. In one or more embodiments, each constrained tubing set contained within the kit provides one or more different flow rates of a specific medication at one or more different concentrations. In one or more embodiments, each constrained tubing set contained within the kit provides one or more different flow rates of a specific medication at one or more different concentrations at the same administration temperature. In some embodiments, the specific administration temperature is room temperature as governed by the ISO-1 standard. In some embodiments, the specific administration temperature is approximately 20°C.

In one or more embodiments, a kit of components is provided, comprising a plurality of tubing sets, each with assembled constraint apparatus applied as described herein, wherein the constraint member length and constraint profile of each applied constraint corresponds to one or more discrete desired flow rates for a non-Newtonian (e.g., biologic) medication at the anticipated administration temperature. In one or more embodiments, each constrained tubing set contained within the kit provides a different flow rate of a non-Newtonian (e.g., biologic) medication. In one or more embodiments, each constrained tubing set contained within the kit provides the same flow rate of a non-Newtonian (e.g., biologic) medication at one or more different administration temperatures. In one or more embodiments, each constrained tubing set contained within the kit provides the same flow rate of a non-Newtonian (e.g., biologic) medication at one or more different concentrations. In one or more embodiments, each constrained tubing set contained within the kit provides the same flow rate of a non-Newtonian (e.g., biologic) medication at one or more different concentrations at the same administration temperature. In one or more embodiments, each constrained tubing set contained within the kit provides one or more different flow rates of a non-Newtonian (e.g., biologic) medication at one or more different concentrations. In one or more embodiments, each constrained tubing set contained within the kit provides one or more different flow rates of a non-Newtonian (e.g., biologic) medication at one or more different concentrations at the same administration temperature. In some embodiments, the specific administration temperature is room temperature as governed by the ISO-1 standard. In some embodiments, the specific administration temperature is approximately 20°C.

In one or more embodiments, a kit of components is provided, comprising a plurality of tubing sets, each with assembled constraint apparatus applied as described herein, wherein the constraint member length and constraint profile of each applied constraint corresponds to one or more discrete desired flow rates for a medication studied in a human clinical trial, each of the discrete desired flow rates corresponding to one or more clinical trial test conditions for the medication being studied in the trial. In one or more embodiments, one or more of the kit, kit components, tubing sets, or tubing set constraints contains indicia related to the clinical trial, clinical trial identifier, clinical trial medication, clinical trial test condition, clinical trial randomization schedule identifier, or patient identifier.

In one or more embodiments, each constrained tubing set contained within the kit provides a different flow rate of a specific clinical trial medication under investigation. In one or more embodiments, each constrained tubing set contained within the kit provides the same flow rate of a specific clinical trial medication under investigation at one or more different administration temperatures. In one or more embodiments, each constrained tubing set contained within the kit provides the same flow rate of a specific clinical trial medication under investigation at one or more different concentrations. In one or more embodiments, each constrained tubing set contained within the kit provides the same flow rate of a specific clinical trial medication under investigation at one or more different concentrations at the same administration temperature. In one or more embodiments, each constrained tubing set contained within the kit provides one or more different flow rates of a specific clinical trial medication under investigation at one or more different concentrations. In one or more embodiments, each constrained tubing set contained within the kit provides one or more different flow rates of a specific clinical trial medication under investigation at one or more different concentrations at the same administration temperature. In some embodiments, the specific administration temperature is room temperature as governed by the ISO-1 standard. In some embodiments, the specific administration temperature is approximately 20°C.

In one or more embodiments, each clinical trial subject is assigned a kit of components comprising a plurality of tubing sets, each with assembled constraint apparatus applied as described herein, wherein the constraint member length and constraint profile of each applied constraint corresponds to one or more discrete desired flow rates for a medication studied in a human clinical trial, and wherein each of the discrete desired flow rates correspond to one or more clinical trial test conditions for the medication being studied in the trial, and wherein a clinical trial subject is administered medication through one of the sets within the kit in one or more instances. In one or more embodiments, each clinical trial participant is assigned a kit of components comprising a plurality of tubing sets, each with assembled constraint apparatus applied as described herein, wherein the clinical trial subject receives a different kit of components at each dosing interval during a clinical trial. In one or more embodiments, each clinical trial subject is assigned a kit of components comprising a plurality of tubing sets, each with assembled constraint apparatus applied as described herein, and wherein a clinical trial subject is administered medication with only one of the tubing sets within the kit. In one or more embodiments, each clinical trial subject is assigned a kit of components comprising a plurality of tubing sets, each with assembled constraint apparatus applied as described herein, and wherein a clinical trial subject is administered medication with one or more of the tubing sets within the kit. In one or more embodiments, each clinical trial subject is assigned a kit of components comprising a plurality of tubing sets, each with assembled constraint apparatus applied as described herein, and wherein a clinical trial subject is administered medication using one or more of the tubing sets within the kit based on their physiologic response to a medication studied in a human clinical trial. In one or more embodiments, one or more of the kit, kit components, tubing sets, or tubing set constraints contains indicia related to the clinical trial, clinical trial identifier, clinical trial medication, clinical trial test condition, clinical trial randomization schedule identifier, or patient identifier.

In one or more embodiments, the kit of components used in a clinical trial is provided in a commercial presentation of a medication studied in a human clinical trial once approved for general use by a regulatory approval body. In one or more embodiments, a subset of the kit of components used in a clinical trial is provided in a commercial presentation of a medication studied in a human clinical trial once approved for general use by a regulatory approval body. In one or more embodiments, multiple different kits, each containing a subset of the kit of components used in a clinical trial is provided in a commercial presentation of a medication studied in a human clinical trial once approved for general use by a regulatory approval body. In one or more embodiments, one or more of the kit, kit components, tubing sets, or tubing set constraints provided in a commercial presentation of an approved medication contains different indicia than that used in one or more clinical trials of the medication.

In addition to the aspects described herein related to constraint of one or more medications flowing through a tubing set, constraint members may also serve to identify the tubing set, providing a more intuitive experience for the user and avoiding potential medication errors caused by incorrect set selection. In an alternative embodiment illustrated in Figure 14A, the assembled first and second constraint members comprise a series of different shapes or profiles, by way of example a substantially rectilinear shape 1401, a substantially elliptical shape 1402, a substantially circular shape 1403, or a substantially polygonal shape 1404, each corresponding to different medications or different doses of the same medication. Although Figure 14A and the accompanying specification shows a variety of geometric profiles by way of example, it will be clear to those skilled in the art that many geometric profiles are possible, and the foregoing example shall not be construed as limiting the apparatus to one of the geometric shapes or profiles described or illustrated herein.

The constraint members may be provided in a variety of shapes and colors. In some embodiments, constraint members are molded in one or more colors associated with a pharmaceutical brand. In some embodiments, the constraint members are molded in one or more colors associated with a dose of a specific medication. In some embodiments, the first constraint member is molded in a different color than the second constraint member.

As illustrated in Figure 17B, one or more assembled constraint members 405 may be provided with indicia related to the medication and flow rate. In another embodiment, one or more assembled constraint members 1705 may be provided with indicia 1706 related to the medication and flow rate. In another embodiment, one or more assembled constraint members 1705 may be provided with indicia 1706, 1707, 1708, 1709 related to the medication and an ordinal identifier identifying the flow rate in simple terms for a user lacking healthcare training, such as a patient. Indicia 1706, 1707, 1708, and 1709 may be applied to the constrain members by molding, co-molding of a polymeric material (optionally in a contrasting color), in-mold decoration, by laser marking, pad printing, or other means. In some embodiments, the indicia includes the specific temperature used to calibrate the flowrate noted in the indicia. In some embodiments, the indicia includes one or more warnings, cautions, or instructions to a user of the tubing set. In one or more embodiments, one or more of the kit, kit components, tubing sets, or tubing set constraints contains indicia related to the clinical trial, clinical trial identifier, clinical trial medication, clinical trial test condition, clinical trial randomization schedule identifier, or patient identifier. In one or more embodiments, one or more of the kit, kit components, tubing sets, or tubing set constraints contains indicia that disguise or "blind" a clinical participant to one or more of the medication (or a placebo medication in a randomized clinical trial), clinical trial test condition, clinical trial randomization schedule, or clinical trial sponsor.

The constraints described herein may also be used to identify the constraint elements during manufacturing or assembly, avoiding mix-ups and improper component selection. Referring to Figure 17C, an assembled constraint 1720 may be provided with one or more machine-readable indicia 1721, 1722, 1723, 1724, and 1725. In one or more embodiments, one or more of the machine-readable indicia 1721, 1722, 1723, and 1725 comprise a bar code, near-field communication (NFC) or a radiofrequency identification (RFID) tag. In one or more embodiments, information on one or more of the machine-readable indicia 1721, 1722, 1723, 1724, and 1725 is populated during manufacturing of the apparatus. In one or more embodiments, one or more of the machine-readable indicia 1721, 1722, 1723, 1724, and 1725 are configured to be written one time with information during manufacturing and read-only thereafter. In one or more embodiments, one or more of the machine-readable indicia 1721, 1722, 1723, 1724, and 1725 are configured to be written after manufacturing.

In one or more embodiments, information on one or more of the machine-readable indicia 1721, 1722, 1723, 1724, and 1725 is populated during dispensing of the apparatus. In one or more embodiments, information on one or more of the machine-readable indicia 1721, 1722, 1723, 1724, and 1725 is populated with patient information from an electronic health record. In one or more embodiments, information on one or more of the machine-readable indicia 1721, 1722, 1723, 1724, and 1725 is populated with medication administration or monitoring instructions from an electronic health record. In one or more embodiments, information on one or more of the machine-readable indicia 1721, 1722, 1723, 1724, and 1725 is populated with either or both of physiologic or laboratory values which constitute safe medication administration parameters for the specific tubing set and assembled constraint 1720.

In one or more embodiments, one or more of the machine-readable indicia 1721, 1722, 1723, 1724, and 1725 are located on the exterior surface of the assembled constraint and/or the constraint members. In one or more embodiments, one or more of the machine-readable indicia 1721, 1722, 1723, 1724, and 1725 are located below the exterior surface of the assembled constraint. In one or more embodiments, one or more of the machine-readable indicia 1721, 1722, 1723, 1724, and 1725 are inserted into the constraint members during injection molding. In one or more embodiments, one or more of the machine-readable indicia 1721, 1722, 1723, 1724, and 1725 are applied to one or more of the constraint members or assembled constraint after molding.

In one or more embodiments, a first electronic indicia 1722 is provided on the first constraint member, and a second electronic indicia 1723 is provided on a second constraint member, both constraint members comprising an assembled constraint 1720, configured so that during manufacturing operations, the first 1722 and second 1723 electronic indicia may be verified by inspection equipment to verify the proper components are selected, the apparatus components are assembled in the correct orientation, and the apparatus matches the expected medication and flow rate. In one or more embodiments, electronic indicia 1725 is provided on the assembled constraint 1725 as a verification during manufacturing and packaging that the apparatus matches the expected medication and flow rate.

In one or more embodiments, machine readable indicia 1721, 1724 is provided on one or more exterior surfaces of the assembled constraint 1720 as a verification during manufacturing and packaging that the apparatus matches the expected medication and flow rate. In one or more embodiments, the machine-readable indicia 1721, 1724 comprises a one or more of a QR code, data matrix, 2D bar code, or linear bar code. In one or more embodiments, human-readable indicia 1727 is provided on one or more exterior surfaces of the assembled constraint 1720 as verification that the apparatus matches the expected medication and flow rate. In one or more embodiments, the information contained in the machine-readable indicia 1721, 1724 is the same as that information contained in the human-readable indicia 1727. In one or more embodiments, the information contained in the human-readable indicia 1727 is a subset of the information contained in the machine-readable indicia 1721, 1724. In one or more embodiments, the information contained in the machine-readable indicia 1721, 1724 is different from that information contained in the human-readable indicia 1727.

In one or more embodiments, one or more of the indicia 1721, 1722, 1723, 1724, 1725, or 1727 encodes one or more of tubing set outer diameter, tubing set material, tubing set material lot code, constraint material lot code, internal batch control numbers, number of fluid lumens disposed within the tubing set, tubing set medication lumen diameters, tubing set medication lumen arrangement within the tubing set cross section, number of electrical conductors disposed within the tubing set, number of optical conductors disposed within the tubing set, the medication name, the medication dose, the medication concentration, the medication lot number, the medication expiration date, the numeric medication flow rate (e.g., in mL/h) corresponding to the constraint, the ordinal identifier of the flow rate corresponding to the constraint (e.g., "slow set," "fast set," or "Set A") the medication administration temperature corresponding to the flow rate, the presence or absence of a clamping device in the apparatus, tubing set apparatus lot code, tubing set apparatus serial number or unique device identifier, tubing set apparatus Global Trade Item Number (GTIN), or tubing set apparatus expiration date.

The constraints described herein may also be used to identify the constraint elements during dispensing to a patient or clinical use, avoiding mix-ups and improper component selection. In one or more embodiments, electronic indicia 1725 are provided on the assembled constraint for scanning or sensing by a drug delivery system, patient, clinician, or other user of the device to ensure proper tubing sets are used. In one or more embodiments, one or more of the kit, kit components, tubing sets, or tubing set constraints contains indicia that disguise or "blind" a clinical participant to one or more of the medication (or a placebo medication in a randomized clinical trial), clinical trial test condition, clinical trial randomization schedule, or clinical trial sponsor.

Reference throughout this specification to "one embodiment," "certain embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in one embodiment" or "in an embodiment" in various places throughout this specification are not necessarily referring to the same embodiment of the disclosure. Furthermore, the particular features, structures, materials, or characteristics may be combined in any suitable manner in one or more embodiments.

Although the disclosure herein has been described with reference to particular embodiments, those skilled in the art will understand that the embodiments described are merely illustrative of the principles and applications of the present disclosure. It will be apparent to those skilled in the art that various modifications and variations can be made to the method and apparatus of the present disclosure without departing from the scope of the disclosure. Thus, the present disclosure can include modifications and variations that are within the scope of the appended claims

In one or more embodiments, the constraint apparatus, systems, methods and kits described immediately above with respect to Figures 14-17 are configured to be used or combined with the apparatus, systems and methods described with respect to Figures 1-10 and the numbered embodiments described with respect to Figures 1-10 . Thus, in some embodiments, the apparatus, systems and methods described and claimed with respect to Figures 1-10 further comprise the constraint apparatus, systems, methods and kits described immediately above with respect to Figures 14-17 in combination or addition with the various embodiments described with respect to Figures 1 to 10, including the numbered embodiments.

Optionally, the drive system used in a medicament delivery device as described herein is a pressure-based drive. In certain pump configurations, it may be desirable to provide a pneumatic drive for one or more fluidic reservoirs. Alternatively, where a pneumatic drive is described herein, another pressure-based drive such as a hydraulic drive may be used instead. Figure 21 shows two configurations of a system to which the present disclosure is directed, allowing for centralized (bottom) or decentralized/distributed (top) distribution of components on a patient receiving medication with the medicament delivery device. The medicament delivery device comprises a (typically reusable) device 2101 (powerpack portion), for example a housing containing a powerpack (including a pump, for example a pneumatic pump), and a (typically single use (optionally refurbishable)) cartridge unit 2102 (cassette portion). Either the device and cartridge are centralized (bottom), for example by being directly attached to one another, or decentralized/distributed (top), for example spaced apart and only attached together by an umbilical comprising, for example, a tubing set 2103, which comprises a tube with at least one lumen, and optionally comprises one or more conductors (e.g. one or more cables). In the bottom example in particular, the cartridge unit may be wearable, and may be supported separately. For completeness, the tubing 2104 between the cartridge unit 2102 and the patient 2105 is also shown - this could be multiple tubes as shown, or a single tube. A medicament delivery member such as a cannula, a needle or a jet injector can be provided at the end of the tubing 2104 distal to the cartridge unit 2102.

Figure 22 depicts a device 2201; the device 2201 could be used for the device shown as device 2101 in Figure 21. The device comprises separate valving for each medication (2202 A-C) and optionally also for an emergency medication (2202 E). The device 2201 may also comprise a power source 2210, one or more pumps 2211 and a user interface 2212. The power source could be a battery, a spring, and/or a gas canister, for example. One pump 2211 could be provided, or one pump could be provided for valves A to C and a second pump could be provided for valve E, for example. Optionally, one pump is provided for each valve. The user interface 2212 could include one or more buttons and/or a screen, for example. Each valve could be associated with a separate medication - in the example in Figure 22, there could therefore be three medications (A-C) and an emergency medication (E).

Figure 23 shows a similar device to that in Figure 22, but with a single valve 2202A/B/C for multiple medications instead. This may also allow a sequential pneumatic delivery as described previously for the expected medication administration (A/B/C), and a separate air line for contingent administration of an optional emergency medication (E). Further details of administration options are outlined in more detail elsewhere in the application and will not be repeated here. In particular, one or more of the lumens in the tubing sets described elsewhere in the application could contain a fluid such as a gas (e.g. air or nitrogen or argon) or a liquid (e.g. water), rather than containing medication, with the fluid being pressurized by a pump and thereby squeezing a medication out of a container and thereby delivering the medication to a patient. Such fluids may be run through one more lumens in a multiple lumen tubing set as desired, for example based on the clinical applications as described below in representative scenarios.

Provision of pneumatic elements (i.e. a lumen or lumens providing drive elements), medicament delivery elements (i.e. a lumen or lumens providing medicament delivery elements), and/or communication elements (for example to connect monitoring systems within different parts of a system electrically and/or optically) within separate lumens of the same tube can be beneficial as it can simplify device assembly and/or device use by reducing the number of separate tubes that are required.

In general, a tubing set for a medicament delivery device may be provided. The tubing set comprises one or more lumens. Optionally, the tubing set comprises multiple lumens; this can be beneficial for ease of assembly and for ease of use. Optionally, one of the lumens is a medicament lumen; that is, a lumen for transmission of medicament. Optionally, one of the lumens is a pneumatic fluid lumen; in such a case, a fluid (such as a gas, e.g. air, nitrogen or argon, or a liquid such as water) would typically be pressurized by a drive, for example to drive a medicament out of a medicament container by compression of the medicament container by transfer of the pneumatic fluid into a container at least partially surrounding the medicament container. Optionally, the tubing set comprises a conductor; this could be used to transfer data from one part of the medicament delivery device to another, or to transfer commands from one part of the medicament delivery device to another. The conductor could be an electrical conductor or an optical conductor. The conductor could be in one of the lumens. Alternatively, the conductor could be embedded in the tubing set, or could be attached to an outer wall of the tubing set. Optionally, the conductor is in an undercut of the tubing set (see for example Figure 26). This can help reduce or avoid the risk of the conductor detaching from the tubing set when the tubing set is flexed during use. This can also help provide structural support to the tubing set, particularly in the case of an electrical conductor. The tubing set has a longitudinal axis extending along the length of the tubing set (optionally in the direction of the multiple lumens, as in Figure 12G for example), with the undercut arranged on a long cross-sectional axis, the long cross-sectional axis being the longest axis of the tubing set perpendicular to the longitudinal axis. For reference, the longitudinal axis would be in the direction of the arrows of Figure 18D, for example, or would be into the page in Figures 12 and 24 to 26. A short cross-sectional axis is perpendicular to the longitudinal axis and perpendicular to the long cross-sectional axis. The long cross-sectional axis is the first axis 1240 in Figure 12G, for example, and the short cross-sectional axis is the second axis 1241 in Figure 12G, for example. Optionally, some or all of the lumens are arranged along the long cross-sectional axis, as in Figures 12G and 25A, for example. Optionally, one of the multiple lumens is arranged along a short cross-sectional axis, as in Figures 12, 24, 25 and 26, for example. Optionally, the tubing set is oval when viewed in cross-section perpendicular to the longitudinal axis, as in Figure 24, for example, although other cross-sectional shapes, such as round, square, hexagonal or other regular or irregular shapes, could alternatively be used. A medicament delivery device could comprise one or more tubing sets as described in this paragraph or as described elsewhere in this application.

In one example, medicament delivery device is configured to deliver a therapeutic medication to a patient, and comprises a tubing set as described herein (for example any tubing set according to Figure 12, 24, 25 or 26) along with a reservoir containing a therapeutic medication. The apparatus can comprise a medicament delivery member, such as a cannula, a needle or a jet injector. The apparatus can comprise at least one sensor configured to detect at least one of a physiological aspect of the patient and a physical aspect of the apparatus. The apparatus can comprise a controller configured to receive data from the sensor, and to start and stop delivery of the therapeutic medication to the patient in response to data received from the sensor. Alternatively or additionally, the apparatus can comprise a controller configured to control the injection rate of one or more medicaments. The apparatus can comprise a drive unit configured to deliver the contents of the reservoir (typically a medicament) into the patient. The drive unit can be a pressure-based drive such as a pneumatic drive unit, for example using a pressurized fluid such as pressurized gas from a gas canister or using a pump to pressurize a fluid. Other drive units, such as mechanical drives, for example using springs, or even manually driven drives could alternatively be used. Optionally, the medicament delivery device is for delivery of an oncology medicament. Optionally, the medicament delivery device is for delivery of two or more medicaments. Optionally, the medicament delivery device is for delivery of one or more medicaments and for contingent delivery of an emergency medicament, wherein the device is configured to deliver the emergency medicament to the patient if one or more pre-determined conditions are met.

A medicament delivery device can have two or more tubing sets. In one example, a first tubing set (for example tubing set 2103) connects a drive unit (such as device 2101) to a cartridge unit (such as cartridge 2102), and a second tubing set (for example tubing set 2104) connects a cartridge unit (such as cartridge 2102) to a medicament delivery site. In more detail, a configuration such as the top configuration from Figure 21 can be used, with a pneumatic drive in the device 2101, one or more lumens in one or more tubing sets 2103 (these lumens being used for pneumatic fluid - so for pneumatic communication), one or more medicament containers in the cartridge 2102, one or more lumens in one or more tubing sets 2104 (these lumens being used for medicament - so for fluidic communication). Optionally, electrical or optical communication could also be provided in one or both of the first and second tubing sets, for example to transmit sensor data and/or to transmit commands. As mentioned previously, a medicament delivery member such as a cannula, a needle or a jet injector can be provided at the end of the tubing 2104 distal to the cartridge unit 2102 (optionally one medicament delivery member used for a plurality of lumens, or one medicament delivery member per lumen, or a combination of both).

Further examples of pneumatic, fluidic, electrical and optical communication are described below. These approaches could be used in the top configuration from Figure 21, for example.

### Pneumatic Communication

The tubing sets described elsewhere in this application may be used for pneumatic and/or fluidic elements of the drug delivery system. Figure 24A shows a tubing set 2400 with a cross section 2401 and multiple lumens 2402, 2403, 2404. Lumens 2402, 2303, 2404 may be configured to communicate with components of the system as described elsewhere herein and/or with components of the systems as described in 63/392,539. For instance, lumen 2403 may be configured to be in pneumatic communication with a controller and drive (e.g. air pump) and a pressurized portion of a fluid reservoir (cassette) as shown in Figure 21, while lumens 2403-2404 may be in fluidic communication with a reservoir (a fluidic reservoir, for example a fluidic reservoir containing a medicament) as described elsewhere herein.

### Pneumatic & Fluidic Communication

Figure 24B shows a tubing set 2410 with a cross section 2413 and multiple lumens 2411, 2412 configured to communicate with components of the drug delivery system as described elsewhere herein and/or with components of the systems as described in 63/392,539,the For instance, lumen 2411 may be configured to be in pneumatic communication with a controller and drive (e.g. air pump) and a pressurized portion of a fluid reservoir (cassette) as shown in Figure 23 for delivery of medications in a regimen (denoted "A/B/C") while lumen 2411 may be used to pressurize a fluid reservoir (cassette) as shown in Figure 23 for contingent delivery of medication (e.g. emergency medications). Put another way, lumen 2411 may be used for medications delivered in the normal course of therapy, while lumen 2412 may be used for contingent administration of an emergency medication.

### Pneumatic, Electrical, and/or Optical Communication

Figure 25A shows a tubing set 2500 with a cross section 2504, lumen 2501, and conductors 2502, 2503. Lumen 2501 and conductors 2502, 2503 may be configured to communicate with components of the drug delivery system as described elsewhere herein and/or with components of the systems as described in 63/392,539,the For example, lumen 2501 may be configured to be in pneumatic communication with a controller and drive (i.e., air pump) and a pressurized portion of a fluid reservoir (cassette) as shown in Figure 1, while conductors 2502, 2503 may be in electrical and/or optical communication (or combinations thereof) with the controller as described elsewhere herein.

Figure 25B shows a tubing set 2510 with a cross section 2513, lumen 2511, and conductor 2512. Lumen 2511 and conductors 2512 may be configured to communicate with components of the system as described elsewhere herein and/or with components of the systems as described in 63/392,539,the For instance, lumen 2511 may be configured to be in pneumatic communication with a controller and drive (i.e., air pump) and a pressurized portion of a fluid reservoir (cassette) as shown in Figure 21, while conductor 2512 may be in electrical communication with a controller or sensors as described elsewhere herein.

Figure 26 shows a tubing set 2600 with a cross section 2607, lumen 2604, undercut 2602, and conductors 2605, 2606, all as described elsewhere herein. Lumen 2604 and conductors 2605, 2606 may be configured to communicate with components of the drug delivery system as described elsewhere herein and/or with components of the systems as described in 63/392,539,the For example lumen 2604 may be configured to be in pneumatic communication with a controller and drive (i.e., air pump) and a pressurized portion of a fluid reservoir (cassette) as shown in Figure 21, while conductors 2605, 2606 may be in electrical and/or optical communication (or combinations thereof) with the controller as described elsewhere herein.

The drug delivery devices and components described herein can be used for the treatment and/or prophylaxis of one or more of many different types of disorders. Exemplary disorders include, but are not limited to: rheumatoid arthritis, inflammatory bowel diseases (e.g. Crohn's disease and ulcerative colitis), hypercholesterolaemia, diabetes (e.g. type 2 diabetes), psoriasis, migraines, multiple sclerosis, anaemia, lupus, atopic dermatitis, asthma, nasal polyps, acute hypoglycaemia, obesity, anaphylaxis, cancer and allergies. Exemplary types of drugs that could be included in the medicament delivery devices described herein include, but are not limited to, antibodies, proteins, fusion proteins, peptibodies, polypeptides, pegylated proteins, protein fragments, protein analogues, protein variants, protein precursors, and/or protein derivatives. Exemplary drugs that could be included in the drug delivery devices described herein include, but are not limited to (with non-limiting examples of relevant disorders in brackets): etanercept (rheumatoid arthritis, inflammatory bowel diseases (e.g. Crohn's disease and ulcerative colitis)), evolocumab (hypercholesterolaemia), exenatide (type 2 diabetes), secukinumab (psoriasis), erenumab (migraines), alirocumab (rheumatoid arthritis), methotrexate (amethopterin) (rheumatoid arthritis), tocilizumab (rheumatoid arthritis), interferon beta-1a (multiple sclerosis), sumatriptan (migraines), adalimumab (rheumatoid arthritis), darbepoetin alfa (anaemia), belimumab (lupus), peginterferon beta-1a' (multiple sclerosis), sarilumab (rheumatoid arthritis), semaglutide (type 2 diabetes, obesity), dupilumab (atopic dermatis, asthma, nasal polyps, allergies), glucagon (acute hypoglycaemia), epinephrine (anaphylaxis), insulin (diabetes), atropine and vedolizumab (inflammatory bowel diseases (e.g. Crohn's disease and ulcerative colitis)). Pharmaceutical formulations including, but not limited to, any drug described herein are also contemplated for use in the drug delivery devices described herein, for example pharmaceutical formulations comprising a drug as listed herein (or a pharmaceutically acceptable salt of the drug) and a pharmaceutically acceptable carrier. Pharmaceutical formulations comprising a drug as listed herein (or a pharmaceutically acceptable salt of the drug) may include one or more other active ingredients, or may be the only active ingredient present.

In general in this application, unless indicated otherwise, a 'tubing set' or 'tubing' may comprise one or more tubes ,each tube comprising one or more lumens.

### Reference numerals

- 101: Patient
- 102: Superior Vena Cava
- 103: Catheter
- 104: Patient Interface [Luer Connector(s)]
- 105: Patient
- 106: Superior Vena Cava
- 107: Catheter
- 108: Patient Interface [Luer Connector(s)]
- 109: Superior Vena Cava
- 113: Patient Arm
- 114: Peripheral vein
- 115: Catheter
- 116: Patient Interface [Luer Connector(s)]
- 120: Catheter
- 121: Patient
- 122: Tubing set
- 123: Patient Skin
- 124: Port access [Huber] needle
- 125: Patient Interface (Implanted Port Below Patient Skin)
- 126A: Port Septum
- 126B: Patient interface (port septum of implanted port housing below 127 patient skin 123)
- 127: Implanted Port Housing
- 128: Catheter
- 129: Needle Entry Point (Center of Port Septum)
- 130: Hand of Patient, Caregiver, or Healthcare Provider
- 140: SC Needle Assembly
- 141: Patient Skin
- 142: SC Needle Cannula
- 143: SC Needle Point (Hollow)
- 144: Needle Insertion Grip Affordance
- 145: Tubing Set
- 146: Patient Epidermis
- 147: Patient Dermis
- 148: Patient Subcutaneous Tissue
- 149: Patient Muscle Tissue
- 150: Hand of Patient, Caregiver, or Healthcare Provider
- 151: IM Needle Assembly
- 152: Patient Skin
- 153: Needle Insertion Grip Affordance
- 154: Tubing Set
- 155: IM Needle Cannula
- 156: IM Needle Point (Hollow)
- 157: Hand of Patient, Caregiver, or Healthcare Provider
- 158: SC Needle Assembly
- 159: Patient Skin
- 160: Angled SC Needle Cannula
- 170: SC Needle Point (Hollow)
- 171: Needle Insertion Grip Affordance
- 172: Tubing Set
- 173: Patient Epidermis
- 174: Patient Dermis
- 175: Patient Subcutaneous Tissue
- 176: Patient Muscle Tissue
- 180: Hand of Patient, Caregiver, or Healthcare Provider
- 181: Two-Part SC Needle Assembly (Soft Cannula and Rigid Inserter Needle)
- 182: Patient Skin
- 183: Soft, Flexible SC Administration Cannula
- 184: Rigid Cannula Inserter Needle
- 185: Soft, Flexible SC Administration Cannula Tip (Hollow)
- 186: Needle Insertion Grip Affordance and Inserter Needle Removal Means
- 187: Tubing Set
- 188: Hand of Patient, Caregiver, or Healthcare Provider
- 189: Removed First Part of SC Needle Assembly
- 190: Removed Rigid Cannula Inserter Needle
- 191: Retained Second Part of SC Needle Assembly
- 192: Retained Soft, Flexible SC Administration Cannula
- 193: Patient Subcutaneous Tissue
- 194: Soft, Flexible SC Administration Cannula Tip (Hollow)
- 195: Patient Muscle Tissue
- 197: Tubing Set
- 208: Medication Reservoir
- 209: Medication Reservoir
- 210: Medication Reservoir
- 211: Fluidic Connection
- 212: Fluidic Connection
- 213: Fluidic Connection
- 215: Tubing
- 216: Patient Interface
- 217: Patient
- 218: Fluid Pump
- 219: Outer Housing
- 220: Therapeutic Medication
- 221: Therapeutic Medication
- 222: Therapeutic Medication
- 224: Medication Reservoir
- 225: Medication Reservoir
- 226: Medication Reservoir
- 227: Pre-Medication(s)
- 228: Therapeutic Medication
- 229: Post-Medication(s)
- 230: Fluidic Connection
- 231: Fluidic Connection
- 232: Fluidic Connection
- 233: Fluid Pump
- 234: Tubing
- 235: Patient Interface
- 236: Patient
- 240: Outer Housing
- 241: Medication Reservoir
- 242: Medication Reservoir
- 243: Medication Reservoir
- 244: Medication Reservoir
- 245: Pre-Administration Flush Solution
- 246: Therapeutic Medication
- 247: First Post-Administration Flush Solution
- 248: Fluidic Connection
- 249: Fluidic Connection
- 250: Fluidic Connection
- 251: Fluidic Connection
- 252: Fluid Pump
- 253: Tubing
- 254: Patient Interface
- 255: Patient
- 256: Second Post-Administration Flush Solution
- 271: Time Delay
- 272: Time Delay
- 273: Time Delay
- 274: Time Delay
- 275: Time Delay
- 276: Time Delay
- 277: Sequential administration
- 278: Concurrent administration
- 279: Sequential administration with time-delay start
- 280: Sequential administration with time delays between one or more medications
- 281: Concurrent and sequential administration with time delays between one or more medications
- 282: Beginning of administration
- 283: End of administration
- 285: Outer Housing
- 286: Medication Reservoir
- 287: Therapeutic Medication of Interest
- 288: Medication Reservoir
- 289: Emergency Medication
- 290: Fluidic Connection
- 291: Fluidic Connection
- 292: Fluid Pump
- 293: Single Lumen Tubing
- 293': Double Lumen Tubing
- 294: Patient Interface
- 295: Patient
- 297: First Medication Lumen
- 298: Second Medication Lumen
- 318: No; additional Phase 2 study required
- 319: Desired Efficacy Signal Achieved with One or More Dosing Regimens?
- 320: Formulation Development (Physical Form Concentration, Volume, Viscosity, Stability, Excipients)
- 321: Pharmacokinetic Modeling (In Vitro and/or Animal Studies)
- 322: Determination of tubing sets for Phase 1 clinical trial design
- 323: Hypothesized Dose Range
- 324: Initial Human Use Studies with Varying Doses (Phase 1 Clinical Study)
- 325: Appropriate tubing set(s) used during trial(s) to correspond to desired clinical trial condition(s)
- 326: Clinical Pharmacokinetic Evaluation and Dose Range Determination
- 327: Dosing Regimen Refinement for Phase 2 Clinical Study
- 328: Single Dosing Regimen (flat dose)
- 329: Determination of tubing set for Phase 2 clinical trial design
- 330: Early Efficacy Human Use Studies with Single Dose (Phase 2)
- 331: Single tubing set configuration provided to correspond to desired clinical trial condition
- 332: Multiple Possible Dosing Regimens (dose banding, weight based dosing)
- 333: Determination of tubing set(s) for Phase 2 clinical trial design
- 334: Early Efficiency Human Use Studies with Multiple Doses (Phase 2)
- 335: Either a single or a plurality of tubing sets provided to correspond to desired clinical trial condition
- 336: Dose 1
- 337: Dose 2
- 338: Dose n
- 339: Dose 1
- 340: Yes, positive signal; medication appears effective at one or more doses
- 341: Determination of tubing set(s) for Phase 3 clinical trial design
- 342: Pivotal Human Use Studies With Desired Dosing Regimen(s) (Phase 3)
- 344: Approval and Commercial Use In Various Use Settings Outside of Clinical Trials (at home; in-clinic)
- 360: Formulation Characteristics (concentration, volume, viscosity, stability, excipients)
- 361: Pharmacokinetic Modeling
- 362: Desired Clinical Trial Conditions
- 363: Nominal calculation using Hagen-Pouiselle or other model
- 364: Initial design and component selection
- 365: Mechanical testing of flow rate with initial design and formulated drug product
- 366: Comparison to desired delivery profile
- 367: Refined design and component selection
- 368: Manufacture, Mechanical verification testing and analysis with drug formulation
- 369: Final set used for clinical trial(s)
- 380: P = drug pressure (drive force required). L = tubing length. u = viscosity. D = tubing internal diameter, nominal (or toleranced). T = delivery time. V = volume.
- 391: tubing length
- 392: tubing internal diameter tolerance
- 393: tubing nominal internal diameter
- 401: drug delivery system
- 402: Therapeutic Medication
- 403: Controller
- 403a: Processor
- 403b: Memory
- 403c: Input/Output Devices
- 403d: Infusion Reaction Detection/Response Algorithm
- 404: Patient
- 405: Tubing
- 406: Patient Interface
- 407: Sensor(s)
- 408: Patient Data
- 410: Sensor Data
- 411: Fluidic Connection
- 412: Fluid Flow Control
- 413: Fluid Flow Control
- 414: Fluid Flow Control
- 415: Fluid Pump
- 416: Emergency Medication
- 417: Fluidic Connection
- 501: Medication Administration
- 502: Physiologic Sensor Data
- 503: In-Person or Remote Observation of Patient Condition
- 504: Sensor Sampling
- 505: Sensor Data Pre-Processing
- 506: Sensor Data
- 507: Observations of Patient
- 508: Patient Interaction, Patient Interview
- 509: HCP Evaluation, Clarification
- 510: Heuristic and Clinical Judgment
- 511: Data Consolidation
- 512: Suspected infusion reaction?
- 513: No
- 514: Continue Administration at prior rate
- 515: Yes
- 516: Pause or Stop Administration
- 517: Notify HCP with Appropriate Data (escalation)
- 518: HCP Evaluation of Patient & Data
- 519: Safe to start the Infusion?
- 520: Yes (false alarm, no issue)
- 521: Yes, proceed with caution
- 522: Continue Administration at reduced rate
- 523: No
- 524: Administer one or more emergency medications
- 525: Summon emergency medical services
- 526: Summon emergency medical services and direct to specific address via geolocation services
- 549: Patient Self-Report of Condition
- 550: Alternative Flow for Different types of infusion reaction
- 601: Filter Membrane
- 602: Undesired Particulate
- 603: Inflow medication with potential particulate
- 604: Outflow medication with particulate removed
- 605: Inflow medication
- 606: Smoothed inlet to prevent turbulent flow and protein shearing
- 607: Engineered flow restrictor
- 608: Outflow at reduced rate
- 609: Tubing outer diameter (OD)
- 610: Tubing inner diameter (ID)
- 640: Tubing
- 640': Tubing
- 641: Medication Lumen
- 642: Tubing
- 642': Tubing Profile
- 643: Lumen
- 644: Lumen
- 645: Lumen
- 646: Tubing
- 646': Tubing Profile
- 647: Barrier Coating
- 648: Lumen
- 775: Outer housing
- 776: Investigational Therapeutic Medication
- 776': Medication reservoir
- 777: Fluidic Connection
- 778: Fluid Pump
- 779: Controller
- 780: Tubing
- 781: Communication Means
- 782: Sensor(s)
- 783: Patient
- 784: Clinical Study Team
- 785: Permanent Data Storage
- 786: Data Link to Permanent Storage
- 787: Data Retrieval and Analysis
- 788: Patient Interface
- 794: Processor
- 801: Outer housing
- 803: Controller
- 804: Processor
- 805: Memory
- 806: Input/Output Devices
- 807: Therapeutic Medication
- 807': Reservoir
- 808: Therapeutic Medication
- 808': Reservoir
- 809: Fluidic Connection
- 810: Fluidic Connection
- 811: Fluid Pump
- 812: Tubing Set
- 813: Patient Interface
- 814: Patient
- 815: Coupled Sensor(s)
- 1000: Electronic Health Record System (EHR)
- 1001: (Representative) Medication Order
- 1002: Therapeutic Medication of Interest
- 1003: Patient name
- 1004: Patient identifier
- 1005: Medication order identifier
- 1006: Dispensing and verification instructions for Therapeutic Medication of Interest
- 1007: Administration parameters for Therapeutic Medication of Interest
- 1008: Administration time and date for Therapeutic Medication of Interest
- 1009: Medication Prescriber
- 1010: Healthcare Provider
- 1011: Healthcare Provider Verification of Reservoir, Medication against Order
- 1012: Healthcare Provider Verification of Drug Delivery Device against order via barcode scanning
- 1013: Laboratory Values
- 1020: Drug Delivery Device (Present Invention)
- 1021: EMR Interface
- 1022: Barcode
- 1023: Processor
- 1024: Memory
- 1025: Input/Output Devices
- 1026: Controller
- 1027: Therapeutic Medication
- 1027': Medication Reservoir
- 1028: Fluidic Connection
- 1029: Fluid Pump
- 1029a: Tubing
- 1029b: Patient Interface
- 1029c: Patient
- 1030: (Representative) Order set associated with Therapeutic Medication of Interest
- 1031: Medications given prior to administration of therapeutic medication of interest
- 1032: Therapeutic Medication of Interest
- 1033: Medicines given post-administration of therapeutic medication of interest, or in emergency
- 1034: Required labs and patient monitoring instructions
- 1035: Conditional instructions for nursing
- 1036: Administration contraindications
- 1037: Patient assessment instructions
- 1038: Healthcare Provider Observation of Administration Progress
- 1120: Outer Housing
- 1121: Controller
- 1121a: Processor
- 1121b: Memory
- 1121c: Input/Output Devices
- 1122: Therapeutic Medication
- 1122': Reservoir
- 1123: Fluidic Connection
- 1124: Fluid Pump
- 1125: Tubing
- 1125f: Tubing Fluid Lumen/Medication Lumen
- 1125o: Optical Conductor
- 1126: Patient Interface
- 1127: Sensors
- 1128: Patient
- 1129: Conductor
- 1134: Indicator
- 1200: Tubing
- 1201: Medication Lumen
- 1202: Internal Conductors
- 1203: Tubing
- 1204: Medication Lumen
- 1205: External Conductor
- 1206: Tubing
- 1207: Medication Lumen
- 1208: Optical Conductor
- 1209: Tubing
- 1210: Medication Lumen
- 1211: Optical Conductor
- 1212: Conductor
- 1220: Tubing
- 1221: Medication Lumen
- 1222: External Conductor
- 1223: Barrier Coating
- 1225: Tubing
- 1226: External Conductor
- 1227: Medication Lumen
- 1228: Barrier Coating
- 1230: Tubing
- 1231: Medication Lumen
- 1232: Undercut
- 1233: External Conductor
- 1234: Internal Conductors
- 1235: Protective Sheath
- 1236: Tubing
- 1237: Medication Lumen
- 1238: External Conductor
- 1239: Protective Sheath
- 1240: First Axis
- 1241: Second Axis
- 1300: Tubing Cross Section
- 1301: Visual Feedback
- 1302: Visual Feedback
- 1303: Visual Feedback
- 1304: Feedback Pattern
- 1305: Feedback Pattern
- 1306: Feedback Pattern
- 1401: First Constraint Member
- 1402: Second Constraint Member
- 1403: First Contoured Constraint Profile
- 1404: Second Contoured Constraint Profile
- 1405: Unconstrained Tubing Set
- 1406: Compressive Assembly
- 1406': Compressive Assembly
- 1407: Locking Finger
- 1408: Aperture
- 1409: Aperture
- 1410: Locking Finger
- 1411: Tubing Set Medication Lumen(s)
- 1412: First Constraint Member Length
- 1413: Second Constraint Member Length
- 1414: Unconstrained Tubing Set Outer Diameter
- 1433: Constrained Inner Diameter
- 1435: Unconstricted Inner Diameter
- 1501: First Constraint Member
- 1502: Second Constraint Member
- 1503: First Contoured Constraint Profile
- 1504: Second Contoured Constraint Profile
- 1505: Unconstrained Tubing Set
- 1507: Locking Finger
- 1508: Apertures
- 1509: Apertures
- 1510: Locking Fingers
- 1511: Tubing Set Medication Lumen(s)
- 1520: Stress Relief
- 1521: Tubing Set Entrance
- 1522: Tubing Set Entrance
- 1523: Unconstrained Tubing Set Outer Diameter
- 1551: Assembled Constraint in First Position
- 1552: Assembled Constraint in Second Position
- 1553: Assembled Constraint in Third Position
- 1555: First Compressive Assembly Step
- 1555': First Compressive Assembly Step
- 1556: Second Compressive Assembly Step
- 1556': Second Compressive Assembly Step
- 1557: Third Compressive Assembly Step
- 1557': Third Compressive Assembly Step
- 1561: First Degree of Tubing Constraint
- 1562: Second Degree of Tubing Constraint
- 1571: Medication Inlet
- 1572: Medication Outlet
- 1573: Medication Inlet
- 1574: Medication Outlet
- 1575: Medication Inlet
- 1576: Medication Outlet
- 1600: Tubing Set
- 1601: First Constraint Member
- 1601': Open Side Arms
- 1602: Second Constraint Member
- 1603: (Sliding) Clamp Plate
- 1604: Clamp Travel Tabs
- 1605: Clamp Profile
- 1606: Clamp Travel Slot
- 1606': Clamp Travel Stops
- 1607: Clamp State Indicator
- 1608: Clamp State Indicator
- 1610: Assembled Constraint
- 1611: Sliding Clamp in open position
- 1612: Sliding Clamp in closed position
- 1613: Assembled Constraint
- 1701: Assembled Constraint
- 1702: Assembled Constraint
- 1703: Assembled Constraint
- 1704: Assembled Constraint
- 1705: Constraint
- 1706: Indicia
- 1707: Indicia
- 1708: Indicia
- 1709: Indicia
- 1710: Tubing Set
- 1720: Assembled Constraint
- 1721: Machine-Readable Indicia
- 1722: Electronic Indicia (First Constraint Member)
- 1723: Electronic Indicia (Second Constraint Member)
- 1724: Machine-Readable Indicia
- 1725: Electronic Indicia
- 1727: Human-Readable Indicia
- 1801: First Constraint Member
- 1802: Second Constraint Member
- 1805: Tubing Set
- 1806: Lumen
- 1807: Lumen
- 1808: Lumen
- 1810: Protrusion
- 1811: Recess
- 1821: First Constraint Member
- 1822: Second Constraint Member
- 1825: tubing set
- 1827: hook
- 1828: recess
- 1831: non-fitting constraint member
- 1832: fitting constraint member
- 1901: First Constraint Member
- 1902: Second Constraint Member
- 1903: plunger
- 1904: plunger removal prevention arm
- 1905: tubing set
- 1910: weak point
- 1911: outer part
- 1912: inner part
- 2101: device
- 2102: cartridge
- 2103: tubing set
- 2104: tubing
- 2105: patient
- 2201: device
- 2202A, B, C, D, E: valving
- 2210: power source
- 2211: pump
- 2212: user interface
- 2400: tubing set
- 2401: cross section
- 2402: lumen
- 2403: lumen
- 2404: lumen
- 2410: tubing set
- 2411: lumen
- 2412: lumen
- 2413: cross section
- 2500: tubing set
- 2501: lumen
- 2502: conductor
- 2503: conductor
- 2504: cross section
- 2510: tubing set
- 2511: lumen
- 2512: conductor
- 2513: cross section
- 2600: tubing set
- 2601: external conductor
- 2602: undercut
- 2604: lumen
- 2605: conductor
- 2606: conductor
- 2607: cross section

## Claims

1. An apparatus configured to deliver a therapeutic medication (220) to a patient (217), the apparatus comprising:
a reservoir (208) containing a therapeutic medication; and
a patient interface (216) configured to deliver contents of the reservoir into the patient; and
at least one sensor (407) configured to detect at least one of a physiological aspect of the patient and a physical aspect of the apparatus;
a controller (403) configured to receive data (410) from the sensor, and to start and stop delivery of the therapeutic medication to the patient in response to data received from the sensor; and
a tubing set (1209) with at least one medication lumen (1210) having an internal surface and an external surface, the at least one medication lumen in fluid communication with the reservoir at a proximal end of the tubing set and the patient interface at a distal end of the tubing set, the tubing set also comprising at least one conductor (1212) in electrical or optical communication with the controller at the proximal end of the tubing set and the sensor at a distal end of the tubing set;
**characterised in that**
the apparatus is configured to detect a system infusion reaction using the at least one sensor; arrest delivery of the therapeutic medicine; and administer one or more emergency counteracting medications.

2. The apparatus of claim 1, wherein the at least one conductor (1212) is situated internally within the tubing set (1209).

3. The apparatus of claim 1 or 2, wherein the at least one conductor (1212) is situated internally within the tubing set (1209), and said at least one conductor is substantially parallel to the at least one medication lumen (1210) from the proximal to the distal end of said tubing set.

4. The apparatus of any of claims 1 to 3, wherein the at least one conductor (1212) is situated on an external surface of the tubing set (1209).

5. The apparatus of claim 4, wherein the at least one conductor (1212) situated on the external surface of the tubing set comprises a conductive ink.

6. The apparatus of any of claims 1 to 5, further comprising a barrier coating (1223) situated on the internal surface of the at least one medication lumen (1227), the barrier coating further configured to isolate medication within the at least one medication lumen from undesirable extractable or leachable materials from either the tubing set or the at least one conductor.

7. The apparatus of any of claims 1 to 6, further comprising a barrier coating (1223) interposed between the at least one conductor (1212) and the external surface of the at least one medication lumen (1210).

8. The apparatus of claim 6 or claim 7, wherein the barrier coating (1223) comprises polytetrafluoroethylene.

9. The apparatus of any of claims 1 to 8, further comprising a protective sheath (1235) over the at least one conductor (1212) on the external surface of the at least medication lumen (1210).

10. The apparatus of any of claims 1 to 9, further comprising an undercut feature (1232) situated on an exterior contour of the tubing set (1209), said undercut containing one or more electrical or optical conductors (1212), preferably further comprising a protective sheath (1235) substantially enclosing the undercut feature and any electrical or optical conductors (1212) situated therein.

11. The apparatus of any of claims 1 to 10, wherein the tubing set (1209) comprises an asymmetric cross-section (2607) with an undercut feature (2602) containing one or more electrical or optical conductors (2605), the undercut feature further being situated in the tubing set cross-section exhibiting the highest bending stiffness.

12. The apparatus of any of claims 1 to 11, the tubing set (1125) further comprising an optical conductor (1125o), wherein the optical conductor comprises an optical fiber.

13. The apparatus of any of claims 1 to 12, the tubing set (1125) further comprising an optical conductor (1125o) and the controller (1121) comprising at least one light (1301) disposed therein, the light configured to operate in conjunction with the optical conductor to alert a user as to an aspect or status of a drug delivery apparatus connected to the tubing set.

14. The apparatus of claim 13, further comprising an illuminated alert (1134) to a user of the apparatus, wherein the illuminated alert is optionally presented to a user as a color code including different colors or as a pulsed pattern of one or more different colors.

15. The apparatus of claim 13 or claim 14, wherein the alert (1134) presented to a user of the apparatus comprises a signal as to the status of a drug delivery device connected to the tubing set (1125), selected from one of the group of a) the drug delivery device is properly configured, b) the drug delivery device is ready to administer medication to a patient, c) the drug delivery device is currently administering one or more medications to a patient, d) the drug delivery device detected an error in configuration prior to administration, e) the drug delivery device detected an error during administration of one or more medications by the drug delivery device, f) the drug delivery device has completed medication administration, g) the drug delivery device has detected administration taking place at an unsafe rate, h) the drug delivery device has detected loss of skin contact at the patient interface, i) the drug delivery device has detected disconnection from or occlusion of the patient interface, or j) the tubing set in the apparatus is incorrect or incompatible with the medication within the drug delivery system, k) the drug delivery device has detected a suspected infusion reaction, l) the drug delivery device has detected anomalous physiologic sensor data, m) the drug delivery device has detected a tubing set occlusion, n) the patient interface is incorrectly configured, o) the drug delivery system has administered an emergency medication to a patient, p) the drug delivery system has a low battery level, q) the drug delivery system has lost connection to a sensor, r) the drug delivery system has lost connectivity to an external system or server, s) the drug delivery system has an expired medication or component, t) the drug delivery system has administered a pre-medication, u) the drug delivery system has administered a post-medication, v) the drug delivery system has lost connection to a telemedicine service, w) the drug delivery system has lost wireless connection, or x) the drug delivery system has lost cellular connection.

## Patentansprüche

1. Einrichtung, die konfiguriert ist, um eine therapeutische Medikation (220) an einen Patienten (217) abzugeben, die Einrichtung umfassend:
ein Reservoir (208), das eine therapeutische Medikation enthält; und
eine Patientenschnittstelle (216), die konfiguriert ist, um Inhalte des Reservoirs in den Patienten abzugeben; und
mindestens einen Sensor (407), der konfiguriert ist, um mindestens einen von einem physiologischen Aspekt des Patienten und einem physikalischen Aspekt der Einrichtung zu erfassen;
eine Steuerung (403), die konfiguriert ist, um Daten (410) von dem Sensor zu empfangen und um eine Abgabe der therapeutischen Medikation an den Patienten als Reaktion auf die Daten, die von dem Sensor empfangen werden, zu starten und zu stoppen; und
ein Schlauchset (1209) mit mindestens einem Medikationslumen (1210), das eine innere Oberfläche und eine äußere Oberfläche aufweist, wobei das mindestens eine Medikationslumen in Fluidkommunikation mit dem Reservoir an einem proximalen Ende des Schlauchsets und der Patientenschnittstelle an einem distalen Ende des Schlauchsets steht, das Schlauchset ebenso umfassend mindestens einen Leiter (1212), der in elektrischer oder optischer Kommunikation mit der Steuerung an dem proximalen Ende des Schlauchsets und dem Sensor an einem distalen Ende des Schlauchsets steht;
**dadurch gekennzeichnet, dass** die Einrichtung konfiguriert ist, um unter Verwendung des mindestens einen Sensors eine Systeminfusionsreaktion zu erfassen; die Abgabe der therapeutischen Medizin anzuhalten; und eine oder mehrere entgegenwirkende Notfallmedikationen zu verabreichen.

2. Einrichtung nach Anspruch 1, wobei sich der mindestens eine Leiter (1212) im Inneren innerhalb des Schlauchsets (1209) befindet.

3. Einrichtung nach Anspruch 1 oder 2, wobei sich der mindestens eine Leiter (1212) im Inneren innerhalb des Schlauchsets (1209) befindet und der mindestens eine Leiter von dem proximalen zu dem distalen Ende des Schlauchsets im Wesentlichen parallel zu dem mindestens einen Medikationslumen (1210) ist.

4. Einrichtung nach einem der Ansprüche 1 bis 3, wobei sich der mindestens eine Leiter (1212) auf einer äußeren Oberfläche des Schlauchsets (1209) befindet.

5. Einrichtung nach Anspruch 4, wobei der mindestens eine Leiter (1212), der sich auf der äußeren Oberfläche des Schlauchsets befindet, eine leitfähige Tinte umfasst.

6. Einrichtung nach einem der Ansprüche 1 bis 5, ferner umfassend eine Sperrbeschichtung (1223), die sich auf der inneren Oberfläche des mindestens einen Medikationslumens (1227) befindet, wobei die Sperrbeschichtung ferner konfiguriert ist, um die Medikation innerhalb des mindestens einen Medikationslumens von unerwünschten extrahierbaren oder auslaugbaren Materialien entweder aus dem Schlauchset oder dem mindestens einen Leiter zu isolieren.

7. Einrichtung nach einem der Ansprüche 1 bis 6, ferner umfassend eine Sperrbeschichtung (1223), die zwischen dem mindestens einen Leiter (1212) und der äußeren Oberfläche des mindestens einen Medikationslumens (1210) eingeschoben ist.

8. Einrichtung nach Anspruch 6 oder 7, wobei die Sperrbeschichtung (1223) Polytetrafluorethylen umfasst.

9. Einrichtung nach einem der Ansprüche 1 bis 8, ferner umfassend eine Schutzhülle (1235) über dem mindestens einen Leiter (1212) auf der äußeren Oberfläche des mindestens einen Medikationslumens (1210).

10. Einrichtung nach einem der Ansprüche 1 bis 9, ferner umfassend ein Hinterschnittmerkmal (1232), das sich an einer Außenkontur des Schlauchsets (1209) befindet, wobei der Hinterschnitt einen oder mehrere elektrische oder optische Leiter (1212) enthält, vorzugsweise ferner umfassend eine Schutzhülle (1235), die das Hinterschnittmerkmal und jegliche darin befindliche elektrische oder optische Leiter (1212) im Wesentlichen umschließt.

11. Einrichtung nach einem der Ansprüche 1 bis 10, wobei das Schlauchset (1209) einen asymmetrischen Querschnitt (2607) mit einem Hinterschnittmerkmal (2602) umfasst, das einen oder mehrere elektrische oder optische Leiter (2605) enthält, wobei sich das Hinterschnittmerkmal ferner in dem Schlauchsetquerschnitt befindet, der die höchste Biegesteifigkeit besitzt.

12. Einrichtung nach einem der Ansprüche 1 bis 11, das Schlauchset (1125) ferner umfassend einen optischen Leiter (1125o), wobei der optische Leiter eine optische Faser umfasst.

13. Einrichtung nach einem der Ansprüche 1 bis 12, das Schlauchset (1125) ferner umfassend einen optischen Leiter (1125o) und die Steuerung (1121) umfassend mindestens eine darin angeordnete Leuchte (1301), wobei die Leuchte konfiguriert ist, um zusammen mit dem optischen Leiter zu arbeiten, um einen Benutzer hinsichtlich eines Aspekts oder eines Status einer Arzneimittelverabreichungseinrichtung, die mit dem Schlauchset verbunden ist, zu alarmieren.

14. Einrichtung nach Anspruch 13, ferner umfassend einen leuchtenden Alarm (1134) für einen Benutzer der Einrichtung, wobei der leuchtende Alarm einem Benutzer optional als ein Farbcode, einschließlich verschiedener Farben, oder als ein gepulstes Muster aus einer oder mehreren verschiedenen Farben dargestellt wird.

15. Einrichtung nach Anspruch 13 oder Anspruch 14, wobei der Alarm (1134), der einem Benutzer der Einrichtung dargestellt wird, ein Signal hinsichtlich des Status einer Arzneimittelabgabevorrichtung, die mit dem Schlauchset (1125) verbunden ist, umfasst, der aus einer der Gruppe ausgewählt ist von a) die Arzneimittelabgabevorrichtung ist ordnungsgemäß konfiguriert, b) die Arzneimittelabgabevorrichtung ist bereit, um einem Patienten die Medikation zu verabreichen, c) die Arzneimittelabgabevorrichtung verabreicht einem Patienten aktuell eine oder mehrere Medikationen, d) die Arzneimittelabgabevorrichtung hat vor einer Verabreichung einen Konfigurationsfehler erfasst, e) die Arzneimittelabgabevorrichtung hat während der Verabreichung einer oder mehrerer Medikationen durch die Arzneimittelabgabevorrichtung einen Fehler erfasst, f) die Arzneimittelabgabevorrichtung hat eine Arzneimittelverabreichung abgeschlossen, g) die Arzneimittelabgabevorrichtung hat erfasst, dass die Verabreichung mit einer unsicheren Rate stattfindet, h) die Arzneimittelabgabevorrichtung hat einen Verlust eines Hautkontakts an der Patientenschnittstelle erfasst, i) die Arzneimittelabgabevorrichtung hat eine Trennung von oder Okklusion der Patientenschnittstelle erfasst, oder j) das Schlauchset in der Einrichtung ist falsch oder inkompatibel mit der Medikation innerhalb des Arzneimittelabgabesystems, k) die Arzneimittelabgabevorrichtung hat eine vermutete Infusionsreaktion erfasst, I) die Arzneimittelabgabevorrichtung hat anomale physiologische Sensordaten erfasst, m) die Arzneimittelabgabevorrichtung hat eine Schlauchsetokklusion erfasst, n) die Patientenschnittstelle ist falsch konfiguriert, o) das Arzneimittelabgabesystem hat einem Patienten eine Notfallmedikation verabreicht, p) das Arzneimittelabgabesystem weist einen niedrigen Batteriestand auf, q) das Arzneimittelabgabesystem hat die Verbindung zu einem Sensor verloren, r) das Arzneimittelabgabesystem hat eine Konnektivität zu einem externen System oder Server verloren, s) das Arzneimittelabgabesystem weist eine abgelaufene Medikation oder eine abgelaufene Komponente auf, t) das Arzneimittelabgabesystem hat eine Prämedikation verabreicht, u) das Arzneimittelabgabesystem hat eine Postmedikation verabreicht, v) das Arzneimittelabgabesystem hat die Verbindung zu einem Telemedizindienst verloren, w) das Arzneimittelabgabesystem hat eine drahtlose Verbindung verloren oder x) das Arzneimittelabgabesystem hat eine Mobilfunkverbindung verloren.

## Revendications

1. Appareil configuré pour délivrer un médicament thérapeutique (220) à un patient (217), l'appareil comprenant :
un réservoir (208) contenant un médicament thérapeutique ; et
une interface patient (216) configurée pour délivrer un contenu du réservoir au patient ; et
au moins un capteur (407) configuré pour détecter au moins l'un parmi un aspect physiologique du patient et un aspect physique de l'appareil ;
un dispositif de commande (403) configuré pour recevoir des données (410) du capteur, et pour démarrer et arrêter la délivrance du médicament thérapeutique au patient en réponse aux données reçues du capteur ; et
un ensemble de tubulure (1209) doté d'au moins une lumière de médicament (1210) ayant une surface interne et une surface externe, l'au moins une lumière de médicament étant en communication fluidique avec le réservoir au niveau d'une extrémité proximale de l'ensemble de tubulure et l'interface patient au niveau d'une extrémité distale de l'ensemble de tubulure, l'ensemble de tubulure comprenant également au moins un conducteur (1212) en communication électrique ou optique avec le dispositif de commande au niveau de l'extrémité proximale de l'ensemble de tubulure et le capteur au niveau d'une extrémité distale de l'ensemble de tubulure ;
**caractérisé en ce que** l'appareil est configuré pour détecter une réaction de perfusion de système à l'aide de l'au moins un capteur ; arrêter la délivrance du médicament thérapeutique ; et administrer un ou plusieurs médicaments antagonistes d'urgence.

2. Appareil selon la revendication 1, dans lequel l'au moins un conducteur (1212) est situé de manière interne au sein de l'ensemble de tubulure (1209).

3. Appareil selon la revendication 1 ou 2, dans lequel l'au moins un conducteur (1212) est situé de manière interne au sein de l'ensemble de tubulure (1209), et ledit au moins un conducteur est sensiblement parallèle à l'au moins une lumière de médicament (1210) de l'extrémité proximale à l'extrémité distale dudit ensemble de tubulure.

4. Appareil selon l'une quelconque des revendications 1 à 3, dans lequel l'au moins un conducteur (1212) est situé sur une surface externe de l'ensemble de tubulure (1209).

5. Appareil selon la revendication 4, dans lequel l'au moins un conducteur (1212) situé sur la surface externe de l'ensemble de tubulure comprend une encre conductrice.

6. Appareil selon l'une quelconque des revendications 1 à 5, comprenant en outre un revêtement barrière (1223) situé sur la surface interne de l'au moins une lumière de médicament (1227), le revêtement barrière étant en outre configuré pour isoler le médicament au sein de l'au moins une lumière de médicament de matériaux extractibles ou lixiviables indésirables provenant soit de l'ensemble de tubulure, soit de l'au moins un conducteur.

7. Appareil selon l'une quelconque des revendications 1 à 6, comprenant en outre un revêtement barrière (1223) interposé entre l'au moins un conducteur (1212) et la surface externe de l'au moins une lumière de médicament (1210).

8. Appareil selon la revendication 6 ou la revendication 7, dans lequel le revêtement barrière (1223) comprend du polytétrafluoroéthylène.

9. Appareil selon l'une quelconque des revendications 1 à 8, comprenant en outre une gaine de protection (1235) sur l'au moins un conducteur (1212) sur la surface externe de l'au moins une lumière de médication (1210).

10. Appareil selon l'une quelconque des revendications 1 à 9, comprenant en outre un élément en contre-dépouille (1232) situé sur un contour extérieur de l'ensemble de tubulure (1209), ladite contre-dépouille contenant un ou plusieurs conducteurs électriques ou optiques (1212), comprenant de préférence en outre une gaine de protection (1235) enfermant sensiblement l'élément en contre-dépouille et de quelconques conducteurs électriques ou optiques (1212) situés à l'intérieur de celui-ci.

11. Appareil selon l'une quelconque des revendications 1 à 10, dans lequel l'ensemble de tubulure (1209) comprend une section transversale asymétrique (2607) avec un élément en contre-dépouille (2602) contenant un ou plusieurs conducteurs électriques ou optiques (2605), l'élément en contre-dépouille étant en outre situé dans la section transversale de l'ensemble de tubulure présentant la rigidité en flexion la plus élevée.

12. Appareil selon l'une quelconque des revendications 1 à 11, l'ensemble de tubulures (1125) comprenant en outre un conducteur optique (1125o), dans lequel le conducteur optique comprend une fibre optique.

13. Appareil selon l'une quelconque des revendications 1 à 12, l'ensemble de tubulure (1125) comprenant en outre un conducteur optique (1125o) et le dispositif de commande (1121) comprenant au moins une lumière (1301) disposée à l'intérieur de celui-ci, la lumière étant configurée pour fonctionner conjointement avec le conducteur optique pour alerter un utilisateur quant à un aspect ou un état d'un appareil d'administration de médicament raccordé à l'ensemble de tubulure.

14. Appareil selon la revendication 13, comprenant en outre une alerte lumineuse (1134) pour un utilisateur de l'appareil, dans lequel l'alerte lumineuse est facultativement présentée à un utilisateur sous la forme d'un code couleur comportant différentes couleurs ou sous la forme d'un motif pulsé d'une ou plusieurs couleurs différentes.

15. Appareil selon la revendication 13 ou la revendication 14, dans lequel l'alerte (1134) présentée à un utilisateur de l'appareil comprend un signal quant à l'état d'un dispositif de délivrance de médicament raccordé à l'ensemble de tubulure (1125), choisi parmi l'un du groupe de a) le dispositif de délivrance de médicament est correctement configuré, b) le dispositif de délivrance de médicament est prêt à administrer un médicament à un patient, c) le dispositif de délivrance de médicament administre actuellement un ou plusieurs médicaments à un patient, d) le dispositif de délivrance de médicament a détecté une erreur de configuration avant l'administration, e) le dispositif de délivrance de médicament a détecté une erreur pendant l'administration d'un ou plusieurs médicaments par le dispositif de délivrance de médicament, f) le dispositif de délivrance de médicament a terminé l'administration de médicament, g) le dispositif de délivrance de médicament a détecté une administration se produisant à un débit dangereux, h) le dispositif de délivrance de médicament a détecté une perte de contact cutané au niveau de l'interface patient, i) le dispositif de délivrance de médicament a détecté une déconnexion ou une occlusion de l'interface patient, ou j) l'ensemble de tubulure dans l'appareil est incorrect ou incompatible avec le médicament au sein du système de délivrance de médicament, k) le dispositif de délivrance de médicament a détecté une réaction suspectée à la perfusion, I) le dispositif de délivrance de médicament a détecté des données anormales de capteur physiologique, m) le dispositif de délivrance de médicament a détecté une occlusion de l'ensemble de tubulure, n) l'interface patient est configurée de manière incorrecte, o) le système de délivrance de médicament a administré un médicament d'urgence à un patient, p) le système de délivrance de médicament a un niveau de batterie faible, q) le système de délivrance de médicament a perdu la connexion à un capteur, r) le système de délivrance de médicament a perdu la connectivité à un système ou serveur externe, s) le système de délivrance de médicament a un médicament ou un composant périmé, t) le système de délivrance de médicament a administré un pré-médicament, u) le système de délivrance de médicament a administré un post-médicament, v) le système de délivrance de médicament a perdu la connexion à un service de télémédecine, w) le système de délivrance de médicament a perdu la connexion sans fil, ou x) le système de délivrance de médicament a perdu la connexion cellulaire.
